(19) Europäisches Patentamt
European Patent Office
Office européen des brevets

(11) Numéro de publication : **0 176 399**
**B1**

# (12) FASCICULE DE BREVET EUROPÉEN

(45) Date de publication du fascicule du brevet : **26.04.89**

(21) Numéro de dépôt : **85401672.2**

(22) Date de dépôt : **22.08.85**

(51) Int. Cl.$^4$ : **C 07 J 21/00**, C 07 J 1/00, C 07 J 31/00, C 07 J 33/00, C 07 J 41/00, C 07 J 43/00, C 07 J 51/00, C 07 J 53/00, A 61 K 31/565, A 61 K 31/58

## (54) Nouveaux stéroides substitués en position 10 par un radical comportant une double ou triple liaison, leur procédé de préparation, leur application comme médicaments, les compositions pharmaceutiques les renfermant.

**Demande divisionnaire 88117295.1 déposée le 22/08/85.**

(30) Priorité : **24.08.84 FR 8413189**

(43) Date de publication de la demande : **02.04.86 Bulletin 86/14**

(45) Mention de la délivrance du brevet : **26.04.89 Bulletin 89/17**

(84) Etats contractants désignés : **AT BE CH DE FR GB IT LI LU NL SE**

(56) Documents cités :
**EP-A- 0 031 591**
**FR-A- 1 519 387**
**FR-A- 2 344 286**
**GB-A- 2 078 749**
**US-A- 3 102 127**
**US-A- 3 218 316**
**US-A- 3 275 622**
**JOURNAL OF THE AMERICAN CHEMICAL SOCIETY, vol. 79, no. 17, 11 septembre 1957, pages 4808-4809, Washington DC, US; J.A. CELLA et al.: "Steroidal lactones"**
**STEROIDS, vol. 39, no. 3, mars 1982, pages 325-344, San Francisco, US; P.A. MARCOTTE et al.: "Synthesis and evaluation of 10beta-substituted 4-estrene-3,17-diones as inhibitors of human placental microsomal aromatase"**
**Bulletin Soc. Chim. France 1970, No. 7, page 2556**

(73) Titulaire : **ROUSSEL-UCLAF**
**35, boulevard des Invalides**
**F-75007 Paris (FR)**

(72) Inventeur : **Torelli, Vesperto**
**5, rue de la Convention**
**F-94700 - Maisons Alfort (FR)**
Inventeur : **Nedelec, Lucien**
**45, boulevard de L'Ouest**
**F-93340 - Le Raincy (FR)**
Inventeur : **Moguilewsky, Martine**
**80, boulevard Gambetta - Rue H. Ruel**
**F-94130 - Nogent Sur Marne (FR)**
Inventeur : **Moura, Anne-Marie**
**7, rue d'Arsonval**
**F-75015 - Paris (FR)**

(74) Mandataire : **Fritel, Hubert et al**
**Département des Brevets ROUSSEL UCLAF B.P. no 9**
**F-93230 Romainville (FR)**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

**EP 0 176 399 B1**

Jouve, 18, rue St-Denis, 75001 Paris, France

## Description

La présente invention concerne de nouveaux stéroïdes substitués en position 10 par un radical comportant une double ou triple liaison, leur procédé de préparation, leur application comme médicament et les compositions pharmaceutiques les renfermant.

Dans le brevet spécial de médicament FR. M 6768 correspondant au brevet FR A 1 519 837, sont décrits des 19-alcénylstéroïdes. Cependant l'activité décrite pour ces produits est différente de celle des produits de la présente demande. Dans le brevet américain US 3 218 316 sont décrits des stéroïdes comportant un radical éthynyle en position 10. De même que dans le brevet français précité, l'activité indiquée pour les produits du brevet américain est différente de celle des produits de la présente demande. Enfin, sont décrits, dans le brevet français FR 2 344 286, des 17-spirosulfines stéroïdes présentant une activité antagoniste de l'aldostérone. Ces produits ne comportent cependant pas de substituant alcényle ou alcynyle en position 10. Il n'était donc pas évident que l'introduction d'un radical alcényle ou alcynyle en position 10 des stéroïdes pourrait conduire à des produits présentant une activité antialdostérone comme cela est le cas des produits de la présente demande.

L'invention a pour objet les produits de formule générale I :

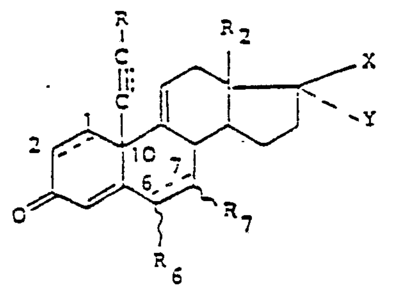

(I)

dans laquelle R représente :

— soit un atome d'hydrogène,

— soit un radical alkyle, alkényle ou alkynyle ayant au plus 8 atomes de carbone, éventuellement substitué par les radicaux hydroxyle, carboxy éventuellement estérifié, amino, tritylamino, chloroacétylamino, trifluoroacétylamino ; trichloroéthoxycarbonylamino, monoalkyl ou dialkylamino ou halogènes,

— soit un radical aryle ou aralkyle éventuellement substitué par les radicaux hydroxyle, carboxy éventuellement estérifié, amino, mono ou dialkylamino, alkyle, alkoxy ou alkylthio,

— soit un radical hydroxyle, tétrahydropyrannyloxy, tert-butyloxy, carboxy éventuellement estérifié, amino, mono ou dialkylamino, tritylamino, chloroacétylamino, trifluoroacétylamino ou trichloroéthoxycarbonylamino,

— soit un atome d'halogène,

— soit un radical trialkylsilyle,

$R_6$ et $R_7$ sont tels que soit $R_6$ et $R_7$ forment avec les carbones qui les portent un radical cyclopropyle, soit $R_6$ représente un atome d'hydrogène et $R_7$ représente le radical $R_1$, $R_1$ représentant :

— soit un atome d'hydrogène,

— soit un radical alkyle, alkényle ou alkynyle ayant au plus 6 atomes de carbone éventuellement substitué par les radicaux hydroxyle, carboxy éventuellement estérifié, amino, tritylamino, chloroacétylamino, trifluoroacétylamino, trichloroéthoxycarbonylamino, monoalkyl ou dialkylamino ou halogènes.

$R_2$ représente un radical méthyle ou éthyle,

X et Y sont tels que :

— soit X représente un radical hydroxyle, acétyloxy, propionyloxy, méthoxy ou éthoxy et Y représente ou bien un radical $—CH_2CH_2CO_2M$ dans lequel

M représente un atome d'hydrogène un atome de métal alcalin ou un radical ammonium,

ou bien un radical $—CH_2CH_2—CH_2OH$,

— soit X et Y représentent ensemble un radical :

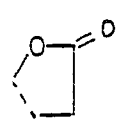 ou 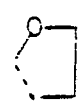

— soit X et Y représentent ensemble un radical

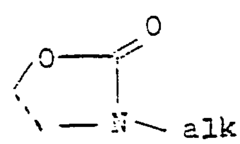

dans lequel alk représente un radical alkyle renfermant au plus 8 atomes de carbone,

— soit X et Y représentent ensemble un radical

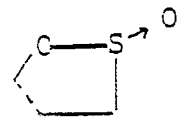

ou X représente un radical hydroxyle et Y un radical

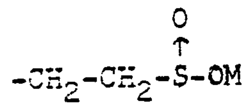

M étant défini comme ci-dessus,

— soit X représente un radical hydroxyle, acétyloxy, propionyloxy, méthoxy ou éthoxy et Y représente, ou bien un atome d'hydrogène ou bien Y représente $R_4$, $R_4$ représente un radical alkyle, alkényle ou alkynyle ayant au plus 6 atomes de carbone,

Les traits pointillés en position 1 (2) et 6 (7) indiquent la présence éventuelle d'une seconde liaison entre les carbones qui les portent, étant entendu qu'il ne peut pas y avoir de seconde liaison en position 6 (7) lorsque $R_6$ et $R_7$ forment avec les carbones qui les portent un radical cyclopropyle, le trait pointillé sur le substituant R—C$\equiv$C— en position 10 indique la présence éventuelle d'une troisième liaison entre les carbones qui les portent,

Les traits ondulés en position 6 et 7 indiquent que les substituants $R_6$ et $R_7$ peuvent se trouver dans l'une des positions possibles α ou β, étant entendu que R ne peut pas représenter un atome d'hydrogène lorsque $R_6$ et $R_7$ représentent chacun un atome d'hydrogène,

$R_2$ représente un radical méthyle, X représente un radical hydroxy ou acétoxy et Y un atome d'hydrogène, les traits pointillés en position 1 (2) et 6 (7) ne représentent pas une seconde liaison entre les carbones qui les portent et le trait pointillé sur le substituant en position 10 indique la présence d'une troisième liaison entre les carbones qui le portent.

L'invention a pour objet parmi les produits de formule (I), ceux répondant à la formule ($I_1$) :

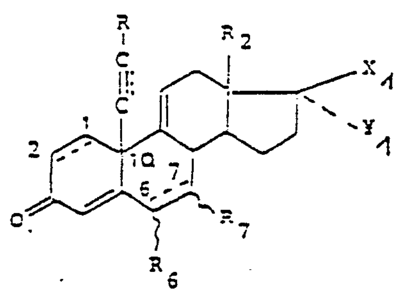

($I_1$)

dans laquelle R, $R_2$, $R_6$ et $R_7$ ont les significations indiquées ci-dessus,

$X_1$ et $Y_1$ sont tels que :

— soit $X_1$ représente un radical hydroxyle et $Y_1$ représente ou bien un radical $—CH_2CH_2CO_2M$ dans lequel M représente un atome d'hydrogène, un atome de métal alcalin ou un radical ammonium, ou bien un radical $—CH_2—CH_2—CH_2—OH$,

— soit $X_1$ et $Y_1$ représentent ensemble un radical :

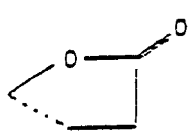

ou

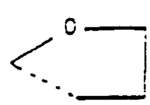

— soit $X_1$ représente un radical hydroxyle éventuellement acylé ou éthérifié et $Y_1$ représente ou bien un atome d'hydrogène ou bien $Y_1$ représente $R_4$, $R_4$ représentant un radical alkyle, alkényle ou alkynyle ayant au plus 6 atomes de carbone,

les traits pointillés en position 1 (2) et 6 (7) indiquent la présence éventuelle d'une seconde liaison entre les carbones qui les portent étant entendu qu'il ne peut pas y avoir de seconde liaison en position 6 (7) lorsque $R_6$ et $R_7$ forment avec les carbones qui les portent un radical cyclopropyle, le trait pointillé sur le substituant R—C$\equiv$C— en position 10 indique la présence éventuelle d'une troisième liaison entre les carbones qui les portent,

les traits ondulés en position 6 et 7 indiquent que les substituants $R_6$ et $R_7$ peuvent se trouver dans l'une des positions possibles α ou β, étant entendu que R ne peut pas représenter un atome d'hydrogène lorsque $R_6$ et $R_7$ représentent chacun un atome d'hydrogène, $R_2$ représente un radical méthyle, $X_1$ représente un radical hydroxy ou acétoxy et $Y_1$ un atome d'hydrogène, les traits pointillés en position 1 (2) et 6 (7) ne représentent pas une seconde liaison entre les carbones qui les portent et le trait pointillé sur le substituant en position 10 indique la présence d'une troisième liaison entre les carbones qui le portent.

Parmi les valeurs de R on peut citer les radicaux méthyle, éthyle, propyle, isopropyle, butyle, butyle secondaire, tert-butyle et les radicaux pentyle, hexyle, heptyle ou octyle éventuellement ramifiés.

On peut également citer les radicaux alkényles suivant : vinyle, allyle, 1-propényle, butényle, les radicaux alkynyles tels que éthynyle, propargyle ou butynyle.

Ces différents radicaux peuvent être substitués, par exemple par les radicaux hydroxyle, carboxy éventuellement estérifié tel que méthoxycarbonyle, éthoxycarbonyle, tritylamino, chloroacétylamino, trifluoroacétylamino, trichloroéthoxycarbonylamino, monoalkyl ou dialkylamino tel que méthylamino et diméthylamino, les atomes d'halogènes, de préférence chlore ou brome.

Les radicaux aryles et aralkyles que peut également représenter R sont de préférence un radical phényle, benzyle ou phényléthyle. Ces radicaux peuvent également être substitués, par exemple par les radicaux hydroxyle, carboxy éventuellement estérifiés, amino, mono ou dialkylamino, alkyle tel que méthyle, alkoxy tel que méthoxy, alkylthio tel que méthylthio.

La valeur trialkylsilyle préférée est la valeur triméthylsilyle.

Les valeurs que peut représenter le radical $R_1$ peuvent également être choisies dans les valeurs citées précédemment pour R. Il en est de même pour $R_4$.

M représente de préférence un atome de sodium, de potassium ou de lithium.

Lorsque $R_1$ représente un substituant différent d'un atome d'hydrogène, le substituant est de préférence en position 7α. Le radical cyclopropyle que $R_6$ et $R_7$ peuvent former avec les carbones qui les portent est également de préférence 6α, 7β.

Les composés de formule (I) dans laquelle X et Y représentent ensemble un radical

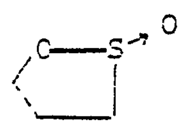

existent sous forme de deux diastéréoisomères au niveau de l'atome de soufre, éventuellement séparables l'un de l'autre et dénommés par convention isomères A et B, l'isomère A étant l'isomère dont le point de fusion est le plus élevé.

Parmi les produits de formule I on préfère d'abord les produits de formule :

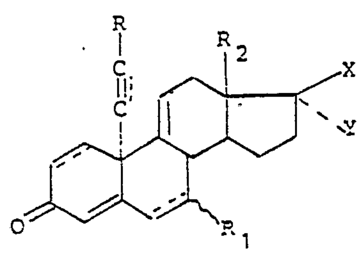

dans laquelle R, $R_1$, $R_2$, X, et Y, les traits pointillés et les traits ondulés ont la signification indiquée ci-dessus.

L'invention a plus particulièrement pour objet les produits de formule I telle que décrite ci-dessus dans laquelle les substituants que peuvent comporter les radicaux R et $R_1$ sont choisis dans le groupe formé par les radicaux hydroxyle, carboxy éventuellement estérifié, amino, amino protégé, mono ou dialkylamino et les atomes d'halogènes.

L'invention a également particulièrement pour objet les produits de formule I telle que définie ci-dessus dans laquelle le trait pointillé sur le substituant en position 10 représente une troisième liaison et R est choisi dans le groupe formé par un atome d'hydrogène, un radical alkyle ayant de 1 à 3 atomes de carbone, un radical hydroxyméthyle et un radical phényle, ainsi que les produits de formule I dans laquelle le substituant $R_1$ est choisi dans le groupe formé par un atome d'hydrogène, un radical alkyle ayant de 1 à 4 atomes de carbone et un radical acétylthio et X et Y sont tels que :

— soit X représente un radical hydroxy et Y représente ou bien un radical $CH_2CH_2CO_2M'$ dans lequel M' représente un atome d'hydrogène ou un atome de métal alcalin, ou bien Y représente un atome d'hydrogène,

— soit X et Y représentent un radical

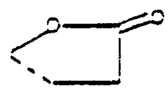

L'invention a plus particulièrement pour objet les produits décrits ci-après dans les exemples et notamment :

— la γ-lactone de l'acide 10β-éthynyl 17β-hydroxy 3-oxo 19-nor 17α-pregna 4,9 (11)-diène 21-carboxylique

— la γ-lactone de l'acide 10β-(1-propynyl) 17β-hydroxy 3-oxo 19-nor 17α-pregna 4,9 (11)-diène 21-carboxylique

— la γ-lactone de l'acide 10β-(3-hydroxy prop 1-ynyl) 17β-hydroxy 3-oxo 19-nor 17α-pregna 4,9 ((11)-diène 21-carboxylique

— la γ-lactone de l'acide 10β-éthynyl 7α-acétylthio 17β-hydroxy 3-oxo 19-nor 17α-pregna 4,9 (11) diène 21-carboxylique

— la γ-lactone de l'acide 10β-éthynyl 17β-hydroxy 3-oxo 19-nor 7α-propyl 17α-pregna 4,9 (11) diène 21 carboxylique

— la 17β hydroxy 10β-(prop-1-ynyl) estra 4,9 (11) diène 3-one.

Parmi ces produits, les produits plus particulièrement retenus sont :

— la γ-lactone de l'acide 10β-éthynyl 17β-hydroxy 3-oxo 19-nor 17α-pregna 4,9 (11)-diène 21-carboxylique

et la γ-lactone de l'acide 10β-(1-propynyl) 17β-hydroxy 3-oxo 19- nor 17α-pregna 4,9 (11)-diène 21-carboxylique.

L'invention a également pour objet un procédé de préparation des produits de formule I telle que définie ci-dessus, caractérisé en ce que l'on traite un produit de formule II :

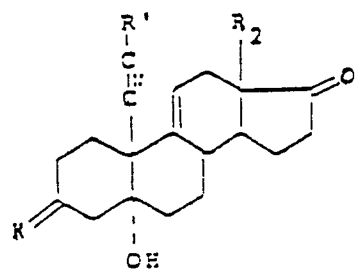

(II)

dans laquelle R' représente soit R, R ayant la signification indiquée ci-dessus soit le substituant R dans lequel les fonctions réactivées sont protégées, $R_2$ a la signification indiquée ci-dessus et K représente un groupement protecteur de la fonction cétone,

I) soit d'abord par un halogénure de triméthylsulfonium en présence d'une base forte puis par un dérivé métallique de l'acétonitrile et enfin par une base puis par un acide, soit d'abord par un réactif de formule :

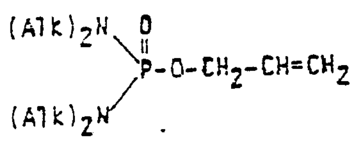

dans lequel Alk représente un radical alkyle ayant de 1 à 4 atomes de carbone en présence d'une base forte puis par un acide pour obtenir un produit de formule $I_A$ :

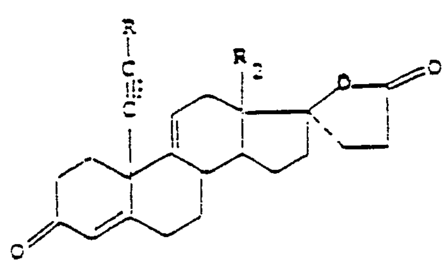

($I_A$)

II) soit par un réactif de formule :

$$XMg—CH_2CH_2CH_2OB$$

dans lequel X représente un atome d'halogène et B représente un groupement protecteur du radical hydroxyle ou un alcoolate de magnésium pour obtenir après traitement par un acide un produit de formule $I'_A$ :

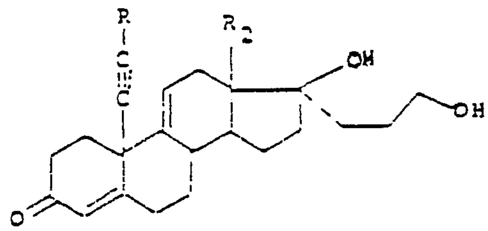

($I_A'$)

produit de formule $I'_A$ que l'on traite éventuellement par un halogénure d'acide sulfonique en présence d'une base pour obtenir un produit de formule $I''_A$ :

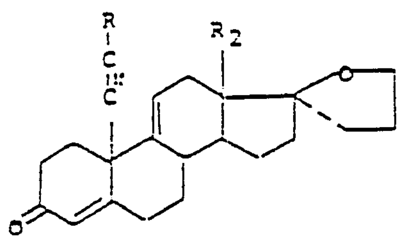

($I_A''$)

III) Soit par un réactif de réduction puis par un acide pour obtenir un produit de formule $I_{3A}$ :

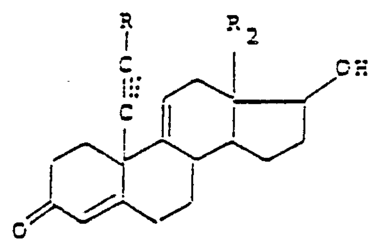

($I_{3A}$)

IV) soit par un organo métallique $R_4$ MgX ou $(R_4)_2$—CuLi, X représentant un atome d'halogène, puis par un acide, pour obtenir un produit de formule $I_{4A}$ :

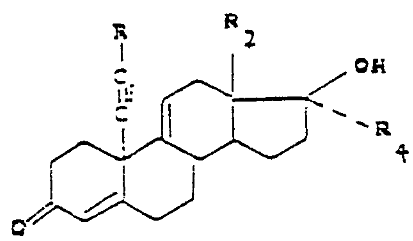

($I_{4A}$)

V) soit d'abord par un halogénure de triméthylsulfonium en présence d'une base forte, puis par une amine primaire de formule $H_2N$-alk, alk étant défini comme précédemment, en présence d'un acide, par un chloroformiate d'alkyle, par une base forte et enfin par un acide, pour obtenir un produit de formule $I_{5A}$ :

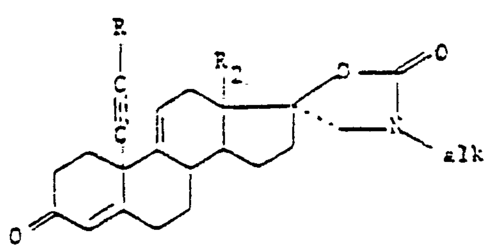

($I_{5A}$)

VI) soit d'abord par un halogénure de triméthylsulfonium en présence d'une base forte, puis par le méthyl terbutyl sulfoxyde en présence de n-butyl-lithium pour obtenir un produit de formule

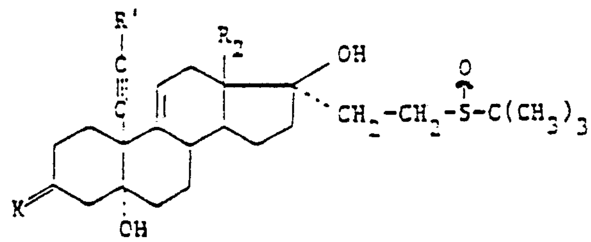

sous forme d'un mélange de deux diastéréoisomères au niveau de l'atome de soufre, sépare si désiré les deux diastéréoisomères, puis soumet soit leur mélange, soit chacun d'eux séparément, à l'action d'un acide, pour obtenir un produit de formule

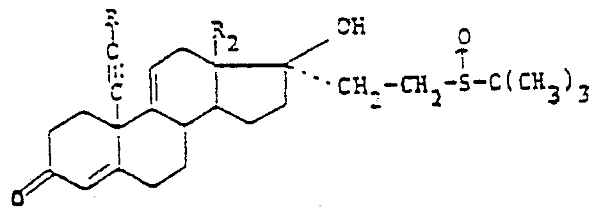

sous forme d'un mélange de deux diastéréoisomères ou un diastéréoisomère, sépare le cas échéant les diastéréoisomères, puis soumet soit leur mélange, soit chacun d'eux séparément, à l'action du N-chloro ou du N-bromo succinimide, pour obtenir un produit de formule $I_{6A}$ :

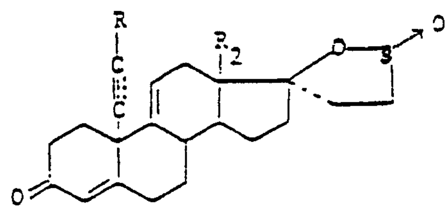

($I_{6A}$)

et que, si désiré, l'on traite les produits de formules $I_A$, $I'_A$, $I''_A$, $I_{3A}$, $I_{4A}$, $I_{5A}$ et $I_{6A}$ par un orthoformiate d'alkyle en présence d'un acide puis par un agent de déshydrogénation pour obtenir les produits de formule $I_B$, $I'_B$, $I''_B$, $I_{3B}$, $I_{4B}$, $I_{5B}$ et $I_{6B}$ de formules suivantes :

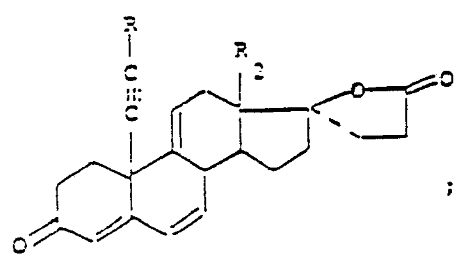

$I_B$

;

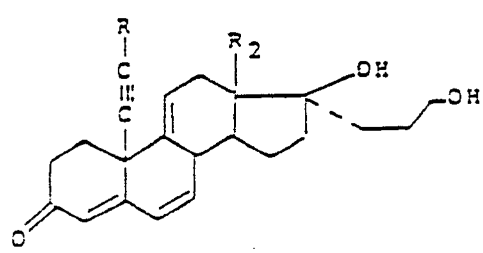

$I'_B$

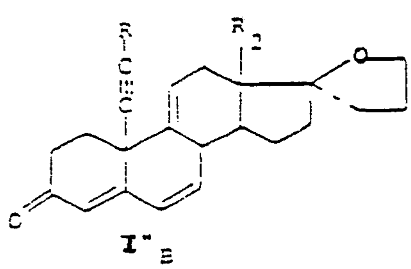

$I''_B$

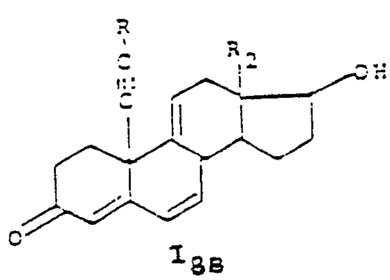

$I_{3B}$

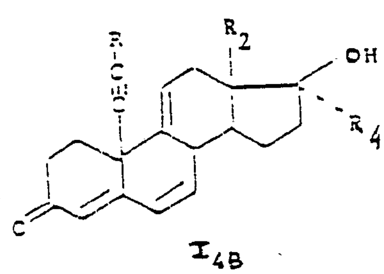

$I_{4B}$

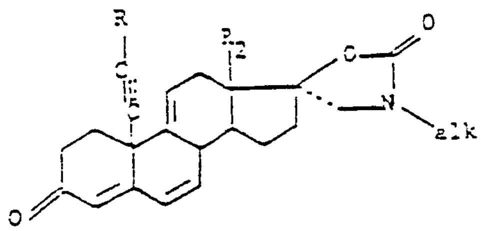

$I_{5B}$

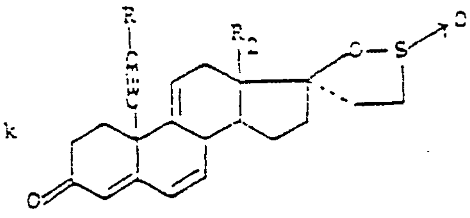

$I_{6B}$

produits de formules $I_B$, $I'_B$, $I''_B$, $I_{3B}$, $I_{4B}$, $I_{5B}$ et $I_{6B}$ que l'on traite si désiré :

— ou bien par un organo magnésien de formule $R_1MgX'$ éventuellement en présence d'un sel de cuivre, formule dans laquelle $R_1$ a la signification indiquée ci-dessus et X' représente un atome d'halogène

— ou bien par un organo métallique de formule $(R_1)_2CuLi$ puis par un acide, pour obtenir les produits de formules $I_C$, $I'_C$, $I''_C$, $I_{3C}$, $I_{4C}$, $I_{5C}$ et $I_{6C}$ :

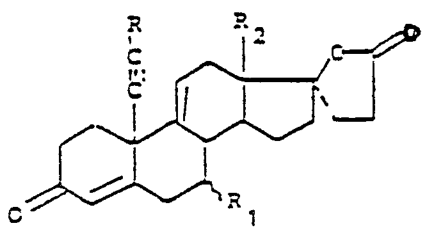

$I_C$

;

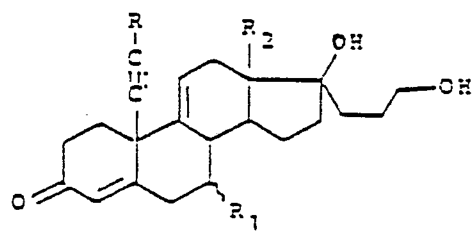

$I'_C$

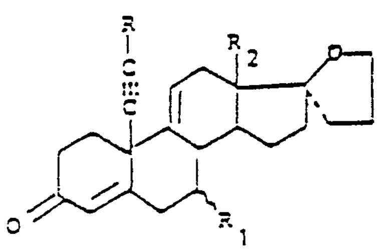

$I''_C$

;

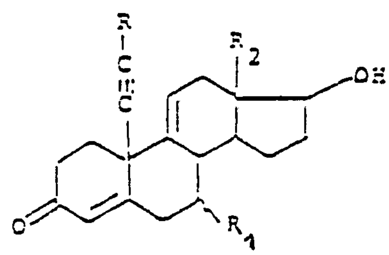

$I_{3C}$

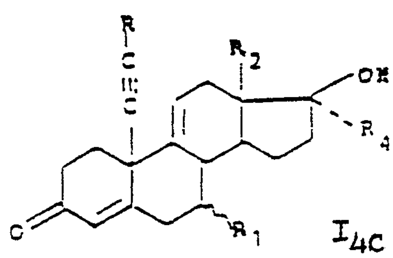

$I_{4C}$

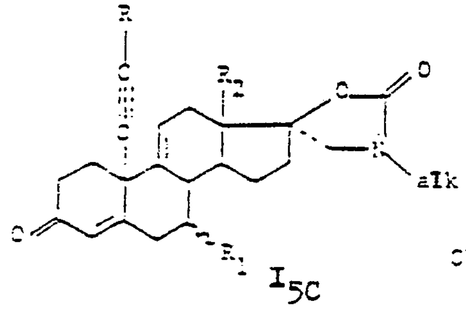

$I_{5C}$

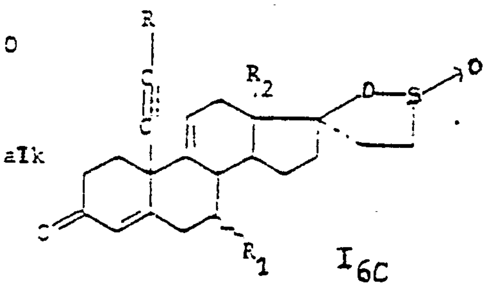

$I_{6C}$

sous forme d'un mélange d'isomères 7α et 7β, mélange que, si désiré, l'on sépare et que, si désiré, l'on soumet les produits 7β à un réactif de déshydrogénation pour obtenir les produits Δ6 (7) correspondants ;

— ou bien l'on soumet les produits de formules $I_B$, $I'_B$, $I''_B$, $I_{3B}$, $I_{4B}$, $I_{5B}$ et $I_{6B}$ à l'action d'un réactif choisi dans le groupe constitué par l'iodure de triméthylsulfonium et l'iodure de triméthylsulfoxonium en présence d'une base forte pour obtenir les produits de formules $I_D$, $I'_D$, $I''_D$, $I_{3D}$, $I_{4D}$, $I_{5D}$ et $I_{6D}$ :

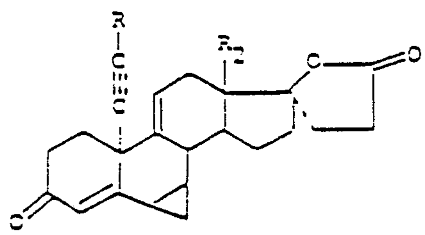

$I_D$

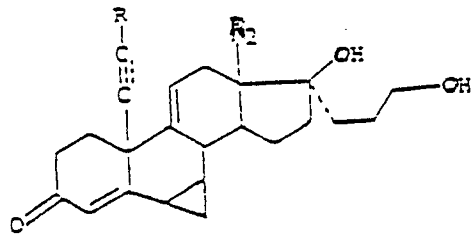

$I'_D$

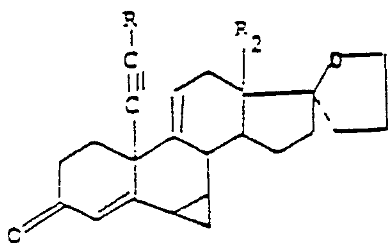

$I''_D$

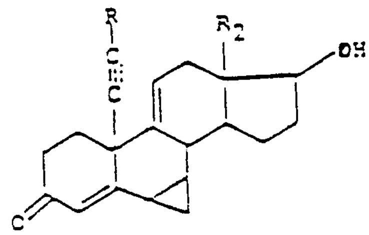

$I_{3D}$

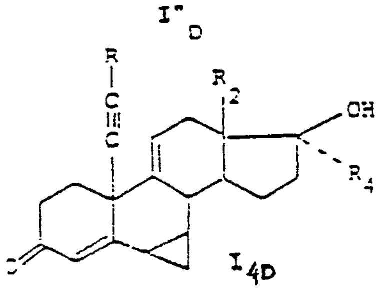

$I_{4D}$

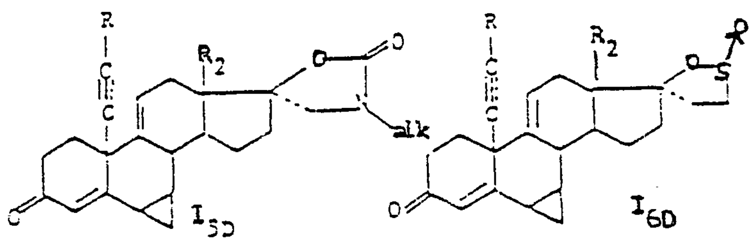

$I_{5D}$ $I_{6D}$

sous forme d'un mélange 6α, 7α et 6β, 7β et sépare, si désiré, les isomères ainsi obtenus et que si désiré l'on traite les produits $I_A$, $I'_A$, $I''_A$, $I_{3A}$, $I_{4A}$, $I_{5A}$, $I_{6A}$, $I_B$, $I'_B$, $I''_B$, $I_{3B}$, $I_{4B}$, $I_{5B}$, $I_{6B}$, $I_C$, $I'_C$, $I''_C$, $I_{3C}$, $I_{4C}$, $I_{5C}$, $I_{6C}$, $I_D$, $I'_D$, $I''_D$, $I_{3D}$, $I_{4D}$, $I_{5D}$, $I_{6D}$, par un réactif de déshydrogénation ou par un microorganisme susceptible de déshydrogéner la molécule en position 1 (2) pour obtenir les produits correspondants comportant une insaturation en position 1 (2) et que, si désiré, l'on traite les produits de formules $I_A$, $I_B$, $I_C$ et $I_D$ et les produits correspondants comportant une insaturation en position 1 (2) à l'aide d'un hydroxyde alcalin ou de l'ammoniaque pour obtenir les produits correspondants dans lesquels X représente un radical hydroxyle et Y représente un radical $-CH_2CH_2CO_2M'$ dans lequel M' représente un atome de métal alcalin ou un radical ammonium et, si désiré, soumet les produits ainsi obtenus à l'action d'un agent acide pour obtenir les produits dans lesquels X représente un radical hydroxyle et Y représente un radical $-CH_2CH_2CO_2H$, et que si désiré l'on traite les produits dans lesquels le substituant en position 10 comporte un substituant R du type hydroxyalkyle, par un tétra-bromure ou tétrachlorure de carbone en présence de triphénylphosphine, pour obtenir les dérivés bromés et chlorés correspondants, et que si désiré, l'on traite les produits dans lesquels le substituant en position 10 comporte une triple liaison par un agent d'hydrogénation sélective pour obtenir les produits correspondants dans lesquels le substituant en position 10 comporte une double liaison et que si désiré, l'on traite les produits comportant en position 10 un substituant éthynyl par un halo-succinimide pour obtenir les produits correspondant comportant en position 10 un substituant $-C{\equiv}C-Hal$, et que si désiré l'on acyle ou éthérifie le radical hydroxyle en position 17β des produits comportant en position 17α un atome d'hydrogène, un radical $R_4$ ou un radical $-CH_2-CH_2-CH_2OH$.

Les groupements protecteurs des fonctions que peut comporter R, par exemple les groupements hydroxy ou amino, sont choisis parmi les groupements connus.

Le groupement protecteur de la fonction cétone que représente K peut être un cétal, un thiocétal, un oxime ou un alkoxime. On utilise de préférence un cétal tel que le bis méthoxy ou un cétal cyclique tel que l'éthylène dioxy. Ces groupements sont éliminés en milieu acide.

L'halogénure de triméthylsulfonium que l'on utilise de préférence est l'iodure. La base forte en présence de laquelle on agit est de préférence le terbutylate de potassium.

On fait ensuite agir un dérivé métallique de l'acétonitrile qui peut par exemple être le dérivé lithien préparé en présence de butyllithium. Le dérivé ainsi obtenu qui comporte en position 17 un radical cyanoéthyle est ensuite soumis à l'action d'une base qui peut être par exemple la soude ou la potasse et on acidifie le produit obtenu par l'acide chlorhydrique de préférence.

L'action du tétralkyl phosphorodiamidate d'allyle, de préférence le tétraméthyle, sur le produit de formule II est effectuée selon la méthode décrite par STURTZ et YAOUANC, Synthesis 1980 p. 289. La base forte en présence de laquelle on agit est de préférence le butyllithium. On peut également opérer en plus en présence de diazabicyclo octane (DABCO) ou d'un éther couronne. On acidifie ensuite à l'aide d'acide chlorhydrique, de préférence.

L'action du produit de formule XMg—$CH_2CH_2CH_2OB$ sur le produit de formule II est effectuée selon les méthodes classiques de préparation de magnésien. Le radical B peut représenter un groupement protecteur habituel du radical hydroxyle, tel que le tétrahydropyrannyle ou le ter-butyle. B peut également représenter un sel de magnésium tel que MgCl. La préparation et l'utilisation d'un tel groupement sont décrites par CAHIEZ, ALEXAKIS et NORMANT Tetr. lett. n° 33, 1978, p. 3013. On acidifie ensuite à l'aide d'un acide, l'acide chlorhydrique de préférence.

La transformation des produits de formule $I'_A$ en produits $I''_A$ est effectuée de préférence en présence de chlorure de tosyle et d'une base aminée telle que la pyridine.

La réduction des produits de formule II en produits de formule $I_{3A}$ est effectuée par les méthodes classiques. On peut utiliser par exemple un hydrure tel que le borohydrure de sodium.

L'hydrolyse du groupement protecteur K est effectuée de manière générale par les méthodes classiques en milieu acide par exemple, par addition d'acide chlorhydrique.

L'addition du magnésien $R_4MgX$ et la réaction avec $(R_4)_2CuLi$ sont effectuées selon les méthodes classiques, on opère par exemple dans un solvant anhydre tel que le tétrahydrofuranne ou l'éther éthylique et à une température inférieure à la température ambiante. Comme pour les autres réactions indiquées précédemment on fait agir ensuite un acide tel que l'acide chlorhydrique pour régénérer la fonction cétone en position 3.

La base forte utilisée dans la cyclisation conduisant au produit $I_{5A}$ est de préférence la potasse méthanolique.

L'acide en présence duquel est effectuée la réaction avec l'amine est de préférence l'acide paratoluènesulfonique.

L'halogénure de triméthylsulfonium est de préférence un iodure et la base forte utilisée est la soude, la potasse ou le terbutylate de potassium.

La séparation des diastéréoisomères est effectuée par les moyens classiques de chromatographie et de cristallisation.

L'action d'un orthoformiate sur les produits $I_A$, $I'_A$, $I''_A$, $I_{3A}$, $I_{4A}$, $I_{5A}$ et $I_{6A}$ a pour but de préparer les produits 3-alkoxy Δ3,5 de formules :

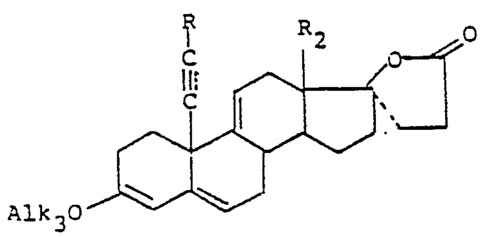

$I_{OA}$

;

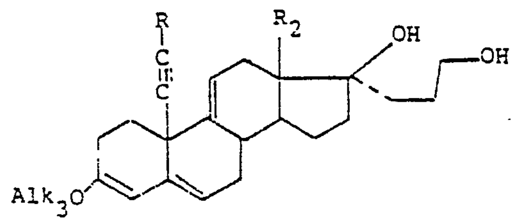

$I'_{OA}$

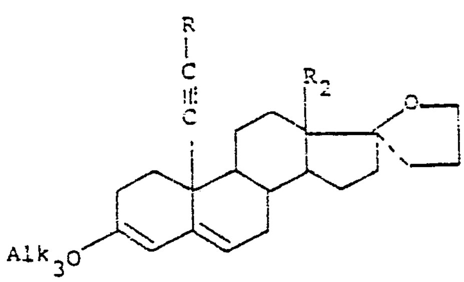

$I''_{OA}$

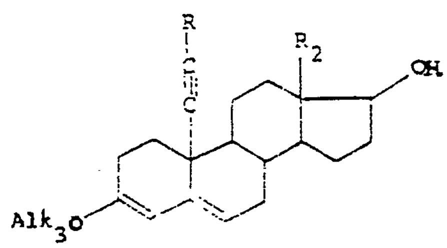

$I_{O3A}$

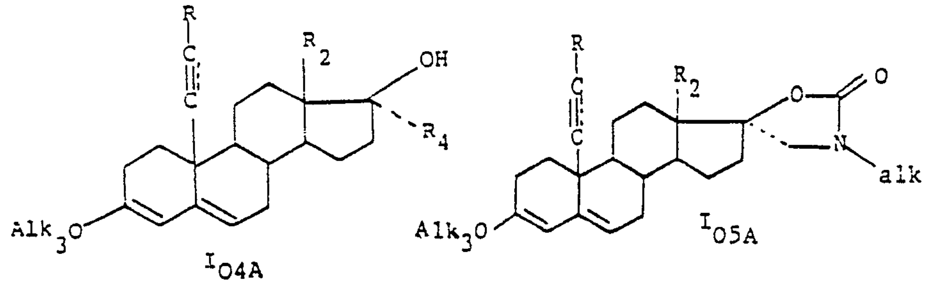

$I_{O4A}$ $I_{O5A}$

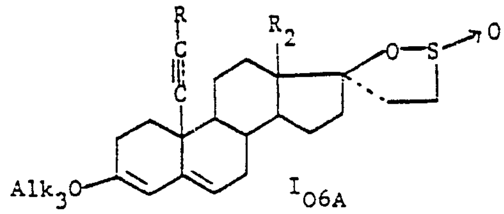

$I_{O6A}$

Dans les formules $I_{OA}$, $I'_{OA}$, $I''_{OA}$, $I_{O3A}$, $I_{O4A}$, $I_{O5A}$ et $I_{O6A}$ les radicaux R, $R_2$ et $R_4$ ont la signification précédente, $Alk_3$ représente un radical alkyle ayant de 1 à 4 atomes de carbone. L'orthoformiate utilisé est de préférence l'orthoformiate d'éthyle en présence d'acide paratoluènesulfonique. L'agent de déshydrogénation que l'on utilise est de préférence le chloranile (tétrachloro 1,4-benzoquinone). On peut également utiliser le DDQ (2,3-dichloro 5,6-dicyano 1,4-benzoquinone). L'action du produit de formule $R_1MgX'$ dans laquelle X' représente un atome de chlore, brome ou iode est effectuée de préférence en présence d'un sel cuivrique tel que l'acétate, le chlorure, le bromure cuivrique ou un sel cuivreux tel que le chlorure, le bromure ou l'iodure cuivreux.

L'acide que l'on utilise après action d'un produit de formule $(R_1)_2CuLi$ est l'acide chlorhydrique, nitrique ou sulfurique.

La séparation éventuelle des différents isomères est réalisée par chromatographie ou par cristallisation fractionnée. La déshydrogénation éventuelle des produits 7β est effectuée dans les conditions énoncées précédemment.

La base utilisée pour former l'ylide correspondant à l'iodure de triméthylsulfonium et l'iodure de triméthylsulfoxonium est l'hydrure de sodium ou le tert-butylate de potassium.

La séparation éventuelle est effectuée selon les méthodes classiques énoncées ci-dessus.

La transformation des produits $I_A$ à $I_{6A}$, $I_B$ à $I_{6B}$, $I_C$ à $I_{6C}$ et $I_D$ à $I_{6D}$ en produits comportant une insaturation en position 1 (2) est effectuée de préférence en faisant agir par voie biochimique, la bactérie « Arthrobacter simplex » On peut également utiliser un autre microorganisme. Dans ce cas, la réaction est effectuée de préférence en milieu aqueux tamponné.

On peut également utiliser la voie chimique en soumettant les produits à l'action d'un dérivé de la p-benzoquinone ou du chloranile, la réaction a lieu au sein, par exemple, d'une solution dans un solvant organique tel que le dioxanne, l'acétone, le benzène ou l'alcool tert-butylique.

On peut également utiliser, comme réactif de déshydrogénation, l'anhydride sélénieux ou benzène sélénique.

L'hydroxyde alcalin auquel on soumet éventuellement les produits de formules $I_A$, $I_B$ et $I_C$ ainsi que les produits correspondants comportant une insaturation en position 1 (2) est de préférence la soude ou la potasse.

L'agent acide auquel on soumet éventuellement les produits ainsi obtenus est l'acide chlorhydrique, sulfurique, nitrique ou acétique.

L'agent d'hydrogénation sélective auquel on soumet éventuellement les produits comportant en position 10 un substituant ayant une triple liaison est de préférence l'hydrogène en présence d'un catalyseur tel que le catalyseur de Lindlar.

L'halosuccinimide que l'on fait agir avec les produits comportant en position 10 un substituant éthynyl est le N-bromo ou le N-chloro-succinimide. On opère de préférence en présence de nitrate d'argent.

L'acylation éventuelle des produits comportant en position 17 un radical hydroxyle et en position 17β un atome d'hydrogène ou un radical $R_4$ est effectuée selon les méthodes usuelles. On peut utiliser par exemple un anhydride ou un halogénure d'acyle tel que l'anhydride acétique ou le chlorure d'acétyle. L'éthérification des mêmes produits est effectuée également dans les conditions usuelles par exemple à l'aide d'un halogénure d'alkyle.

Les produits de formule II utilisés au départ du procédé de la présente demande peuvent être préparés :

1) soit en faisant agir un lithien de formule R'—C≡C—Li sur un produit de formule III :

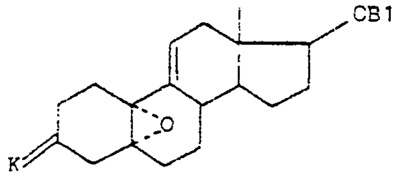

(III)

dans lequel $B_1$ représente un groupement protecteur du radical hydroxyle pour obtenir un produit de formule IV :

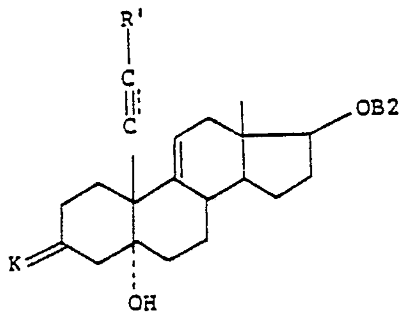

(IV)

dans laquelle $B_2$ représente un atome d'hydrogène ou le radical $B_1$ précédent, produit que l'on soumet d'abord à une réaction de déprotection du radical hydroxyle si le groupement protecteur n'a pas été clivé lors de la réaction précédente puis à une réaction d'oxydation pour obtenir les produits de formule II ;

2) soit en faisant agir un magnésien de formule R'—C≡C—MgX sur un produit de formule $III_1$ :

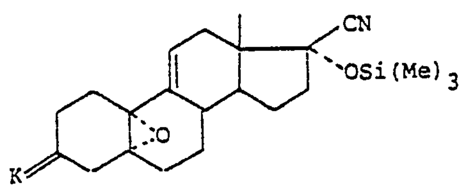

($III_1$)

pour obtenir un produit de formule $IV_1$ :

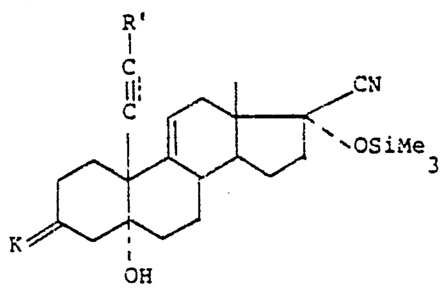

($IV_1$)

produit qui par hydrolyse alcaline donne le produit de formule II attendu.

1) Le groupement protecteur que peut représenter $B_1$ est de préférence un groupement acyle tel que benzoyle. Le lithien R'—C≡C—Li peut être préparé par les méthodes usuelles ; l'hydrolyse du groupement $B_1$ est également réalisée par les méthodes usuelles si le clivage n'est pas déjà intervenu lors de la réaction précédente. On peut utiliser une hydrolyse en milieu basique. L'oxydation en position 17 est effectuée par exemple par utilisation de chlorochromate de pyridinium préparé selon Tet. Letters. n° 31 (1975) p. 2647. Un tel exemple de préparation est décrit dans le brevet européen 0.023.856 (ex. 5).

2) L'action du magnésien de formule R'—C≡C—MgX sur le produit de formule $III_1$ est effectuée dans les conditions usuelles. Lorsque le trait pointillé représente une troisième liaison entre les carbones, le magnésien peut être préparé in situ par action du produit R'—C≡C—H sur un magnésien tel que le bromure d'éthyl-magnésium. On opère alors dans un solvant tel que le tétrahydrofuranne ou l'éther éthylique. L'hydrolyse du produit de formule $IV_1$ obtenu est effectuée dans les conditions usuelles. Des exemples de telles préparations figurent ci-après dans la partie expérimentale.

Les produits de formules III et $III_1$ sont soit connus, par exemple par le brevet français 2.377.417, soit peuvent être préparés par des méthodes usuelles à partir de ces produits connus.

Les produits de formule I telle que définie ci-dessus ainsi que la 10β-éthynyl 17β-hydroxy estra 4,9 (11)-dien-3-one et la 10β-éthynyl 17β-acétoxy estra 4,9 (11)-dièn-3-one, cet ensemble constituant la formule générale I', présentent de très intéressantes propriétés pharmacologiques ; ils sont en particulier des

antagonistes de l'aldostérone et augmentent la diurèse hydrosodée avec conservation du potassium organique.

Les produits de formule (I') offrent de plus l'avantage de ne présenter que très peu d'effets hormonaux secondaires.

Des tests effectués sur récepteur et in vivo ont, en particulier, montré que les produits de formule (I') sont moins anti-androgènes que des produits anti-aldostérone décrits précédemment.

Les produits de formule (I') peuvent donc être utilisés avantageusement pour lutter, notamment, contre l'hypertension artérielle et les insuffisances cardiaques.

L'invention a donc pour objet, à titre de médicaments, les produits de formule (I') :

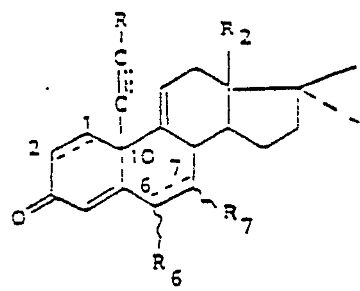

dans laquelle R représente :

— soit un atome d'hydrogène,

— soit un radical alkyle, alkényle ou alkynyle ayant au plus 8 atomes de carbone, éventuellement substitué par les radicaux hydroxyle, carboxy éventuellement estérifié, amino, tritylamino, chloroacétylamino, trifluoroacétylamino ; trichloroéthoxycarbonylamino, monoalkyl ou dialkylamino ou halogènes,

— soit un radical aryle ou aralkyle éventuellement substitué par les radicaux hydroxyle, carboxy éventuellement estérifié, amino, mono ou dialkylamino, alkyle, alkoxy ou alkylthio,

— soit un radical hydroxyle, tétrahydropyrannyloxy, tert-butyloxy, carboxy éventuellement estérifié, amino, mono ou dialkylamino, tritylamino, chloroacétylamino, trifluoroacétylamino ou trichloroéthoxycarbonylamino,

— soit un atome d'halogène,

— soit un radical trialkylsilyle,

$R_6$ et $R_7$ sont tels que soit $R_6$ et $R_7$ forment avec les carbones qui les portent un radical cyclopropyle, soit $R_6$ représente un atome d'hydrogène et $R_7$ représente le radical $R_1$, $R_1$ représentant :

— soit un atome d'hydrogène,

— soit un radical alkyle, alkényle ou alkynyle ayant au plus 6 atomes de carbone éventuellement substitué par les radicaux hydroxyle, carboxy éventuellement estérifié, amino, tritylamino, chloroacétylamino, trifluoroacétylamino, trichloroéthoxycarbonylamino, monoalkyl ou dialkylamino ou halogènes.

$R_2$ représente un radical méthyle ou éthyle,

X et Y sont tels que :

— soit X représente un radical hydroxyle, acétyloxy, propionyloxy, méthoxy ou éthoxy et Y représente ou bien un radical $-CH_2CH_2CO_2M$ dans lequel

M représente un atome d'hydrogène un atome de métal alcalin ou un radical ammonium, ou bien un radical $-CH_2CH_2-CH_2OH$,

— soit X et Y représentent ensemble un radical :

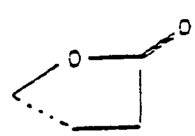

ou

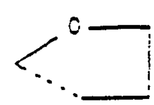

— soit X et Y représentent ensemble un radical

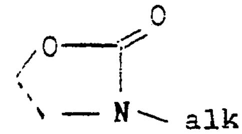

dans lequel alk représente un radical alkyle renfermant au plus 8 atomes de carbone,

— soit X et Y représentent ensemble un radical

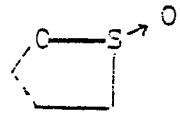

ou X représente un radical hydroxyle et Y un radical

$$-CH_2-CH_2-\overset{O}{\overset{\uparrow}{S}}-OM$$

M étant défini comme ci-dessus,

— soit X représente un radical hydroxyle, acétyloxy, propionyloxy, méthoxy ou éthoxy et Y représente, ou bien un atome d'hydrogène ou bien Y représente $R_4$, $R_4$ représente un radical alkyle, alkényle ou alkynyle ayant au plus 6 atomes de carbone,

Les traits pointillés en position 1 (2) et 6 (7) indiquent la présence éventuelle d'une seconde liaison entre les carbones qui les portent, étant entendu qu'il ne peut pas y avoir de seconde liaison en position 6 (7) lorsque $R_6$ et $R_7$ forment avec les carbones qui les portent un radical cyclopropyle, le trait pointillé sur le substituant R—C=C— en position 10 indique la présence éventuelle d'une troisième liaison entre les carbones qui les portent,

Les traits ondulés en position 6 et 7 indiquent que les substituants $R_6$ et $R_7$ peuvent se trouver dans l'une des positions possibles α ou β.

L'invention a plus particulièrement pour objet, à titre de médicaments les produits de formule générale I' dans laquelle R est choisi dans le groupe formé par un atome d'hydrogène, un radical alkyle ayant de 1 à 3 atomes de carbone, un radical hydroxyméthyle et un radical phényle, $R_1$ est choisi dans le groupe formé par un atome d'hydrogène, un radical alkyle ayant de 1 à 4 atomes de carbone et un radical acétylthio et X et Y sont tels que soit X représente le radical hydroxy et Y représente ou bien un radical $CH_2CH_2CO_2M'$ dans lequel M' représente un atome d'hydrogène ou un atome de métal alcalin ou bien Y représente un atome d'hydrogène soit X et Y représentent un radical

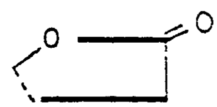

L'invention a plus spécialement pour objet, à titre de médicaments les produits de formule générale I' dont les noms suivent :

— la γ-lactone de l'acide 10β-éthynyl 17β-hydroxy 3-oxo 19-nor 17α-pregna 4,9 (11)-diène 21-carboxylique

— la γ-lactone de l'acide 10β-(1-propynyl) 17β-hydroxy 3-oxo 19-nor 17α-pregna 4,9 (11)-diène 21-carboxylique et la 10β-éthynyl 17β-hydroxy estra 4,9 (11)diène-3-one.

La posologie utile varie en fonction de l'affectation à traiter et de la voie d'administration ; elle peut aller par exemple de 5 mg à 500 mg et de préférence de 10 à 200 mg par jour chez l'adulte par voie orale pour le produit de l'exemple 1.

Les composés de formule (I') sont utilisés par voie buccale, rectale, transcutanée, ou intraveineuse. Ils peuvent être prescrits sous forme de comprimés, de comprimés enrobés, de cachets, de capsules, de granulés, d'émulsions, de sirops, de suppositoires et de préparations injectables.

L'invention a donc également pour objet les compositions pharmaceutiques renfermant comme principe actif au moins un composé de formule (I'). Ces compositions sont préparées selon les méthodes usuelles. Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

Les exemples suivants illustrent l'invention sans toutefois la limiter.

Exemple 1 : γ-lactone de l'acide 10β-éthynyl 17β-hydroxy 3-oxo 19-nor 17α-pregna 4,9 (11)-diène 21-carboxylique

Stade A : (17S) 3,3-éthylène dioxy 10β-éthynyl spiro/estr 9 (11)-ène 17, 2'-oxiran/5α-ol :

On ajoute sous agitation une solution de 3,06 g d'iodure de triméthylsulfonium dans 60 $cm^3$ de diméthylsulfoxyde à une suspension de 4,25 g de 3,3 éthylène dioxy 10β-éthynyl 5α-hydroxy estra 9 (11)èn 17-one dans 60 $cm^3$ de tétrahydrofuranne et 12 $cm^3$ d'une solution 2,4 M de tertiobutylate de potassium dans le tétrahydrofuranne. Après une heure de réaction on verse le mélange réactionnel dans de l'eau additionnée de chlorure d'ammonium. On extrait à l'éther éthylique, lave la phase organique, sèche et

obtient 4,5 g de produit attendu après évaporation du solvant sous pression réduite. Le produit est empâté dans de l'hexane, on essore les cristaux, les lave à l'hexane et obtient 3,895 g de produit attendu F : 142 °C.

Après recristallisation dans l'éthanol F : 144 °C.

Analyse : $C_{23}H_{30}O_4$ = 370,49
Calculé : C 74,56 H 8,16 %
Trouvé : C 74,7 H 8,2 %

Stade B : 5α, 17β-dihydroxy 3,3-éthylènedioxy estr 9 (11)-èn-17α-yl 3-propionitrile :

On refroidit à — 60 °C une solution de 11 $cm^3$ de N-isopropyl cyclohexyl amine dans 60 $cm^3$ de tétrahydrofuranne anhydre et ajoute goutte à goutte 40 $cm^3$ de n-butyl lithium en solution dans l'hexane (titre : 15 %) puis une solution de 3,5 $cm^3$ d'acétonitrile dans 10 $cm^3$ de tétrahydrofuranne anhydre. On obtient une suspension que l'on agite pendant 15 minutes à — 5, — 10 °C. On introduit ensuite une solution de 4 g de produit obtenu au stade A dans 20 $cm^3$ de tétrahydrofuranne.

On rince avec 5 $cm^3$ de tétrahydrofuranne puis laisse réagir à température ambiante pendant 20 heures. On verse le mélange dans une solution de chlorure d'ammonium et extrait à l'éther. On lave la phase organique à l'eau, sèche et obtient 9 g d'une huile après évaporation du solvant.

On filtre l'huile sur silice, élue avec un mélange cyclohexane-acétate d'éthyle (7-3) et sépare dans l'ordre 340 mg de produit de départ puis 5 g d'un produit que l'on cristallise dans l'éther au reflux. On refroidit, essore, lave à l'éther et obtient 2,827 g de produit attendu F : 200 °C. Par recristallisation dans le chlorure de méthylène on obtient un échantillon analytique F : 203 °C.

Analyse : $C_{25}H_{33}O_4N$ = 411,54
Calculé : C 72,96 H 8,08 N 3,4 %
Trouvé : C 72,8 H 8,2 N 3,4 %

Stade C : γ-lactone de l'acide 10β-éthynyl 17β-hydroxy 3-oxo 19-nor 17α-pregna 4,9 (11)-diène 21-carboxylique :

On chauffe au reflux pendant 4 heures trente une suspension de 1,9 g de produit obtenu au stade B dans 30 $cm^3$ de méthanol avec 2 $cm^3$ de potasse 6N. On refroidit et acidifie avec 3 $cm^3$ d'acide chlorhydrique concentré. On dilue avec 15 $cm^3$ de méthanol et chauffe de nouveau au reflux pendant une heure trente. On refroidit, dilue à l'eau et extrait à l'acétate d'éthyle. On obtient après lavage, séchage et évaporation de la phase organique 1,57 g de produit brut.

On filtre ce produit sur silice avec un mélange cyclohexane-acétate d'éthyle (1-1) et obtient 1,225 g de produit purifié que l'on empâte dans de l'éther isopropylique et essore. On obtient 1,17 g de produit attendu F : 210 °C.

Exemple 2 : 10β-éthynyl 17β-hydroxy 3-oxo estra-4,9 (11)-diène-17α-propanoate de potassium

On porte au reflux pendant 15 minutes une suspension de 387 mg de produit obtenu à l'exemple 1 dans 4,3 $cm^3$ de potasse méthanolique 0,246 N et 4,3 $cm^3$ d'eau. On distille à sec à 50 °C sous pression réduite la solution obtenue puis élimine les traces d'eau par deux distillations d'éthanol absolu. La mousse obtenue est dissoute dans 2 $cm^3$ d'éthanol à 95 °C, ajoute de l'éther lentement en grattant (environ 6 $cm^3$), glace, essore, lave abondamment à l'éther puis sèche. On obtient 380 mg de produit F # 200 °C.

Analyse : $C_{23}H_{27}O_4K$ = 406,57
Calculé : C 61,22 H 7,13 %
Trouvé : C 61,4 H 7,0 %

Exemple 3 : γ-lactone de l'acide 10β-éthényl 17β-hydroxy 3-oxo 19-nor 17α-pregna 4,9 (11)diène 21-carboxylique

On dissout 180 mg de γ-lactone de l'acide 10β-éthynyl 17β-hydroxy 3-oxo 19-nor 17α-pregna 4,9 (11)-diène-21-carboxylique (préparé à l'exemple 1) dans 4 $cm^3$ d'acétate d'éthyle avec 1 $cm^3$ de pyridine, ajoute 19 mg de catalyseur de Lindlar et agite en atmosphère d'hydrogénation jusqu'à absorption de la quantité théorique d'hydrogène soit pendant 25 minutes environ. On filtre le catalyseur, le rince à l'acétate d'éthyle, dilue le filtrat avec de l'eau légèrement acidulée avec de l'acide chlorhydrique, décante la phase organique, lave à l'eau sèche et concentre à sec sous pression réduite. On chromatographie le résidu sur silice imprégnée de nitrate d'argent, élue par un mélange éther-hexane (4-1) recueille 150 mg de produit que l'on recristallise dans l'éther isopropylique pour obtenir 121 mg de produit attendu F = 144 °C.

$/\alpha/_D$ : — 57° (c = 1 %, $CHCl_3$)

Exemple 4 : γ-lactone de l'acide 17β-hydroxy 3-oxo 10β-(1-propynyl) 19-nor 17α-pregna 4,9 (11)-diène-21-carboxylique

Stade A : 3,3-éthylène dioxy 5α-hydroxy 10β(1-propynyl) estr 9 (11)èn-17-one :

Dans 25 $cm^3$ d'une solution éthérée de bromure d'éthylmagnésium 1 M diluée avec 25 $cm^3$ de tétrahydrofuranne anhydre on fait barboter entre 0 et 5 °C du méthylacétylène pendant 1 heure. On ajoute 2,15 g de 3,3-éthylènedioxy 5α, 10α-époxy 17α-triméthylsilyloxy estr-9 (11)ène 17β-carbonitrile dans 10 $cm^3$ de tétrahydrofuranne, chauffe à 40 °C pendant 2 heures 30 et laisse 16 heures à température ambiante. On ajoute entre 0 et 5 °C, 20 $cm^3$ d'une solution de chlorure d'ammonium, extrait au chlorure de méthylène, lave à l'eau, sèche et concentre à sec. On dissout le résidu dans 30 $cm^3$ d'éthanol, ajoute 3 $cm^3$ de lessive de soude, agite pendant 1 heure, dilue avec 90 $cm^3$ d'eau et extrait au chloroforme, lave à l'eau, sèche et concentre à sec. On recristallise le résidu dans le mélange chlorure de méthylène-éther isopropylique et recueille 1,21 g de produit attendu F = 204 °C.

Stade B : γ-lactone de l'acide 17β-hydroxy 3-oxo 10β-(1-propynyl) 19-nor 17α-pregna 4,9 (11)-diène-21-carboxylique :

On ajoute en 15 minutes sous azote et à — 50 °C une solution de 3,6 $cm^3$ de tétraméthylphosphorodiamidate d'allyle dans 15 $cm^3$ de tétrahydrofuranne dans une solution de 23 $cm^3$ de butyllithium à 1,6 M dans l'hexane et 20 $cm^3$ de tétrahydrofuranne anhydre. On agite cette solution pendant une heure à — 30 °C et ajoute en 5 minutes, 1,78 g de produit obtenu comme précédemment.

Après 2 heures 20, on introduit dans le mélange 38 $cm^3$ d'acide chlorhydrique 2N, agite 30 minutes, extrait au chloroforme, lave à l'eau, sèche et concentre à sec. On dissout le résidu dans 30 $cm^3$ d'éthanol, ajoute 6 $cm^3$ d'acide chlorhydrique 6N, chauffe vers 50 °C pendant 1 heure 15 minutes, distille l'éthanol, reprend par du chlorure de méthylène, lave à l'eau, sèche et concentre à sec. On chromatographie le résidu sur silice, élue par un mélange éther de pétrole (eb. : 60-80 °C)-acétate d'éthyle (1-1) et obtient 920 mg de produit que l'on reprend par 2 $cm^3$ de chlorure de méthylène, filtre, ajoute 10 $cm^3$ d'éther isopropylique, concentre jusqu'à cristallisation, glace, essore et obtient après séchage 790 mg de produit attendu. F = 200 °C.

$/\alpha/_D$ : + 32° (c = 1 %, dans $CHCl_3$)

Exemple 5 : γ-lactone de l'acide 17β-hydroxy 3-oxo 10β-(phényléthynyl) 19-nor 17α-pregna 4,9 (11)-diène-21-carboxylique

Stade A : 3,3-éthylène dioxy 5α-hydroxy 10β-(phényléthynyl) 17α-triméthylsilyloxy estr 9 (11)-ène 17β-carbonitrile :

Dans 50 $cm^3$ d'une solution éthérée de bromure d'éthylmagnésium (M) on ajoute goutte à goutte 8 $cm^3$ de phénylacétylène, dilue avec 35 $cm^3$ de tétrahydrofuranne anhydre et laisse 1 heure à température ambiante. On ajoute, alors 6,45 g de 5α, 10α-époxy 3-oxo 17α-triméthylsilyloxy estr-9 (11)ène 17β-carbonitrile, agite pendant 4 heures à température ambiante, chauffe 1 heure à 35 °C. On refroidit et verse le mélange réactionnel dans une solution de chlorure d'ammonium, extrait à l'acétate d'éthyle, lave à l'eau, sèche, concentre à sec et obtient 8,9 g de produit. On le chromatographie sur silice en éluant par un mélange cyclohexane-acétate d'éthyle (9-1) et recueille 4,6 g de produit que l'on recristallise dans l'hexane et on obtient 3,47 g de produit attendu F = 176 °C.

Stade B : 3,3-éthylène dioxy 5α-hydroxy 10β-(phényléthynyl) estr 9 (11)-ène 17-one :

On agite 3,1 g de produit obtenu ci-dessus dans 31 $cm^3$ d'éthanol, ajoute 3,1 $cm^3$ de lessive de soude puis 10 $cm^3$ d'éthanol et poursuit l'agitation pendant 2 heures. On dilue avec 100 $cm^3$ d'eau, essore, lave à l'eau, sèche et obtient 2,49 g de produit attendu F = 199 °C.

Stade C : γ-lactone de l'acide 17β-hydroxy 3-oxo 10β-(phényléthynyl) 19-nor 17α-pregna 4,9 (11)diène-21-carboxylique :

On refroidit à — 70 °C, 10 $cm^3$ d'une solution 1,6 M de n-butyllithium dans l'hexane, dilue avec 10 $cm^3$ de tétrahydrofuranne anhydre puis ajoute goutte à goutte à — 70 °C 1,6 $cm^3$ de N,N,N',N'-tétraméthylphosphorodiamidate d'allyle dans 10 $cm^3$ de tétrahydrofuranne et laisse réagir pendant 1 heure à — 10 °C.

On ajoute 865 mg du produit obtenu ci-dessus, agite pendant 15 heures à température ambiante. On acidifie en ajoutant 10 $cm^3$ d'acide chlorhydrique 2N et 1 $cm^3$ d'acide chlorhydrique concentré et agite pendant 30 minutes. On extrait à l'acétate d'éthyle, lave à l'eau la phase organique, sèche et concentre à sec sous pression réduite. On ajoute 25 $cm^3$ de méthanol au résidu, puis 5 $cm^3$ d'acide chlorhydrique 6N chauffe à 50 °C pendant 1 heure. Après refroidissement on dilue à l'eau et extrait à l'acétate d'éthyle, lave

à l'eau la phase organique, sèche et concentre à sec sous pression réduite. On chromatographie le résidu sur silice en éluant par un mélange cyclo-hexane-acétate d'éthyle (1-1) et recueille 540 mg de produit attendu.

Après 2 cristallisations dans l'éther isopropylique puis l'éthanol aqueux le produit fond à 196 °C.

Exemple 6 : γ-lactone de l'acide 17β-hydroxy 10β-/3-hydroxy prop-1-ynyl/3-oxo 19-nor 17α-pregna 4,9 (11)-diène 21-carboxylique

Stade A : 3,3-éthylène dioxy 5α-hydroxy 10β-/3-(2RS-tétrahydropyrannyloxy) prop-1-ynyl/ méthylsilyloxy estr-9 (11)-ène 17β-carbonitrile :

Dans 30 $cm^3$ d'une solution éthérée de chlorure de propylmagnésium 0,9 M on ajoute goutte à goutte 4,4 $cm^3$ de 3-(2-tétrahydropyrannyloxy) 1-propyne dans 15 $cm^3$ d'éther anhydre. On dilue avec 20 $cm^3$ de tétrahydrofuranne et agite pendant 50 minutes à température ambiante. On introduit 1,32 g de 3,3-éthylène dioxy 5α, 10α-époxy 17α-triméthylsilyloxy estr-9 (11)ène-17β-carbonitrile et agite pendant 5 heures à température ambiante, chauffe 1 heure à 35 °C puis verse dans une solution diluée de phosphate monosodique. On extrait avec de l'acétate d'éthyle, lave à l'eau, sèche et concentre à sc. On chromatographie le résidu sur silice, élue par un mélange éther de pétrole-acétate d'éthyle (4-1) et obtient 1,335 g de produit attendu.

Stade B : 3,3-éthylène dioxy 5α-hydroxy 10β-/3-(2RS-tétrahydropyrannyloxy) prop-1-ynyl/ estr 9 (11)ène 17-one :

1,330 g de produit obtenu ci-dessus sont dissous dans 60 $cm^3$ de méthanol. On ajoute 3 $cm^3$ de lessive de soude et laisse réagir pendant 2 heures. On dilue avec 200 $cm^3$ d'eau, amorce la cristallisation, essore, lave à l'eau, sèche et obtient 900 mg de produit attendu F = 143 °C.

Stade C : γ-lactone de l'acide 5α, 17β-dihydroxy 3,3-éthylène dioxy 10β-/3-(2RS-tétrahydropyrannyloxy) prop-1-ynyl/ 19-nor 17α-pregna 9 (11)-ène-21-carboxylique :

A une solution de 12,5 $cm^3$ de butyllithium dans l'hexane à 1,6 M et refroidie à — 50 °C, on ajoute 12,5 $cm^3$ de tétrahydrofuranne anhydre puis ajoute à — 65 °C une solution de 2,2 $cm^3$ de tétraméthylphosphorotriamidate d'allyle dans 8 $cm^3$ de tétrahydrofuranne sec. On amène à — 10°C, — 15 °C et laisse agir pendant une heure puis ajoute 890 mg de produit obtenu ci-dessus et laisse réagir pendant 18 heures à température ambiante. On dilue à l'eau, extrait à l'acétate d'éthyle, lave à l'eau, sèche et concentre à sec. On chromatographie le résidu sur silice en éluant par un mélange benzène-acétate d'éthyle (7-3). On recueille 560 mg de produit attendu.

Stade D : γ-lactone de l'acide 17β-hydroxy 10β-/3-hydroxy prop-1-ynyl/ 3-oxo 19-nor 17α-pregna 4,9 (11)-diène-21-carboxylique :

On dissout 555 mg du produit obtenu ci-dessus dans 10 $cm^3$ de méthanol avec 5 $cm^3$ d'acide chlorhydrique 6 N et laisse réagir pendant 18 heures à température ambiante puis 1 heure à 60 °C. On refroidit, dilue à l'eau, extrait au chlorure de méthylène, lave à l'eau, sèche et concentre à sec sous pression réduite. On chromatographie le résidu sur silice élue avec un mélange cyclohexane-acétate d'éthyle (1-1). On sépare 350 mg de produit attendu.

Après recristallisation dans l'acétate d'éthyle le produit fond à 198 °C.

$/\alpha/_D = + 31° \pm 1$ (c = 1 %, $CHCl_3$).

Exemple 7 : γ-lactone de l'acide 10β-éthynyl 17β-hydroxy 3-oxo 19-nor 17α-pregna 4, 6, 9 (11)-triène 21-carboxylique

Stade A : γ-lactone de l'acide 3 éthoxy-10β-éthynyl 17β-hydroxy 19-nor 17α-pregna 3, 5, 9 (11)-triène 21-carboxylique :

A une solution de 6,77 g de produit obtenu à l'exemple 1 dans 68 $cm^3$ de dioxanne, on ajoute sous agitation et azote 20 $cm^3$ d'orthoformiate d'éthyle puis 203 mg d'acide paratoluènesulfonique monohydraté. Après 2 heures 30 on ajoute 0,6 $cm^3$ de triathylamine puis verse dans 60 $cm^3$ de solution aqueuse saturée de bicarbonate de sodium. On extrait le produit attendu au chlorure de méthylène et obtient 12,35 g de produit brut que l'on chromatoraphie sur silice (éluant : cyclohexane-acétate d'éthyle (7-3), triéthylamine 1 ‰). On obtient 6,1 g de produit attendu F = 196 °C.

Stade B : γ-lactone de l'acide 10β-éthynyl 17β-hydroxy 3-oxo 19-nor 17α-pregna 4, 6, 9 (11)-triène 21-carboxylique :

A une solution de 2,164 g de produit obtenu au stade A dans 43 $cm^3$ d'acétone à 5 % d'eau, on ajoute sous agitation 2,164 g de chloranile.

On agite 22 heures à température ambiante, précipite le mélange réactionnel dans 100 $cm^3$ d'un mélange eau saturée de bicarbonate de sodium-eau à 10 % de thiosulfate de sodium (1-1). On extrait au chlorure de méthylène, lave à l'eau, sèche et amène à sec. On obtient 2,1 g de produit brut que l'on chromatographie sur silice (éluant chlorure de méthylène-acétone 4-1) et isole 1,947 g de produit cristallisé que l'on empâte dans 10 $cm^3$ d'éther éthylique. Après essorage, lavage à léther éthylique, séchage sous vide on isole 1,847 g de produit que l'on dissout dans un mélange éthanol-chlorure de méthylène, on chasse le chlorure de méthylène, glace, essore, lave à l'éthanol et sèche. On obtient 1,751 g de produit.

$/\alpha/_D = 243{,}5° \pm 4{,}5°$ (c = 0,5 %, $CHCl_3$)

| | | |
|---|---|---|
| Analyse : | $C_{23}H_{24}O_3$ | |
| Calculé : | C 79,28 | H 6,94 % |
| Trouvé : | C 79,2 | H 7,0 % |

Exemple 8 : γ-lactone de l'acide 7α-(acétylthio) 10β-éthynyl 17β-hydroxy 3-oxo 19-nor 17α-pregna 4,9 (11)-diène 21-carboxylique

On agite 24 heures à température ambiante sous azote un mélange de 1,7 g de produit obtenu à l'exemple 7,85 $cm^3$ d'acide acétique, 3,4 $cm^3$ d'acide chlorhydrique concentré et 0,85 $cm^3$ d'acide thioacétique. On verse dans une solution aqueuse saturée de bicarbonate de sodium, extrait à l'acétate d'éthyle, lave à l'eau et obtient 2 g de résine que l'on chromatographie sur silice (éluant : cyclohexaneacétate d'éthyle 3-2) et isole 753 mg de produit attendu amorphe.

| | | | |
|---|---|---|---|
| Analyse : | $C_{25}H_{28}O_5S$ | | |
| Calculé : | C 70,72 | H 6,64 | S 7,55 % |
| Trouvé : | C 70,5 | H 6,7 | S 7,3 % |

Exemple 9 : γ-lactone de l'acide 10β-éthynyl 17β-hydroxy 3-oxo 7α-propyl 19-nor 17α-pregna 4,9 (11)-diène 21-carboxylique

a) Préparation du bromure de propylmagnésium :

A un mélange sous azote de 900 mg de magnésium et de 27 $cm^3$ de tétrahydrofuranne on ajoute en 30 minutes à 30 °C une solution de 2,7 $cm^3$ de bromure de n-propyle dans 2,7 $cm^3$ de tétrahydrofuranne. On agite une nuit à température ambiante sous azote.

b) Addition du magnésien :

A 14 $cm^3$ du magnésien précédent agité sous azote à — 40 °C on ajoute en 30 minutes la solution suivante : 1,514 g d'iodure cuivreux 0,69 g de bromure de lithium dans 3,7 $cm^3$ de tétrahydrofuranne et on rince avec 1 $cm^3$ de tétrahydrofuranne. On agite 15 minutes à — 40 °C la suspension obtenue. On refroidit à — 70 °C et ajoute en 10 minutes 1,05 $cm^3$ d'éthérate de trifluorure de bore. On agite 20 minutes à — 70 °C et ajoute en 35 minutes 923 mg de γ-lactone de l'acide 10β-éthynyl 17β-hydroxy 3-oxo 19-nor 17α pregna 4, 6, 9 (11)-triène 21-carboxylique préparé à l'exemple 7 en solution dans 50 $cm^3$ de tétrahydrofuranne. On rince avec 2 fois 5 $cm^3$ de tétrahydrofuranne, agite à — 70 °C. Après 45 minutes on ajoute 4 $cm^3$ d'acide chlorhydrique 5N. On laisse remonter à température ambiante puis agite 3 heures. On dilue à l'eau saturée de chlorure de sodium. On extrait à l'acétate d'éthyle, lave la phase organique à l'ammoniaque dilué puis à l'eau distillée, sèche et amène à sec sous vide et chromatographie le résidu sur silice (cyclohexaneacétate d'éthyle 7-3). On isole 480 mg de produit attendu. On dissout le produit dans le mélange chlorure de méthylène-éther isopropylique, distille le chlorure de méthylène et isole 460 mg de produit attendu.

F = 148 °C. $/\alpha/_D = +40° \pm 1°$ (c = 0,8 % dans l'éthanol)

| | | |
|---|---|---|
| Analyse : | $C_{26}H_{32}O_3 = 392{,}5$ | |
| Calculé : | C 79,55 | H 8,21 % |
| Trouvé : | C 79,6 | H 8,3 % |

Lors de la chromatographie on isole également 247 mg de l'isomère 7β le moins mobile que l'on recristallise de la même façon dans le mélange chlorure de méthylène-éther isopropylique et obtient 121 mg de produit F = 147 °C.

$/\alpha/_D = -14° \pm 2°$ (c = 0,5 % dans l'éthanol).

Exemple 10 : γ-lactone de l'acide 17β-hydroxy 3-oxo 10β-(1-propynyl) 19-nor 17α-pregna 4, 6, 9 (11)-triène 21-carboxylique

Stade A : γ-lactone de l'acide 3-éthoxy 17β-hydroxy 10β-(1-propynyl) 19-nor 17α-pregna 3, 5, 9 (11)-triène 21-carboxylique

On agite à température ambiante pendant 4 heures un mélange de 20,3 g de γ-lactone de l'acide 17β-hydroxy 3-oxo 10β-(1-propynyl) 19- nor 17α-pregna 4,9 (11)diène-21-carboxylique préparé à l'exemple 7 dans 200 $cm^3$ de dioxanne, 60 $cm^3$ d'orthoformiate d'éthyle et 0,6 g d'acide paratoluènesulfonique monohydraté. On ajoute 2 $cm^3$ de triéthylamine puis verse lentement dans 200 $cm^3$ de solution de carbonate acide de sodium. On extrait le précipité par du dichlorométhane. On obtient 31,1 g de produit que l'on dissout dans 100 $cm^3$ de dichlorométhane. A cette solution on ajoute lentement 250 $cm^3$ d'éther isopropylique puis glace. On obtient 5,8 g de cristaux F = 210 °C. Les liqueurs-mères sont concentrées et le résidu est purifié par chromatographie (éluant cyclohexane-acétate d'éthyle 7-3). On récupère ainsi 13,2 mg de produit identique F = 210 °C.

Stade B : γ-lactone de l'acide 17β-hydroxy 3-oxo 10β-(1-propynyl) 19-nor 17α-pregna 4, 6, 9 (11)-triène 21-carboxylique :

On agite à température ambiante pendant 22 heures le mélange de 5,8 g de produit obtenu au stade A, 116 $cm^3$ d'acétone à 5 % d'eau et 5,8 g de chloranile. On verse dans un mélange de 150 g d'une solution à 10 % de carbonate acide de sodium dans l'eau et de 150 g de solution de thiosulfate de sodium.

On extrait du dichlorométhane. On chasse les solvants et obtient 6,9 g de produit que l'on purifie par chromatographie sur silice (éluant acétate d'éthyle-éther de pétrole 1-1). On obtient 5,1 g de produit que l'on recristallise par dissolution dans 20 $cm^3$ de dichlorométhane et addition de 200 $cm^3$ d'oxyde d'isopropyle. On obtient ainsi 4,77 g de cristaux. F = 248 °C.

Exemple 11 γ-lactone de l'acide 10β-(1-butynyl) 17β-hydroxy 3-oxo 19-nor 17α-pregna 4,9 (11)-diène 21-carboxylique :

Stade A : 10β-(1-butynyl) 3,3-(éthylène dioxy) 5α-hydroxy estr-9 (11)ène 17-one

On fait barboter pendant 2 heures 30 de l'éthylacétylène dans un mélange de 330 $cm^3$ de bromure d'éthylmagnésium en solution éthérée 0,5 N et 330 $cm^3$ de tétrahydrofuranne puis introduit en une seule fois 10 g de 3,3-éthylène dioxy 5α, 10α-époxy 17α-triméthylsilyloxy estr-9 (11)ène 17β-carbonitrile. On chauffe à 40-50 °C pendant 4 heures 30, ramène à température ambiante, verse dans un mélange chlorure d'ammonium-glace, décante, lave à l'eau, extrait au chloroforme, sèche puis amène à sec. Les 12 g de produit obtenus sont dissous dans 100 $cm^3$ d'éthanol à 95 °C, on ajoute 10 $cm^3$ de lessive de soude, agite 45 minutes, distille l'éthanol, reprend au chloroforme, lave à l'eau, sèche puis amène à sec. Les 9,3 g de produit ainsi obtenus sont chromatographiés sur silice (éluant : éther de pétrole-acétate d'éthyle (1-1) à 1 ‰ de triéthylamine). On recueille 4,6 g de produit Rf. = 0,35. Ce produit est dissous dans 10 $cm^3$ de chlorure de méthylène. On filtre, ajoute 30 $cm^3$ d'éther isopropylique, glace puis essore et sèche à 50 °C. On obtient 240 mg de produit F = 139 °C.

Analyse : $C_{24}H_{32}O_4$ = 384,52
Calculé : C 74,97 H 8,39 %
Trouvé : C 75,0 H 8,5 %

Stade B : γ-lactone de l'acide 10β-(1-butynyl) 17β-hydroxy 3-oxo 19-nor 17α-pregna 4,9 (11)-diène 21-carboxylique :

On ajoute à —50 °C, 24 $cm^3$ de tétrahydrofuranne à 24 $cm^3$ de n-butyllithium en solution dans l'hexane à 1,65 M puis ajoute en 15 minutes à —50 °C, 4 $cm^3$ de tétraméthylphosphorodiamidate d'allyle en solution dans 16 $cm^3$ de tétrahydrofuranne. On agite pendant 1 heure à —30 °C et introduit 1,92 g de produit obtenu au stade A en solution dans 20 $cm^3$ de tétrahydrofuranne.

On laisse refroidir à température ambiante, agite pendant 2 heures, ajoute entre 0 et + 5 °C 40 $cm^3$ d'acide chlorhydrique 2 N, agite 30 minutes, extrait au chloroforme, lave à l'eau, sèche et amène à sec. On obtient 3 g de produit que l'on dissout dans 30 $cm^3$ d'éthanol, ajoute sous azote 6 $cm^3$ d'acide chlorhydrique au demi et chauffe à 50 °C pendant 2 heures. On amène à sec, reprend au chlorure de méthylène, lave à l'eau puis amène à sec. On obtient 2 g de produit que l'on chromatographie sur silice en éluant par de l'éther de pétrole-acétate d'éthyle 1-1. On obtient 1,26 g de produit Rf. = 0,22. Le produit est dissous dans 20 $cm^3$ d'éther isopropylique au reflux. On concentre au demi, amorce la cristallisation, glace, essore, lave à l'éther isopropylique glacé puis sèche à 50 °C sous vide. On obtient 880 mg de produit que l'on recristallise en le dissolvant dans 8 $cm^3$ d'éther isopropylique au reflux, filtre, concentre jusqu'à début de trouble, amorce à chaud, glace, essore, lave à l'éther de pétrole puis sèche à 50 °C sous vide. On obtient 770 mg de produit. F = 94 °C.

Analyse : $C_{25}H_{30}O_3$ = 378,48
Calculé : C 79,33 H 7,99 %
Trouvé : C 79,5 H 8,2 %
$/\alpha/_D$ = = 35° ± 1° (c = 1 %, chloroforme)

Exemple 12 : γ-lactone de l'acide 17β-hydroxy 3-oxo 10β-(1-pentynyl) 19-nor 17α-pregna 4,9 (11)-diène 21-carboxylique :

Stade A : 3,3-(éthylènedioxy) 5α-hydroxy 10β-(1-pentynyl) estr 9 (11)-ène-17-one :

On opère comme au stade A de l'exemple 11 à partir de 6,45 g de 3,3-éthylène dioxy 5α, 10α-époxy 17α-triméthylsilyloxy estr-9 (11) ène 17β-carbonitrile et obtient après chromatographie 2,61 g de produit attendu. Une recristallisation de 200 mg de ce produit dans 2 $cm^3$ d'éther isopropylique au reflux donne 140 mg de produit attendu pur F = 120 °C.

Analyse : $C_{25}H_{35}O_4$ = 398,55
Calculé : C 75,34 H 8,6 %
Trouvé : C 75,1 H 8,7 %

Stade B : γ-lactone de l'acide 17β-hydroxy 3-oxo 10β-(1-pentynyl) 19-nor 17α-pregna 4,9 (11)-diène 21-carboxylique :

On opère comme au stade B de l'exemple 11 à partir de 2 g de produit obtenu au stade A. Après chromatographie, on obtient 1,13 g de produit attendu. Ce produit est recristallisé par dissolution dans 2 $cm^3$ de chlorure de méthylène et addition de 10 $cm^3$ d'éther isopropylique. On obtient 970 mg de produit pur puis 910 mg lors d'une seconde recristallisation. F = 123 °C.

Analyse : $C_{26}H_{32}O_3$ = 392,52
Calculé : C 79,55 H 8,22 %
Trouvé : C 79,6 H 8,3 %
$/\alpha/_D$ = = 41° ± 1° (c = 1 %, chloroforme)

Exemple 13 : 17β-hydroxy 10β-(but-1-ynyl) estr 4,9 (11) diène-3-one

a) Réduction de la cétone en 17 :
On ajoute 385 mg de borohydrure de sodium à une solution de 770 mg de 10β-(but-1-ynyl) 3,3-(éthylène dioxy) 5α-hydroxy estr 9 (11)-ène 17-one préparé au stade A de l'exemple 11 dans 15 $cm^3$ d'éthanol. On agite 45 minutes, distille à sec sous vide, reprend au chlorure de méthylène, lave à l'eau, sèche puis amène à sec. On obtient 770 mg de produit. F = 195 °C.
b) Hydrolyse acide du cétal :
Les 770 mg de produit obtenus ci-dessus sont dissous sous azote à 50-60 °C dans 15 $cm^3$ d'éthanol. On ajoute 0,8 $cm^3$ d'acide chlorhydrique au demi. On chauffe pendant 4 heures, amène à sec reprend au chlorure de méthylène, lave à l'eau, sèche puis amène à sec. On obtient 750 mg de résine que l'on chromatographie sur silice (éluant éther de pétrole-acétate d'éthyle 1-1) Rf. = 0,33. On obtient 630 mg de produit que l'on recristallise en le dissolvant dans 6 $cm^3$ d'éther isopropylique au reflux, puis on filtre, concentre à moitié, amorce, glace, essore, lave à l'éther isopropylique glacé puis sèche. On obtient 485 mg de produit. F = 95 °C.
$/\alpha/_D$ = = 136° ± 2° (c = 0,5 %, chloroforme).

Exemple 14 : 17β-hydroxy 10β-(pent-1-ynyl) estr 4,9 (11) diène-3-one

On opère comme indiqué à l'exemple 13 à partir de 570 mg de 10β-(pent-1-ynyl) 3,3 (éthylène dioxy) 5α-hydroxy estra 9 (11) ène 17-one préparé au stade A de l'exemple 12. On obtient 280 mg de produit attendu.
$/\alpha/_D$ = = 134° ± 2° (c = 0,5 %, chloroforme)

Exemple 15 : 17β-hydroxy 10β-(prop-1-ynyl) estr 4,9 (11) diène 3-one

On opère comme indiqué à l'exemple 13 à partir de 520 mg de 10β (prop-1-ynyl) 3,3 (éthylène dioxy) 5α-hydroxy estr-9 (11) ène 17-one préparé au stade A de l'exemple 4. On obtient 260 mg de produit attendu F = 154 °C.
$/\alpha/_D$ = 130° ± 1° (c = 1 % chloroforme)

Exemple 16 : γ-lactone de l'acide 17β-hydroxy 3-oxo 7α-propyl 10β (1-propynyl) 19-nor 17α-pregna 4,9 (11) diène 21-carboxylique

En opérant comme à l'exemple 9 au départ du produit de l'exemple 10, on obtient le produit attendu. F = 110 °C Rf. = 0,2 (éluant cyclohexane-acétate d'éthyle : 7-3).

Analyse : $C_{27}H_{34}O_5$
Calculé : C 79,77 H 8,43 %
Trouvé : C 76,6 H 8,5 %

Exemple 17 : γ-lactone de l'acide 7α-acétylthio 17β-hydroxy 3-oxo 10β-(1-propynyl) 19-nor 17α-pregna 4,9 (11) diène 21-carboxylique

En opérant comme à l'exemple 8 au départ du produit de l'exemple 10, on obtient le produit attendu. Rf = 0,1 (éluant : dichlorométhane-acétone 9-2).

Analyse : $C_{26}H_{30}SO_4$ = 458,59
Calculé : C 71,20 H 6,89 S 7,31 %
Trouvé : C 71 H 6,8 S 7,2 %

Exemple 18 : 17β-(3-hydroxy 1-propynyl) 17β-hydroxy estra 4,9 (11) dièn-3-one

a) Réduction de la cétone en 17.

On ajoute 58 mg de borohydrure de sodium à une solution refroidie à + 5 °C comprenant 290 mg de 3,3-éthylènedioxy 5α-hydroxy 10β-/3-(2RS-tétrahydropyrannyloxy) 1-propynyl/ estr-9 (11)-èn-17-one préparé comme à l'exemple 6 stade B et 6 cm³ de méthanol. On laisse 2 heures sous agitation, dilue à l'eau, extrait au chlorure de méthylène, concentre à sec et obtient 300 mg de produit.

b) Hydrolyse acide du cétal.

On reprend le produit ci-dessus dans 6 cm³ de méthanol, ajoute 3 cm³ d'acide chlorhydrique au demi, agite pendant 1 heure, dilue à l'eau, amorce la cristallisation, essore, sèche et obtient 166 mg de produit attendu. F = 204 °C après recristallisation dans un mélange chlorure de méthylène-éther isopropylique.

/α/$_D$ = ± 124° (c = 1 %, chloroforme).

Exemple 19 : γ-lactone de l'acide 17β-hydroxy 3-oxo 10β-(1-propynyl) 19-nor 17α-pregna 1,4,9 (11) trièn-21-carboxylique

On chauffe 20 heures au reflux 2 g de produit obtenu à l'exemple 4 en suspension dans 20 cm³ de toluène, en présence de 3,2 g d'anhydride phényl séléninique, laisse refroidir, verse sur une solution de bicarbonate de sodium, extrait à l'acétate d'éthyle, lave avec une solution aqueuse d'hydroxyde de sodium, sèche et évapore les solvants sous pression réduite. Après chromatographie sur silice (éluant : chlorure de méthylène-acétone 97-3), on obtient 0,511 g de produit attendu.

Spectre IR (chloroforme)

1 764 cm⁻¹ : γ-(lactone ; 1 667 cm⁻¹ : cétone conjuguée ; 1 631-1 608 cm⁻¹ : —C≡C—.

Exemple 20 : γ-lactone de l'acide 17β-hydroxy 7α-méthyl 3-oxo 10β-(1-propynyl) 19-nor 17α-pregna 4,9 (11)-dièn-21-carboxylique

On mélange 75 mg de chlorure cuivrique et 24 mg de chlorure de lithium dans 55 cm³ de tétrahydrofuranne et ajoute sous atmosphère inerte 2 g de produit obtenu à l'exemple 10. On refroidit à + 2°/+ 4 °C et ajoute goutte à goutte en 2 heures 7,9 cm³ d'une solution éthérée d'iodure de méthyl magnésium 1,05M, agite 2 heures en maintenant la température à + 2 °C, ajoute (cm³ d'acide chlorhydrique 5N et laisse revenir à température ambiante. On extrait le milieu réactionnel à l'acétate d'éthyle, lave à l'eau, puis avec une solution aqueuse d'hydroxyde d'ammonium, puis à l'eau, sèche et concentre à sec. Après chromatographie sur silice (éluant : cyclohexane-acétate d'éthyle 8,5-1,5), on obtient 384 mg d'isomère 7α, F = 125 °C et 550 mg d'isomère 7β du produit attendu.

Analyse : $C_{25}H_{30}O_3$ : 378,52
isomère 7α : Calculé : C 79,33 H 7,99 %
Trouvé : C 79,0 H 8,1 %
isomère 7β : Calculé : C 79,33 H 7,99 %
Trouvé : C 79,3 H 8,0 %

Exemple 21 : γ-lactone de l'acide (Z) 17β-hydroxy 3-oxo 10β-(1-propényl) 19-nor 17α-pregna 4,9 (11)-dièn-21-carboxylique

On ajoute 8 cm³ de pyridine puis 0,29 g de catalyseur de Lindlar à une solution de 1,45 g de produit obtenu à l'exemple 4 dans 32 cm³ d'acétate d'éthyle. On hydrogène pendant 2 heures, essore le catalyseur, élimine la pyridine par lavage à l'acide chlorhydrique 2N, décante la phase organique, la lave à

l'eau, la sèche et la concentre à sec. On reprend le résidu dans l'éther, chauffe au reflux, refroidit, essore les cristaux, les sèche et obtient 840 mg de produit attendu. F = 170 °C après recristallisation dans un mélange chlorure de méthylène-éther isopropylique.
/α/$_D$ = — 47° (c = 1 %, chloroforme).

Analyse : $C_{24}H_{30}O_3$ : 366,51
Calculé : C 78,65 H 8,25 %
Trouvé : C 78,4 H 8,4 %

Exemple 22 : γ-lactone de l'acide 10β-(3-chloro 1-propynyl) 17β-hydroxy 3-oxo 19-nor 17α-pregna 4,9 (11)-dièn-21-carboxylique

On ajoute 1 g de triphénylphosphine à une solution de 0,5 g de produit obtenu à l'exemple 6 dans 5 cm$^3$ de tétrahydrofuranne et 5 cm$^3$ de tétrachlorure de carbone. On chauffe 30 minutes à 80 °C sous agitation, concentre à sec sous pression réduite, chromatographie le résidu sur silice (éluant : cyclohexane-acétate d'éthyle 9-1 puis 7-3). On purifie le produit par chromatographie sur silice en éluant à l'éther et obtient 0,38 g de produit attendu. F = 134 °C après recristallisation dans un mélange chlorure de méthylène-éther isopropylique.
/α/$_D$ = = 17,5° (c = 1 %, chloroforme).

Analyse : $C_{24}H_{27}O_3Cl$ : 398,93
Calculé : C 72,26 H 6,82 Cl 8,88 %
Trouvé : C 72,1 H 6,8 Cl 9,2 %

Exemple 23 : (6α, 7α, 17α) γ-lactone de l'acide 6,7-dihydro 17β-hydroxy 3-oxo 10β-(1-propynyl) 3'H-cyclopropa-(6,7)-19-nor pregna 4,9 (11)-dièn-21-carboxylique

Dans 4,04 g d'iodure de triméthyl sulfoxonium en solution dans 15 cm$^3$ de diméthylsulfoxyde, on ajoute sous atmosphère inerte 8,6 cm$^3$ de terbutylate de potassium en solution dans 8,2 cm$^3$ de tétrahydrofuranne, agite 15 minutes, ajoute 2,737 g de produit obtenu comme à l'exemple 10 dans 3 cm$^3$ de tétrahydrofuranne et chauffe à 59°/60 °C pendant 1 heure. On glace, ajoute 8 cm$^3$ d'acide chlorhydrique N, dilue avec une solution aqueuse saturée de chlorure de sodium, extrait à l'acétate d'éthyle, lave la phase organique avec une solution aqueuse saturée de bicarbonate de sodium, puis avec une solution aqueuse saturée de chlorure de sodium. On sèche, concentre à sec, purifie par chromatographie sur silice (éluant : cyclohexane-acétate d'éthyle 6-4) et obtient 1,28 g de produit attendu. F = 243 °C après recristallisation dans l'isopropanol.
/α/$_D$ = — 276° (c = 0,5 %, $CHCl_3$).

Analyse :
Calculé : C 79,75 H 7,49 %
Trouvé : C 79,4 H 7,6 %

Exemple 24 : 13β-éthyl 10β-éthynyl 17β-hydroxy gona 4,9 (11)-dièn-3-one

Stade A : 13β-éthyl 3,3-éthylènedioxy 10β-éthynyl gon-9 (11)-ène 5α, 17β-diol.

On dissout 3,9 g de 3,3-éthylènedioxy 5α, 10α-époxy, 13β-éthyl 17β-hydroxy gon-9 (11)-ène préparé comme dans le brevet français 2 377 417 dans du benzène, concentre sous pression réduite et reprend le résidu dans 40 cm$^3$ d'éthylène diamine. On ajoute sous atmosphère inerte 3,6 g de complexe acétylure de lithium-éthylène diamine, chauffe à 45 °C et maintient 24 heures sous agitation, ajoute de nouveau 3,9 g de complexe, agite 72 heures à 48 °C, refroidit et verse dans une solution de chlorure d'ammonium. On extrait à l'acétate d'éthyle, lave la phase organique à l'eau, la sèche et chasse les solvants sous pression réduite. On chromatographie le résidu sur silice (éluant : cyclohexane-acétate d'éthyle 1-1) et obtient 2,3 g de produit attendu.

Stade B : 13β-éthyl 10β-éthynyl 17β-hydroxy gona 4,9 (11)-dièn-3-one.

On ajoute 3,5 cm$^3$ d'acide chlorhydrique au demi à 0,7 g de produit obtenu au stade A dans 7 cm$^3$ de méthanol. On chauffe à 55 °C pendant 1 heure, dilue à l'eau et extrait au chlorure de méthylène. On lave la phase organique à l'eau, sèche, concentre à sec sous pression réduite, chromatographie le résidu sur silice (éluant : cyclohexane-acétate d'éthyle (1-1), purifie le produit obtenu par une nouvelle chromatographie sur silice (éluant : éther-hexane 6-4) et recueille 0,39 g de produit attendu. F = 120 °C après recristallisation dans l'éther isopropylique.
/α/$_D$ = + 98,5° (c = 1 %, chloroforme).

Analyse : $C_{21}H_{26}O_2$ : 310,44
Calculé : C 81,25 H 8,44 %
Trouvé : C 81,1 H 8,7 %

Exemple 25 : γ-lactone de l'acide 13β-éthyl 10β-éthynyl 17β-hydroxy 3-oxo 18,19-dinor (17α)-pregna-4,9(11) dièn-21-carboxylique

Stade A : 17β-éthyl 10β-éthynyl 3,3-éthylènedioxy 5α-hydroxy gon-9 (11)-èn-17-one.

On ajoute 2,4 g de dichromate de pyridinium dans une solution refroidie à + 4 °C de 0,75 g de produit obtenu au stade A de l'exemple 24 dans 12 cm³ de diméthylformamide. On agite 1 heure 30 minutes, dilue à l'eau et extrait à l'éther. On lave la phase organique à l'eau, la sèche et la concentre à sec. On obtient 0,74 g de produit attendu.

Stade B : γ-lactone de l'acide 13β-éthyl 10β-éthynyl 17β-hydroxy 3-oxo 18,19-dinor (17α)-pregna-4,9 (11) dièn-21-carboxylique.

On opère comme au stade B de l'exemple 4 à partir de 0,95 g de produit obtenu au stade A précédent et en laissant réagir pendant 65 heures à température ambiante. On verse le milieu réactionnel dans une solution de chlorure d'ammonium et extrait à l'acétate d'éthyle. On lave la phase organique à l'eau, la sèche et la concentre à sec sous pression réduite. On reprend le résidu dans 18 cm³ de méthanol, ajoute 9 cm³ d'acide chlorhydrique au demi, chauffe 1 heure 30 minutes à 50°/55 °C, refroidit, dilue à l'eau, extrait au chlorure de méthylène, purifie le produit brut obtenu par chromatographie sur silice (éluant : étheracétate d'éthyle 8,5-1,5) et obtient 0,26 g de produit attendu. F = 198 °C après recristallisation dans l'éther isopropylique.
$/\alpha/_D = +4°$ (c = 1 %, chloroforme).

Analyse : $C_{24}H_{28}O_3$ : 364,49
Calculé : C 79,08 H 7,74 %
Trouvé : C 79,1 H 7,8 %

Exemple 26 : 10β-éthynyl 17β-hydroxy 17α-méthyl estra 4,9 (11) dièn-3-one

Stade A : 3,3-éthylènedioxy 10β-éthynyl 5α, 17β-dihydroxy 17α-méthyl estr-9 (11)-ène.

On ajoute en 15 minutes à — 65 °C 27 cm³ d'une solution de méthyl lithium dans l'éther (1,8M) à une suspension de 3,05 g d'iodure de cuivre dans 30 cm³ de tétrahydrofuranne. On agite 15 minutes, ajoute 713 mg de 3,3-éthylènedioxy 10β-éthynyl 5-hydroxy estra 9 (11)-èn-17-one en solution dans 20 cm³ de tétrahydrofuranne, maintient sous agitation 2 heures à — 65 °C et laisse revenir à température ambiante. On agite 39 heures, glace, ajoute une solution aqueuse saturée en chlorure d'ammonium, extrait à l'acétate d'éthyle, lave la phase organique à l'eau, sèche et concentre à sec. On chromatographie le résidu sur silice (éluant : cyclohexane-acétate d'éthyle 6-4) et obtient 560 mg de produit attendu.

Stade B : 10β-éthynyl 17β-hydroxy 17α-méthyl estra 4,9 (11) dièn-3-one

On chauffe 3 heures 15 minutes sous atmosphère inerte 704 mg de produit préparé comme au stade A précédent, dans 14 cm³ d'éthanol et 3,5 cm³ d'acide chlorhydrique 5N. On glace, ajoute 2 m³ d'ammoniaque, chasse l'éthanol sous pression réduite, dilue à l'eau et extrait à l'acétate d'éthyle. On lave la phase organique à l'eau, sèche et concentre à sec. On chromatographie le résidu sur silice (éluant : cyclohexane-acétate d'éthyle 6-4) et obtient 472 mg de produit attendu. F = 174 °C après cristallisation dans l'éther éthylique.
$/\alpha/_D = +65,5° \pm 2°$ (c = 0,5 %, chloroforme).

Analyse :
Calculé : C 81,24 H 8,44 %
Trouvé : C 81,2 H 8,3 %

Exemple 27 : 10β-éthynyl 17β-hydroxy 17α-éthyl estra 4,9 (11) dièn-3-one

Stade A : 3,3-éthylènedioxy 10β-éthynyl 5α-, 17β-dihydroxy 17α-éthyl estr 9 (11) ène.

On ajoute 100 mg de bromure cuivreux dans 40 cm³ d'une solution de bromure de méthyl magnésium (0,5M) refroidie à + 10 °C. On agite 20 minutes, ajoute 1 g de produit préparé à l'exemple 1 stade A et maintient l'agitation pendant 2 heures 30 minutes. On verse sur une solution aqueuse saturée en chlorure d'ammonium à 0 °C, extrait au chlorure de méthylène, concentre à sec et obtient 1,04 g de produit attendu.

Stade B : 10β-éthynyl 17β-hydroxy 17α-éthyl estra 4,9 (11) dièn-3-one.

On agite 16 heures à température ambiante puis 3 heures à 40 °C 971 mg de produit obtenu au stade A précédent dans 20 $cm^3$ d'éthanol et 10 $cm^3$ d'acide chlorhydrique 2N. On refroidit, neutralise à l'ammoniaque et extrait au chlorure de méthylène. On évapore le solvant, chromatographie le résidu sur silice (éluant : hexane-acétate d'éthyle 6-4) et obtient après cristallisation dans l'éther éthylique 372 mg de produit attendu. F = 147-148 °C.
$/\alpha/_D$ = + 56° + 5° (c = 0,2 %, chloroforme).

Exemple 28 : 10β-éthynyl 17β-hydroxy 17α-propyl estra 4,9 (11) dièn-3-one

Stade A : 3,3-éthylènedioxy 10β-éthynyl 5α, 17β-dihydroxy 17α-propyl estra 9 (11) ène.

On opère comme au stade A de l'exemple 27 à partir de 465 mg de bromure cuivreux, de 90 $cm^3$ d'une solution tétrahydrofurannique de bromure d'éthyl d'ammonium (0,7M) et de 4,650 g de produit préparé comme au stade A de l'exemple 1. Après extraction à l'acétate d'éthyle et purification par chromatographie sur silice (éluant : cyclohexane-acétate d'éthyle), on obtient 4,375 g de produit attendu.

Stade B : 10β-éthynyl 17β-hydroxy 17α-propyl estra 4,9 (11) dièn-3-one.

On ajoute 21 $cm^3$ d'acide chlorhydrique 5N dans une solution de 4,2 g de 3,3-éthylènedioxy 10β-éthynyl 5α, 17β-dihydroxy 17α-propyl estra 9 (11) ène obtenu au stade A précédent dans 130 $cm^3$ d'éthanol et chauffe 2 heures 30 minutes à 55 °C environ sous agitation et atmosphère inerte. On concentre partiellement, dilue à l'acétate d'éthyle, lave à l'eau, sèche et concentre à sec. On chromatographie le résidu sur silice (éluant : cyclohexane-acétate d'éthyle 8-2) et obtient 2,8 g de produit attendu. F = 167 °C après recristallisation dans l'isopropanol.
$/\alpha/_D$ = + 58,5° + 2,5° (c = 0,5 %, chloroforme).

Exemple 29 : 10β-éthynyl 17β-hydroxy 17α-butyl estra 4,9 (11) dièn-3-one

Stade A : 3,3-éthylènedioxy 10β-éthynyl 5α, 17β-dihydroxy 17α-butyl estr 9 (11) ène.

On agite 30 minutes à + 7 °C 100 mg de bromure cuivreux dans 18 $cm^3$ d'une solution tétrahydrofurannique de bromure de n-propyl magnésium et ajoute 1 g de produit préparé comme au stade A de l'exemple 1 en solution dans 10 $cm^3$ de tétrahydrofuranne. On laisse revenir à température ambiante, agite 45 minutes et verse sur une solution aqueuse saturée en chlorure d'ammonium glacée, extrait à l'acétate d'éthyle, lave la phase organique à l'eau, puis avec une solution aqueuse saturée de chlorure de sodium, concentre à sec, purifie le résidu par chromatographie sur silice (éluant : cyclohexane-acétate d'éthyle 6-4 à 1 ‰ de triéthylamine). On obtient après cristallisation dans l'éther 1 g de produit attendu. F ≃ 130 °C.

Stade B : 10β-éthynyl 17β-hydroxy 17α-butyl estra 4,9 (11) dièn-3-one.

On agite 6 heures à 40 °C puis 16 heures à température ambiante 870 mg de produit obtenu au stade A dans 26 $cm^3$ d'éthanol et 34 $cm^3$ d'acide chlorhydrique 2N. On verse dans de l'eau glacée, alcalinise avec de l'ammoniaque concentrée, extrait à l'acétate d'éthyle, lave la phase organique à l'eau puis avec une solution aqueuse saturée de chlorure de sodium, sèche et concentre à sec. On chromatographie le résidu sur silice (éluant : cyclohexane-acétate d'éthyle 6-4) et obtient après recristallisation dans l'éther isopropylique 554 mg de produit attendu. F = 134 °C.
$/\alpha/_D$ = + 45,5° + 2° (c = 0,5 %, chloroforme).

Analyse :

| | | |
|---|---|---|
| Calculé : | C 81,77 | H 9,15 % |
| Trouvé : | C 82,0 | H 9,2 % |

Exemple 30 : 10β-éthynyl 17β-hydroxy 17α-(2-propén-1-yl) estra 4,9 (11) dièn-3-one

Stade A : 3,3-éthylènedioxy 10β-éthynyl 5α, 17β-dihydroxy 17α-(2-propén-1-yl) estr 9 (11) ène.

On introduit en 10 minutes une solution comprenant 1 g de 3,3-éthylènedioxy 10β-éthynyl 5α-hydroxy estr 9 (11) èn-17-one et 8 $cm^3$ de tétrahydrofuranne dans 15,6 $cm^3$ d'une solution tétrahydrufurannique de chlorure d'allyle magnésium 0,9M et agite pendant 4 heures. On verse sur une solution aqueuse saturée en chlorure d'ammonium à 0 °C, extrait au chlorure de méthylène, chasse le solvant, purifie par

chromatographie sur silice (éluant : hexane-acétate d'éthyle 1-1 à 1 % de triéthylamine) et obtient 987 mg de produit attendu.

Stade B : 101β 17β-hydroxy 17α-(2-propén-1-yl) estra 4,9 (11) dièn-3-one.

On agite 2 heures à température ambiante puis 3 heures à 50 °C 940 mg de produit obtenu au stade A précédent dans 20 $cm^3$ et 5 $cm^3$ d'acide chlorhydrique 5N. On refroidit, neutralise à l'ammoniaque et extrait au chlorure de méthylène. On évapore le solvant, chromatographie le résidu sur silice (éluant : cyclohexane-acétate d'éthyle 7-3) et obtient après cristallisation dans l'éthanol 475 mg de produit attendu. F = 150 °C ;

Analyse : $C_{23}H_{28}O_2$ : 336,477
Calculé : C 81,9 H 8,60 %
Trouvé : C 82,1 H 8,39 %

Exemple 31 : 10β-éthynyl 17β-hydroxy 17α-(3-hydroxy propyl) estra 4,9 (11) dièn-3-one

Stade A : 3,3-éthylènedioxy 10β-éthynyl 5α,17β-dihydroxy 17α-/3-(1,1-diméthyléthoxy) propyl/estr 9 (11) ène.

1) Préparation du magnésien.
On chauffe à 60 °C sous atmosphère inerte 16 g de magnésium dans 200 $cm^3$ de tétrahydrofuranne. On ajoute quelques gouttes de 1,2-dibromoéthane puis en 30 minutes 49,7 g de 3-(1,1-diméthyléthoxy) chloropropane en solution dans 100 $cm^3$ de tétrahydrofuranne et chauffe 3 heures au reflux.
2) Condensation.
On chauffe au reflux sous atmosphère inerte 64 $cm^3$ de magnésien préparé ci-dessus dans 64 $cm^3$ d'éther éthylique, ajoute 4 g de 3,3-éthylènedioxy 10β-éthynyl 5α-hydroxy estr 9 (11) èn-17-one, maintient 42 heures au reflux, en ajoutant en 2 fois 96 $cm^3$ du magnésien et 96 $cm^3$ d'éther éthylique. On refroidit le milieu réactionnel, ajoute une solution aqueuse saturée de chlorure d'ammonium, filtre et concentre à sec le filtrat. On chromatographie le résidu sur silice (éluant : cyclohexane-acétate d'éthyle 7-3) et obtient 2,87 g de produit attendu.

Stade B : 10β-éthynyl 17β-hydroxy 17α-/3-(1,1-diméthyléthoxy) propyl/estra 4,9 (11) dièn-3-one

On opère comme au stade B de l'exemple 26 à partir de 1,5 g de produit préparé au stade A précédent dans 45 $cm^3$ d'éthanol et 7,5 $cm^3$ d'acide chlorhydrique 5N. On obtient après purification par chromatographie sur silice (éluant : cyclohexane-acétate d'éthyle 7-3) 0,8 g de produit attendu.

Stade C : 10β-éthynyl 17β-hydroxy 17α-(3-hydroxy propyl) estra 4,9 (11) dièn-3-one

On agite 2 heures 30 minutes à température ambiante sous atmosphère inerte 100 mg de produit obtenu au stade B précédent en solution dans 0,2 $cm^3$ de dioxanne et 0,3 $cm^3$ d'acide chlorhydrique concentré. On verse dans une solution aqueuse saturée de carbonate acide de sodium, extrait à l'acétate d'éthyle, lave la phase organique à l'eau, la sèche et la concentre à sec sous pression réduite. On chromatographie le résidu sur silice (éluant : chlorure de méthylène-méthanol 95-5) et obtient 20 mg de produit attendu. F = 144 °C après cristallisation dans l'éther isopropylique puis le 2,2-diméthoxypropane.
$/\alpha/_D = + 36{,}5° \pm 2°$ (c = 0,5 %, chloroforme).

Analyse :
Calculé : C 77,92 H 8,53 %
Trouvé : C 77,8 H 8,7 %

Exemple 32 : 4',5'-dihydro 10β-éthynyl spiro (estra 4,9 (11) dièn -17,2' (3H) furan)-3-one

On ajoute en 5 minutes sous atmosphère inerte 122 mg de chlorure de tosyle à une solution de 115 mg de produit préparé comme à l'exemple 31 dans 2,3 $cm^3$ de pyridine et agite 15 heures à température ambiante. On verse dans de l'eau additionnée d'acide chlorhydrique, extrait à l'acétate d'éthyle, lave la phase organique à l'eau, sèche et concentre à sec. On obtient 100 mg de produit brut que l'on purifie par chromatographie sur silice (éluant : cyclohexane-acétate d'éthyle 8-2). F = 126 °C après cristallisation dans l'éther isopropylique.
$/\alpha/_D = + 17° \pm 2°$ (c = 0,5 % chloroforme).

Analyse :
Calculé : C 82,10 H 8,38 %
Trouvé : C 82,3 H 8,6 %

Exemple 33 : 10β-éthynyl 17β-acétoxy 17α-(3-hydroxypropyl) estra 4,9 (11) dièn-3-one

Stade A : 10β-éthynyl 17β-acétoxy 17α-(3-acétoxypropyl) estra 4,9 (11) dièn-3-one.

On agite 1 heure 30 minutes sous atmosphère inerte 100 mg de produit obtenu comme au stade B précédent, 0,6 $cm^3$ d'anhydride acétique et 5 mg d'acide paratoluènesulfonique. On verse sur de la glace, ajoute une solution aqueuse saturée de bicarbonate de sodium, sèche et concentre à sec sous pression réduite en éliminant l'acide acétique résiduel par entraînement au toluène. On chromatographie le résidu sur silice (éluant : chlorure de méthylène-acétone 9-1) et obtient 98 mg de produit attendu.

Stade B : 10β-éthynyl 17β-acétoxy 17α-(3-hydroxypropyl) estra 4,9 (11) dièn-3-one.

On chauffe au reflux sous atmosphère inerte pendant 1 heure, 70 mg de produit obtenu au stade C précédent, 17 mg de bicarbonate de potassium dans 1 $cm^3$ de méthanol. On verse dans un mélange d'eau et de glace, extrait au chlorure de méthylène, lave la phase organique à l'eau, puis avec une solution aqueuse saturée de chlorure de sodium, sèche et concentre à sec sous pression réduite. On chromatographie le résidu sur silice (éluant : chlorure de méthylène-méthanol 95-5) et obtient 60 mg de produit attendu.

$/\alpha/_D = + 10° \pm 1°$ (c = 1,2 %, chloroforme).

Exemple 34 : 3-oxo 10β-(1-propynyl) 17β-hydroxy 19-nor 17α-pregna 4,9 (11) dièn-21-carboxylate de potassium

On chauffe au reflux 30 minutes sous atmosphère d'azote 1,69 g de produit préparé à l'exemple 4 dans 21,2 $cm^3$ de potasse méthanolique (0,2 N) et 21,2 $cm^3$ d'eau. On concentre à sec à 50 °C sous pression réduite, élimine les traces d'eau par entraînement à l'éthanol. On reprend le résidu à l'éther, essore, lave à l'éther, sèche sous pression à 50 °C. On obtient 1,92 g de produit brut. On dissout 1,7 g de ce produit dans 9 $cm^3$ d'éthanol tiède, filtre la solution, ajoute 80 $cm^3$ d'éther, amorce la cristallisation, abandonne à température ambiante puis 1 heure à + 4 °C, essore et sèche sous pression réduite à 50 °C le produit cristallisé. On recueille 1,5 g de produit attendu. F = 248 °C.

$/\alpha/_D + 34{,}5° \pm 2°$ (c = 0,5 % éthanol).

Exemple 35 : 10β-(1-propynyl) 17β-hydroxy 17α-méthyl estra 4,9 (11) dièn-3-one

Stade A : 3,3-éthylènedioxy 10β-(1-propynyl) 5α,17β-dihydroxy 17α-méthyl estr 9 (11) ène.

Dans 5,6 $cm^3$ d'une solution éthérée d'iodure de méthyl magnésium (0,9M), on ajoute goutte à goutte sous atmosphère inerte 370 mg de 3,3-éthylènedioxy 5α-hydroxy 10β-(1-propynyl) estr-9-èn-17-one préparé comme au stade A de l'exemple 4 en solution dans 4 $cm^3$ de tétrahydrofuranne. On ajoute 10 $cm^3$ de tétrahydrofuranne, agite 1 heure 30 minutes à température ambiante, verse sur du chlorure d'ammonium glacé, extrait à l'acétate d'éthyle, lave la phase organique à l'eau puis avec une solution aqueuse saturée de chlorure de sodium, sèche et concentre à sec sous pression réduite. On chromatographie le résidu sur silice (éluant : cyclohexane-acétate d'éthyle 6-4 à 1 ‰ de triéthylamine) et obtient 281 mg de produit attendu. F = 183 °C.

Stade B : 10β-(1-propynyl) 17β-hydroxy 17α-méthyl estra 4,9 (11) dièn-3-one.

On chauffe à 50 °C pendant 1 heure 30 minutes 596 mg de produit préparé comme au stade A précédent dans 12 $cm^3$ d'éthanol et 3,2 $cm^3$ d'acide chlorhydrique 5N. On ramène à température ambiante, verse dans l'eau glacée, alcalinise avec de l'ammoniaque, extrait à l'acétate d'éthyle, lave la phase organique à l'eau puis avec une solution aqueuse saturée de chlorure de sodium, sèche et concentre à sec sous pression réduite. On chromatographie le résidu sur silice (éluant : n-hexane-acétate d'éthyle 6-4) et obtient 438 mg de produit attendu. F = 130 °C après recristallisation dans l'éther.

$/\alpha/_D = + 95° + 3°$ (c = 0,3 % chloroforme).

Analyse :

| | | |
|---|---|---|
| Calculé : | C 81,44 | H 8,70 % |
| Trouvé : | C 81,4 | H 8,9 % |

Exemple 36 : (17R,2'S) 2'-oxydo 10β-(1-propynyl) spiro/estra 4,9 (11)-dièn-17,5'-1,2-oxathiolane/3-one (isomère A) et (17R,2'R) 2'-oxydo 10β-(1-propynyl) spiro/estra 4,9 (11)-dièn-17,5'-1,2-oxathiolane/3-one (isomère B)

Stade A : (17S)-3,3-éthylènedioxy 10β-propynyl spiro/estr 9 (11)-èn-17,2′-oxiran/5α-ol.

On ajoute en 10 minutes à température ambiante et sous atmosphère d'azote 16,8 $cm^3$ d'une solution tétrahydrofurannique de terbutylate de potassium (2,4M) à 10 g de 3,3-éthylènedioxy 10β-(1-propynyl) 5α-hydroxy Estr 9 (11) èn-17-one préparé comme au stade A de l'exemple 4 dans 140 $cm^3$ de tétrahydrofuranne, puis ajoute 6,88 g d'iodure de triméthyl sulfonium dans 138 $cm^3$ de diméthyl sulfoxyde et agite pendant 1 heure. On verse sur 200 $cm^3$ d'une solution aqueuse glacée à demi saturée de chlorure d'ammonium, extrait à l'éther, lave la phase organique à l'eau puis avec une solution aqueuse saturée de chlorure de sodium, sèche et concentre à sec. On chromatographie le résidu sur silice (éluant : n-hexane-acétate d'éthyle 7-3 à 1 ‰ de triéthylamine). On obtient 8,79 g de produit attendu.

Stade B : 3,3-éthylènedioxy 10β-éthynyl 5α,17β-dihydroxy 17α-(terbutyl sulfinyl éthyl) estra 9 (11) ène.

On ajoute en 70 minutes à 5 °C 98,8 $cm^3$ d'une solution (1,6M) de butyllithium dans l'hexane à 19,6 $cm^3$ de méthylbutylsulfoxyde dans 164 $cm^3$ de tétrahydrofuranne et agite 15 minutes. On ajoute en 10 minutes une solution de 3,13 g de produit obtenu au stade A précédent dans 16,2 $cm^3$ de tétrahydrofuranne, agite 10 minutes en maintenant à + 5 °C et laisse revenir à température ambiante. On laisse sous agitation pendant 89 heures, refroidit à 5 °C, ajoute avec précaution 50 $cm^3$ d'une solution aqueuse saturée de chlorure d'ammonium, extrait à l'acétate d'éthyle, lave à l'eau, puis avec une solution aqueuse saturée de chlorure de sodium, sèche et concentre à sec sous pression réduite. On chromatographie le résidu sur silice (éluant : chlorure de méthylène-acétone 6-4 à 1 ‰ de triéthylamine) et isole 1,19 g d'isomère A et 2,3 g d'isomère B (impur).

Stade C :

1) 10β-éthynyl 17β-hydroxy 17α-(terbutyl sulfinyl éthyl) estr 4,9 (11) èn-3-one (isomère A).

On chauffe 1 heure 30 minutes à 50 °C 1,2 g de l'isomère A obtenu comme au stade B précédent dans 20 $cm^3$ d'éthanol et 20 $cm^3$ d'acide chlorhydrique 5N. On refroidit, ajoute de l'ammoniaque, concentre partiellement, extrait au chlorure de méthylène, lave la phase organique à l'eau puis avec une solution aqueuse saturée de chlorure de sodium, sèche et concentre à sec. On reprend le résidu dans 20 $cm^3$ de méthanol, chauffe au reflux, refroidit, essore et sèche 0,92 g de produit attendu. F = 178 °C.

2) 10β-éthynyl 17β-hydroxy 17α-(terbutyl sulfinyl éthyl) estr 4,9 (11) èn-3-one (isomère B).

On chauffe 2 heures à 50 °C 2,3 g de l'isomère B obtenu au stade B précédent dans 68 $cm^3$ d'éthanol et 17 $cm^3$ d'acide chlorhydrique 5N. On refroidit, verse sur de l'eau glacée, extrait à l'acétate d'éthyle, lave la phase organique à l'eau puis avec une solution aqueuse saturée de chlorure de sodium, sèche et concentre à sec. On chromatographie le résidu sur silice (éluant : chlorure de méthylène-acétone 6-4) et obtient 1,266 g de produit attendu que l'on concrète dans l'éther. F = 165°-166°C.

Stade D :

1) (17R,2′S) 2′-oxydo 10β-(1-propynyl) spiro/estra 4,9 (11)-dièn-17,5′-1,2-oxathiolane/3-one (isomère A).

On ajoute 0,415 g de N-chlorosuccinimide dans une solution contenant 1,223 g de l'isomère B obtenu au stade C 2) précédent, 15 $cm^3$ de tétrahydrofuranne et 7 $cm^3$ d'eau. On agite 35 minutes, dilue à l'eau et extrait au chlorure de méthylène. On lave la phase organique à l'eau puis avec une solution aqueuse saturée de chlorure de sodium, sèche et concentre à sec sous pression réduite. On chromatographie le résidu sur silice (éluant : acétate d'éthyle-cyclohexane 7-3) et obtient 1,06 g de produit brut. On reprend le produit dans 6 $cm^3$ d'éthanol, concentre jusqu'à l'amorce de cristallisation, glace, essore et sèche 0,586 g de produit attendu. F = 210 °C.

$/\alpha/_D = -5,5° + 2°$ (c = 0,5 % chloroforme).

Analyse :

| | | | |
|---|---|---|---|
| Calculé : | C 71,84 | H 7,34 | S 8,34 % |
| Trouvé : | C 72,1 | H 7,4 | S 8,3 % |

2) (17R,2′R) 2′-oxydo 10β-(1-propynyl) spiro/estra 4,9 (11)-dièn-17,5′-1,2-oxathiolane/3-one (isomère B).

On opère comme au stade D 1) précédent à partir de 1,068 g de l'isomère A obtenu au stade C 1) dans 15 $cm^3$ de tétrahydrofuranne, 7 $cm^3$ d'eau et de 0,415 g de N-chlorosuccinimide. On obtient 0,480 g de produit attendu. F = 198 °C.

$/\alpha/_D = + 3,5° + 2°$ (c = 0,5 % chloroforme).

Analyse :

| | | | |
|---|---|---|---|
| Calculé : | C 71,84 | H 7,34 | S 8,34 % |
| Trouvé : | C 71,5 | H 7,5 | S 8,2 % |

Exemple 37 : (17S) 3'-propyl 10β-(1-propynyl) spiro/estra 4,9 (11) dièn-17,5'-oxazolidine/2',3-dione

Stade A : 3,3-éthylènedioxy 10β-(1-propynyl) 5α,17β-dihydroxy 17α-(propylaminométhyl) estr 9 (11) ène.

On chauffe 4 heures à 140 °C 3 g de (17S) 3,3-éthylènedioxy 10β-(1-propynyl) spiro/estr 9 (11) èn-17,2'-oxiran/5α-ol préparé comme au stade A de l'exemple 36 et 68 mg d'acide paratoluène sulfonique dans 4,1 $cm^3$ de toluène et 5,7 $cm^3$ de propylamine. On refroidit dans un bain de glace, extrait au chlorure de méthylène, lave la phase organique avec une solution aqueuse de bicarbonate de sodium, puis à l'eau, sèche et concentre à sec sous pression réduite. On chromatographie le résidu sur silice (éluant : chlorure de méthylène-méthanol-ammoniaque 95-5-0,5) et obtient 3,33 g de produit attendu.

Stade B : 3,3-éthylènedioxy 10β-(1-propynyl) 5α,17β-dihydroxy 17α-/N-propyl N-(éthoxycarbonyl) amino/méthyl estr 9 (11) ène.

On ajoute en 40 minutes sous atmosphère d'azote 11,5 $cm^3$ d'une solution de chloroformiate d'éthyle dans le chloroforme à 3,3 g de produit obtenu au stade A précédent dans 35 $cm^3$ d'une solution de triéthylamine dans le chloroforme (10-1). On agite 45 minutes, dilue avec une solution aqueuse de bicarbonate de sodium, décante, lave la phase chloroformique à l'eau puis avec une solution aqueuse de chlorure de sodium, sèche et concentre à sec sous pression réduite. On chromatographie le résidu sur silice (éluant : hexane-acétate d'éthyle 6-4 à 1‰ de triéthylamine) et obtient 3,62 g de produit attendu.

Stade C : 3,3-éthylènedioxy 5α-hydroxy (17S) 3'-propyl 10β-(1-propynyl) spiro/estr 9 (11) èn 17,5'-oxazolidine 2'-one.

On chauffe au reflux 1 heure sous atmosphère inerte, 3,6 g de produit obtenu au stade B précédent dans 35 $cm^3$ de potasse méthanolique (0,5M). On ajoute de l'eau glacée, ajuste le pH à 6 à l'aide d'acide chlorhydrique N et extrait à l'acétate d'éthyle. On lave la phase organique à l'eau puis à l'aide d'une solution aqueuse saturée de chlorure de sodium, sèche et concentre à sec sous pression réduite. On obtient 3,25 g de produit attendu.

Stade D : (17S) 3'-propyl 10β-(1-propynyl) spiro/estr 4,9 (11) dièn-17,5'-oxazolidine/2',3-dione.

On opère comme au stade B de l'exemple 35 à partir de 3,2 g de produit obtenu au stade C précédent, 71 $cm^3$ d'éthanol et 18 $cm^3$ d'acide chlorhydrique 5N. On obtient 2,56 g de produit brut que l'on triture dans n-pentane et sèche. On recueille 2,32 g de produit attendu. F = 150 °C après recristallisation dans l'éther isopropylique.

$/\alpha/_D = -8{,}5° \pm 0{,}5°$ (c = 1 % chloroforme).

Analyse :

| | | | |
|---|---|---|---|
| Calculé : | C 76,63 | H 8,16 | N 3,43 % |
| Trouvé : | C 76,4 | H 8,3 | N 3,4 % |

Exemple 38 : γ-lactone de l'acide 3-oxo 10β-éthynyl 17β-hydroxy 19-nor pregna 1,4,9 (11) trièn-21-carboxylique

On chauffe 24 heures au reflux 1 g de produit préparé comme au stade C de l'exemple 1, 50 $cm^3$ de benzène, 1,4 g de dichlorodicyanoquinone et 0,6 g d'acide benzoïque. On refroidit la solution, ajoute 10 $cm^3$ de soude N, extrait à l'acétate d'éthyle. On lave la phase organique à l'eau puis avec une solution aqueuse saturée de chlorure de sodium, sèche et concentre à sec sous pression réduite. On chromatographie le résidu sur silice (éluant : chlorure de méthylène-acétone 95-5) et obtient 508 mg de produit attendu que l'on cristallise dans l'acétate d'éthyle F = 230 °C.

Spectre IR (chloroforme)

3 305 $cm^{-1}$ : —C≡CH ;
1 766 $cm^{-1}$ : —C=O (γ-lactone
1 669 $cm^{-1}$ (cétone conjuguée ;
1 634-1 610 $cm^{-1}$ : —C=C— ;
891 $cm^{-1}$ : —C=C— Δ1,4.

Exemple 39 : γ-lactone de l'acide 10β-(2-bromoéthynyl) 17β-hydroxy 3-oxo 19-nor 17α-pregna 4,9 (11) dièn-21-carboxylique

On ajoute 178 mg de N-bromosuccinimide et 16 mg de nitrate d'argent à une solution de 280 mg de

produit obtenu à l'exemple 1 dans 6 $cm^3$ d'acétone. On agite 1 heure à température ambiante, dilue le milieu réactionnel à l'eau, ajoute une solution aqueuse à 10 % de thiosulfate de sodium, extrait au chlorure de méthylène, sèche et concentre à sec. On concrète le résidu dans l'éther isopropylique et obtient 250 mg de produit attendu.

$/\alpha/_D = + 23,5°$ (c = 1 % chloroforme).

Analyse : $C_{23}H_{25}BrO_3$ : 429,36
Calculé : C 64,34 H 5,86 Br 18,61 %
Trouvé : C 64,3 H 5,9 Br 18,4 %

Exemple 40 : Exemple de composition pharmaceutique

On a préparé des comprimés à 50 mg de produit de l'exemple 4 comme principe actif.

| | |
|---|---|
| Produit de l'exemple 4 : | 50 mg |
| Excipient (talc, amidon, stéarate de magnésium). | |

Etude pharmacologique des produits de l'invention

Etude de l'activité antialdostérone sur le récepteur minéralocorticoïde du rein de rat

Des rats mâles Sprague-Dawley EOPS, pesant 140 à 160 g, surrénalectomisés depuis 4 à 6 jours sont sacrifiés et leurs reins sont perfusés in situ avec 50 ml d'un tampon Tris 10m M-Saccharose 0,25 M, HCl pH 7,4. Les reins sont ensuite prélevés, décapsulés et homogénéisés à 0 °C à l'aide d'un Potter polytétrafluoroéthylène-verre (1 g de tissu pour 3 ml de tampon). L'homogénat est centrifugé pendant 10 minutes à 800 g, à 0 °C.

Afin d'éliminer la fixation de l'aldostérone tritiée sur le récepteur glucocorticoïde, la 11β, 17β-dihydroxy 21-méthyl pregna 1,4,6-trien 20-yn 3-one, stéroïde se fixant uniquement sur le récepteur glucocorticoïde est additionnée au surnageant à la concentration finale de $10^{-6}$ M. Ce surnageant est ultracentrifugé à 105 000 g pendant 60 minutes à 0 °C. Des aliquotes du surnageant ainsi obtenues sont incubées à 0 °C avec une concentration constante (T) d'aldostérone tritiée en présence de concentrations croissantes (0-2 500. $10^{-9}$ M) d'aldostérone froide ou du produit froid à étudier. Après un temps (t) d'incubation, la concentration d'aldostérone tritiée liée (B) est mesurée par la technique d'adsorption au charbon-dextran.

Calcul de l'affinité relative de liaison

Le calcul de l'affinité relative de liaison (ARL) est effectué comme suit :

On trace les 2 courbes suivantes : le pourcentage de l'hormone tritiée liée B/T en fonction du logarithme de la concentration de l'hormone de référence froide et B/T en fonction du logarithme de la concentration du produit froid testé.

On détermine la droite d'équation $I^{50} = (B/T\ max + B/T\ min)/2$

B/T max = Pourcentage de l'hormone tritiée liée pour une incubation de cette hormone tritiée à la concentration (T).

B/T min = Pourcentage de l'hormone tritiée liée pour une incubation de cette hormone tritiée à la concentration (T) en présence d'un grand excès d'hormone froide ($2\,500 \cdot 10_{-9}$ M).

Les intersections de la droite $I_{50}$ et des courbes permettent d'évaluer les concentrations de l'hormone de référence froide (CH) et du produit froid testé (CX) qui inhibent de 50 % la liaison de l'hormone tritiée sur le récepteur.

L'affinité relative de liaison (ARL) du produit testé est déterminée par l'équation

$$ARL = 100 \frac{(CH)}{(CX)}$$

(Voir Tableau p. 30)

Les résultats obtenus sont les suivants :

| Produit de l'exemple \ Temps d'incubation à 0°C | 4 H | 24 H |
|---|---|---|
| 1 | 510 | 165 |
| 4 | 70 | 63 |
| 6 | 240 | 19 |
| 8 | 273 | 64 |
| 9 | 46 | 96 |
| 15 | 151 | 5 |
| Produit P | 164 | 13 |

Le produit P est le 17-hydroxy 10-(éthynyl) estr 4,9 (11) diène 3-one décrit comme produit chimique dans la publication Bull. Soc. Chim. Fr. 1970 n° 7 p. 2556.

## Revendications

1. Les produits de formule générale I :

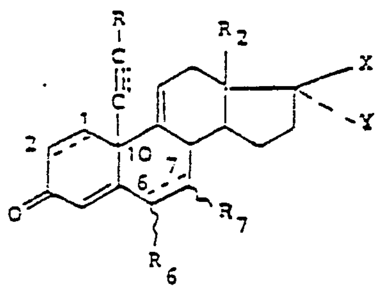

(I)

dans laquelle R représente :

— soit un atome d'hydrogène,

— soit un radical alkyle, alkényle ou alkynyle ayant au plus 8 atomes de carbone, éventuellement substitué par les radicaux hydroxyle, carboxy éventuellement estérifié, amino, tritylamino, chloroacétylamino, trifluoroacétylamino ; trichloroéthoxycarbonylamino, monoalkyl ou dialkylamino ou halogènes,

— soit un radical aryle ou aralkyle éventuellement substitué par les radicaux hydroxyle, carboxy éventuellement estérifié, amino, mono ou dialkylamino, alkyle, alkoxy ou alkylthio,

— soit un radical hydroxyle, tétrahydropyrannyloxy, tert-butyloxy, carboxy éventuellement estérifié, amino, mono ou dialkylamino, tritylamino, chloroacétylamino, trifluoroacétylamino ou trichloroéthoxycarbonylamino,

— soit un atome d'halogène,

— soit un radical trialkylsilyle,

$R_6$ et $R_7$ sont tels que soit $R_6$ et $R_7$ forment avec les carbones qui les portent un radical cyclopropyle, soit $R_6$ représente un atome d'hydrogène et $R_7$ représente le radical $R_1$, $R_1$ représentant :

— soit un atome d'hydrogène,

— soit un radical alkyle, alkényle ou alkynyle ayant au plus 6 atomes de carbone éventuellement substitué par les radicaux hydroxyle, carboxy éventuellement estérifié, amino, tritylamino, chloroacétylamino, trifluoroacétylamino, trichloroéthoxycarbonylamino, monoalkyl ou dialkylamino ou halogènes.

$R_2$ représente un radical méthyle ou éthyle,

X et Y sont tels que :

— soit X représente un radical hydroxyle, acétyloxy, propionyloxy, méthoxy ou éthoxy et Y représente ou bien un radical $—CH_2CH_2CO_2M$ dans lequel

M représente un atome d'hydrogène un atome de métal alcalin ou un radical ammonium,

ou bien un radical $—CH_2CH_2—CH_2OH$,

— soit X et Y représentent ensemble un radical :

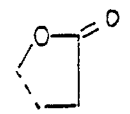

ou

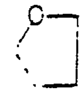

— soit X et Y représentent ensemble un radical

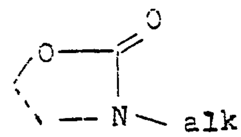

dans lequel alk représente un radical alkyle renfermant au plus 8 atomes de carbone,

— soit X et Y représentent ensemble un radical

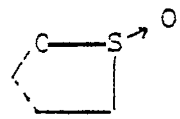

ou X représente un radical hydroxyle et Y un radical

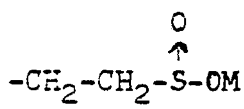

M étant défini comme ci-dessus,

— soit X représente un radical hydroxyle, acétyloxy, propionyloxy, méthoxy ou éthoxy et Y représente, ou bien un atome d'hydrogène ou bien Y représente $R_4$, $R_4$ représente un radical alkyle, alkényle ou alkynyle ayant au plus 6 atomes de carbone,

les traits pointillés en position 1 (2) et 6 (7) indiquent la présence éventuelle d'une seconde liaison entre les carbones qui les portent, étant entendu qu'il ne peut pas y avoir de seconde liaison en position 6 (7) lorsque $R_6$ et $R_7$ forment avec les carbones qui les portent un radical cyclopropyle, le trait pointillé sur le substituant R—C≡C— en position 10 indique la présence éventuelle d'une troisième liaison entre les carbones qui les portent,

les traits ondulés en position 6 et 7 indiquent que les substituants $R_6$ et $R_7$ peuvent se trouver dans l'une des positions possibles α ou β, étant entendu que R ne peut pas représenter un atome d'hydrogène lorsque $R_6$ et $R_7$ représentent chacun un atome d'hydrogène,

$R_2$ représente un radical méthyle, X représente un radical hydroxy ou acétoxy et Y un atome d'hydrogène, les traits pointillés en position 1 (2) et 6 (7) ne représentent pas une seconde liaison entre les carbones qui les portent et le trait pointillé sur le substituant en position 10 indique la présence d'une troisième liaison entre les carbones qui le portent.

2. Les produits de formule générale (I), telle que définie à la revendication 1, répondant à la formule ($I_1$) :

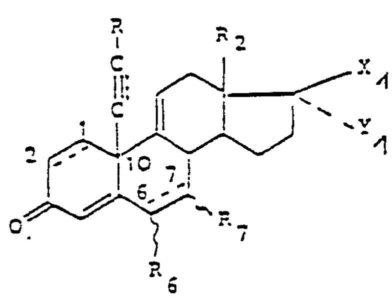

($I_1$)

dans laquelle R, $R_2$, $R_6$ et $R_7$ ont les significations indiquées à la revendication 1,

$X_1$ et $Y_1$ sont tels que :

— soit $X_1$ représente un radical hydroxyle et $Y_1$ représente ou bien un radical $-CH_2CH_2CO_2M$ dans lequel M représente un atome d'hydrogène, un atome de métal alcalin ou un radical ammonium, ou bien un radical $-CH_2-CH_2-CH_2-OH$,

— soit $X_1$ et $Y_1$ représentent ensemble un radical :

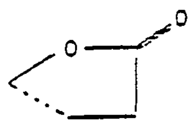

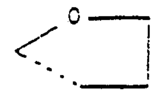

— soit $X_1$ réprésente un radical hydroxyle éventuellement acylé ou éthérifié et $Y_1$ représente ou bien un atome d'hydrogène ou bien $Y_1$ représente $R_4$, $R_4$ représentant un radical alkyle, alkényle ou alkynyle ayant au plus 6 atomes de carbone,

les traits pointillés en position 1 (2) et 6 (7) indiquent la présence éventuelle d'une seconde liaison entre les carbones qui les portent étant entendu qu'il ne peut pas y avoir de seconde liaison en position 6 (7) lorsque $R_6$ et $R_7$ forment avec les carbones qui les portent un radical cyclopropyle, le trait pointillé sur le substituant R—C=C— en position 10 indique la présence éventuelle d'une troisième liaison entre les carbones qui les portent,

les traits ondulés en position 6 et 7 indiquent que les substituants $R_6$ et $R_7$ peuvent se trouver dans l'une des positions possibles α ou β, étant entendu que R ne peut pas représenter un atome d'hydrogène lorsque $R_6$ et $R_7$ représentent chacun un atome d'hydrogène, $R_2$ représente un radical méthyle, $X_1$ représente un radical hydroxy ou acétoxy et $Y_1$ un atome d'hydrogène, les traits pointillés en position 1 (2) et 6 (7) ne représentent pas une seconde liaison entre les carbones qui les portent et le trait pointillé sur le substituant en position 10 indique la présence d'une troisième liaison entre les carbones qui le portent.

3. Les produits de formule générale $I_1$, telle que définie à la revendication 2, répondant à la formule

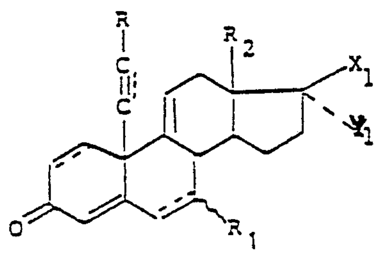

dans laquelle R, $R_1$, $R_2$, $X_1$, $Y_1$ les traits pointillés et les traits ondulés ont la signification indiquée à la revendication 2.

4. Les produits de formule I telle que définie à l'une quelconque des revendications 1 à 3, dans laquelle le trait pointillé sur le substituant en position 10 représente une troisième liaison et $R_2$ est choisi dans le groupe formé par un atome d'hydrogène, un radical alkyle ayant de 1 à 3 atomes de carbone, un radical hydroxyméthyle et un radical phényle.

5. Les produits de formule I telle que définie à l'une quelconque des revendications 1 à 4, dans laquelle le substituant $R_1$ est choisi dans le groupe formé par un atome d'hydrogène, un radical alkyle ayant de 1 à 4 atomes de carbone et un radical acétylthio et X et Y sont tels que soit X représent un radical hydroxy et Y représente ou bien un radical $CH_2CH_2CO_2M'$ dans lequel M' représente un atome d'hydrogène ou atome de métal alcalin, ou bien Y représente un atome d'hydrogène,

— soit X et Y représentent un radical

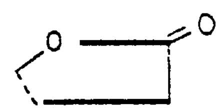

6. Les produits de formule générale I dont les noms suivent :

— la γ-lactone de l'acide 10β-éthynyl 17β-hydroxy 3-oxo 19-nor 17α-pregna 4,9 (11)-diène 21-carboxylique

et la γ-lactone de l'acide 10β-(1-propynyl) 17β-hydroxy 3-oxo 19-nor 17α-pregna 4,9 (11)-diène 21-carboxylique.

7. Procédé de préparation des produits de formule I telle que définie à la revendication 1, caractérisé en ce que l'on traite un produit de formule II :

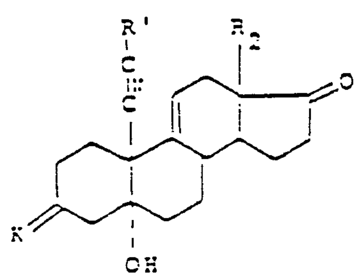

(II)

dans laquelle R' représente soit R, R ayant la signification indiquée à la revendication 1 soit le substituant R dans lequel les fonctions réactivées sont protégées, $R_2$ a la signification indiquée à la revendication 1 et K représente un groupement protecteur de la fonction cétone.

I) soit d'abord par un halogénure de triméthylsulfonium en présence d'une base forte puis par un dérivé métallique de l'acétonitrile et enfin par une base puis par un acide, soit d'abord par un réactif de formule :

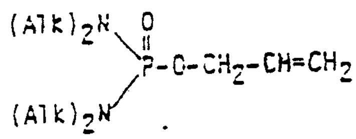

dans lequel Alk représente un radical alkyle ayant de 1 à 4 atomes de carbone en présence d'une base forte puis par un acide pour obtenir un produit de formule $I_A$ :

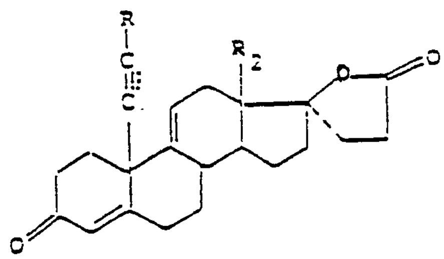

($I_A$)

II) soit par un réactif de formule :

$$XMg—CH_2—CH_2—CH_2—OB$$

dans lequel X représente un atome d'halogène et B représente un groupement protecteur du radical hydroxyle ou un alcoolate de magnésium pour obtenir après traitement par un acide un produit de formule $I'_A$ :

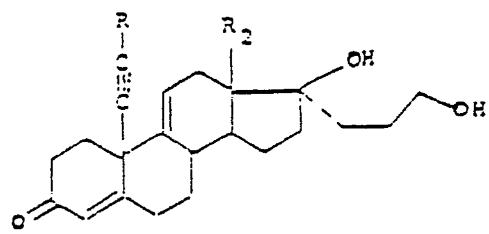

($I'_A$)

produit de formule $I'_A$ que l'on traite éventuellement par un halogénure d'acide sulfonique en présence d'une base pour obtenir un produit de formule $I''_A$ :

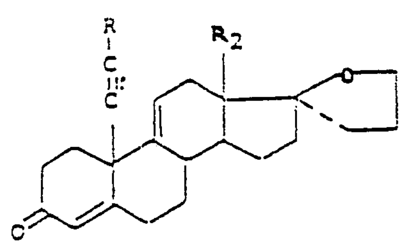

($I''_A$)

III) soit par un réactif de réduction puis par un acide pour obtenir un produit de formule $I_{3A}$ :

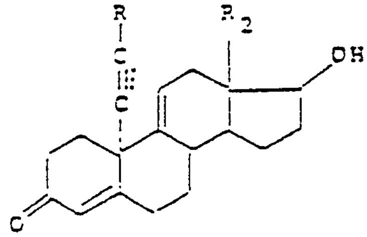

($I_{3A}$)

IV) soit par un organométallique $R_4MgX$ ou $(R_4)_2$—CuLi, X représentant un atome d'halogène, puis par un acide, pour obtenir un produit de formule $I_{4A}$ :

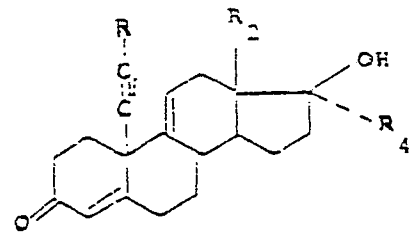

($I_{4A}$)

V) soit d'abord par un halogénure de triméthylsulfonium en présence d'une base forte, puis par une amine primaire de formule $H_2N$—alk, alk étant défini comme précédemment, en présence d'un acide, par un chloroformiate d'alkyle, par une base forte et enfin par un acide, pour obtenir un produit de formule $I_{5A}$ :

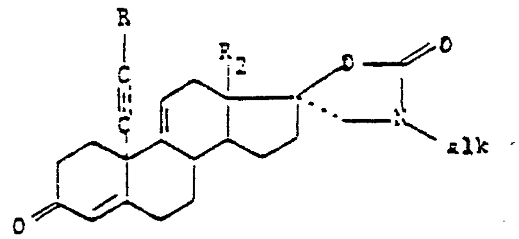

($I_{5A}$)

VI) soit d'abord par un halogénure de triméthylsulfonium en présence d'une base forte, puis par le méthyl terbutyl sulfoxyde en présence de n-butyl-lithium pour obtenir un produit de formule

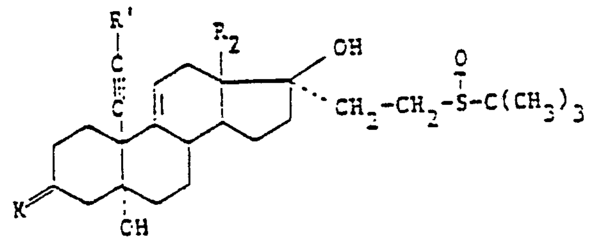

sous forme d'un mélange de deux diastéréoisomères au niveau de l'atome de soufre, sépare si désiré les deux diastéréoisomères, puis soumet soit leur mélange, soit chacun d'eux séparément, à l'action d'un acide, pour obtenir un produit de formule

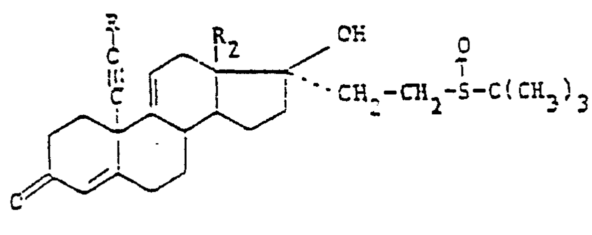

sous forme d'un mélange de deux diastéréoisomères ou un diastéréoisomère, sépare le cas échéant les diastéréoisomères, puis soumet soit leur mélange, soit chacun d'eux séparément, à l'action du N-chloro ou du N-bromo succinimide, pour obtenir un produit de formule $I_{6A}$ :

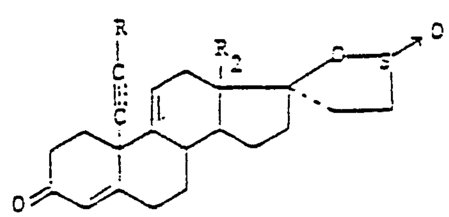

($I_{6A}$)

et que, si désiré, l'on traite les produits de formules $I_A$, $I'_A$, $I''_A$, $I_{3A}$, $I_{4A}$, $I_{5A}$ et $I_{6A}$ par un orthoformiate

d'alkyle en présence d'un acide puis par un agent de déshydrogénation pour obtenir les produits de formule $I_B$, $I'_B$, $I''_B$, $I_{3B}$, $I_{4B}$, $I_{5B}$ et $I_{6B}$ de formules suivantes :

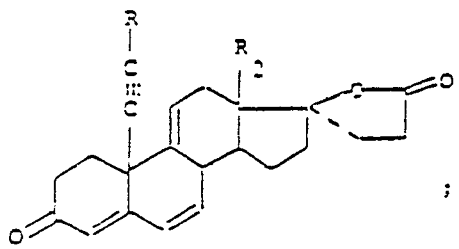

$I_B$

;

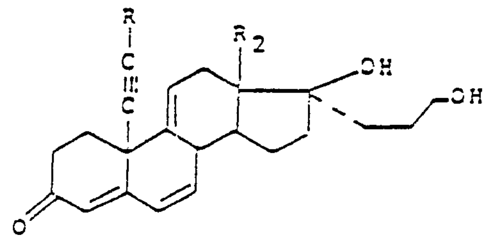

$I'_B$

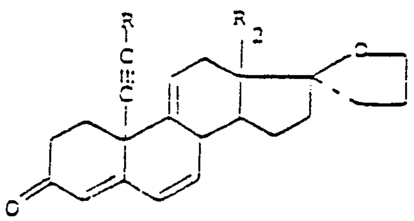

$I''_B$

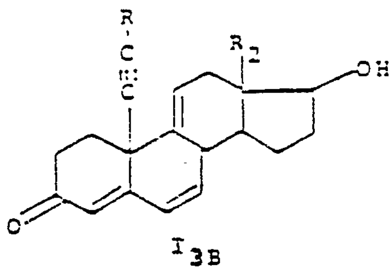

$I_{3B}$

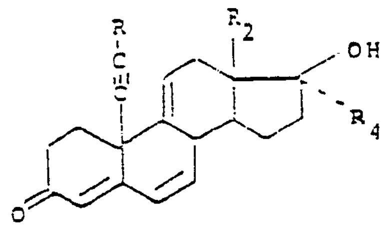

$I_{4B}$

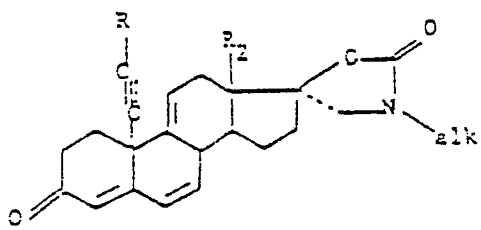

$I_{5B}$

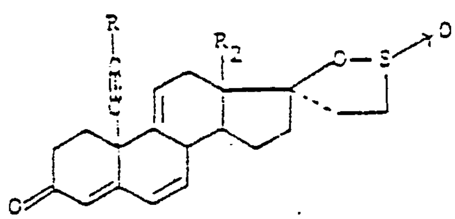

$I_{6B}$

produits de formules $I_B$, $I'_B$, $I''_B$, $I_{3B}$, $I_{4B}$, $I_{5B}$ et $I_{6B}$ que l'on traite si désiré :

— ou bien par un organo magnésien de formule $R_1MgX'$ éventuellement en présence d'un sel de cuivre, formule dans laquelle $R_1$ a la signification indiquée ci-dessus et X' représente un atome d'halogène

— ou bien par un organo métallique de formule $(R_1)_2CuLi$ puis par un acide, pour obtenir les produits de formules $I_C$, $I'_C$, $I''_C$, $I_{3C}$, $I_{4C}$, $I_{5C}$ et $I_{6C}$ :

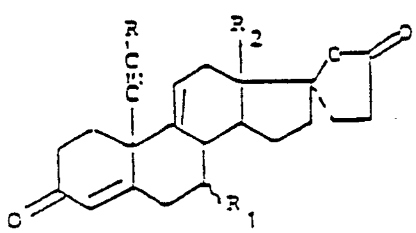

$I_C$

;

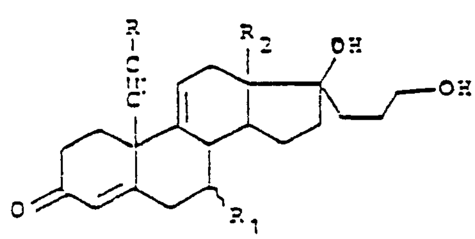

$I'_C$

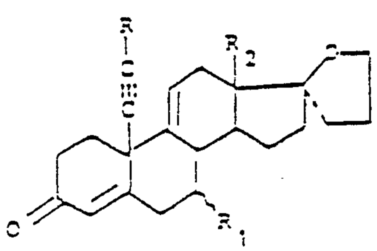

$I''_C$

;

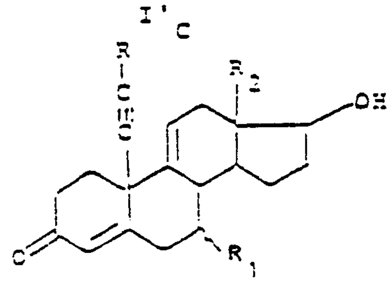

$I_{3C}$

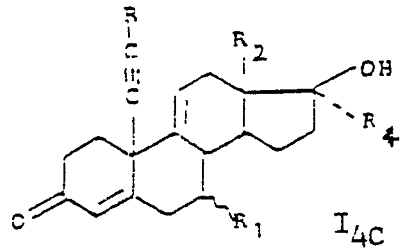

I4C

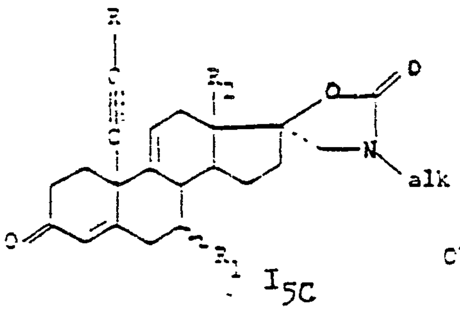

I5C

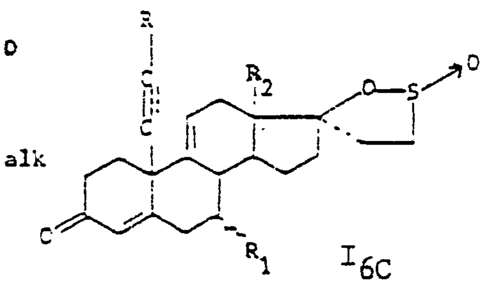

I6C

sous forme d'un mélange d'isomères 7α et 7β, mélange que, si désiré, l'on sépare et que, si désiré, l'on soumet les produits 7β à un réactif de déshydrogénation pour obtenir les produits Δ6 (7) correspondants ;

— ou bien l'on soumet les produits de formules $I_B$, $I'_B$, $I''_B$, $I_{3B}$, $I_{4B}$, $I_{5B}$ et $I_{6B}$ à l'action d'un réactif choisi dans le groupe constitué par l'iodure de triméthylsulfonium et l'iodure de triméthylsulfoxonium en présence d'une base forte pour obtenir les produits de formules $I_D$, $I'_D$, $I''_D$, $I_{3D}$, $I_{4D}$, $I_{5D}$ et $I_{6D}$ :

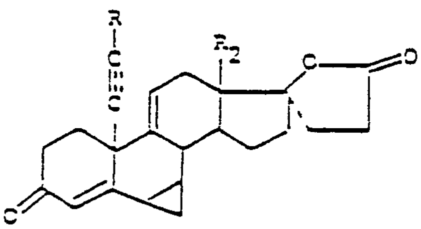

$I_D$

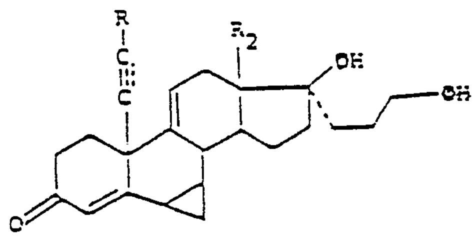

$I'_D$

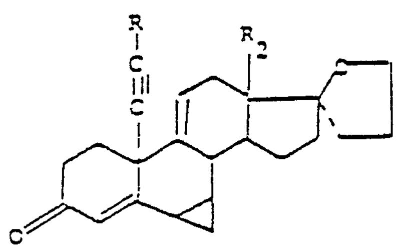

$I''_D$

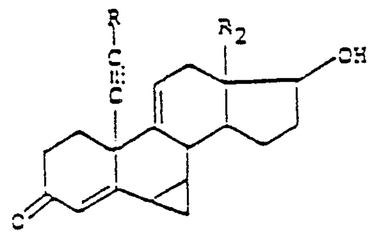

$I_{3D}$

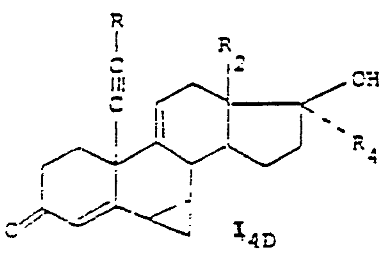

$I_{4D}$

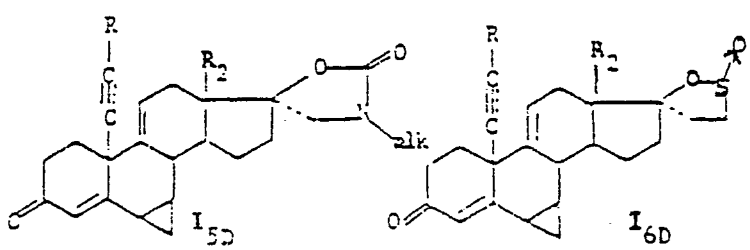

$I_{5D}$ $I_{6D}$

sous forme d'un mélange 6α, 7α et 6β, 7β et sépare, si désiré, les isomères ainsi obtenus et que si désiré l'on traite les produits $I_A$, $I'_A$, $I''_A$, $I_{3A}$, $I_{4A}$, $I_{5A}$, $I_{6A}$, $I_B$, $I'_B$, $I''_B$, $I_{3B}$, $I_{4B}$, $I_{5B}$, $I_{6B}$, $I_C$, $I'_C$, $I''_C$, $I_{3C}$, $I_{4C}$, $I_{5C}$, $I_{6C}$, $I_D$, $I'_D$, $I''_D$, $I_{3D}$, $I_{4D}$, $I_{5D}$, $I_{6D}$, par un réactif de déshydrogénation ou par un microorganisme susceptible de déshydrogéner la molécule en position 1 (2) pour obtenir les produits correspondants comportant une insaturation en position 1 (2) et que, si désiré, l'on traite les produits de formules $I_A$, $I_B$, $I_C$

et $I_D$ et les produits correspondants comportant une insaturation en position 1 (2) à l'aide d'un hydroxyde alcalin ou de l'ammoniaque pour obtenir les produits correspondants dans lesquels X représente un radical hydroxyle et Y représente un radical $—CH_2CH_2CO_2M'$ dans lequel M' représente un atome de métal alcalin ou un radical ammonium et, si désiré, soumet les produits ainsi obtenus à l'action d'un agent acide pour obtenir les produits dans lesquels X représente un radical hydroxyle et Y représente un radical $—CH_2CH_2CO_2H$, et que si désiré l'on traite les produits dans lesquels le substituant en position 10 comporte un substituant R du type hydroxyalkyle, par un tétrabromure ou tétrachlorure de carbone en présence de triphénylphosphine, pour obtenir les dérivés bromés et chlorés correspondants, et que si désiré, l'on traite les produits dans lesquels le substituant en position 10 comporte une triple liaison par un agent d'hydrogénation sélective pour obtenir les produits correspondants dans lesquels le substituant en position 10 comporte une double liaison et que si désiré, l'on traite les produits comportant en position 10 un substituant éthynyl par un halo-succinimide pour obtenir les produits correspondants comportant en position 10 un substituant $—C{\equiv}C—Hal$, et que si désiré l'on acyle ou éthérifie le radical hydroxyle en position 17β des produits comportant en position 17α un atome d'hydrogène, un radical $R_4$ ou un radical $—CH_2—CH_2—CH_2OH$.

8. A titre de médicaments, les produits de formule générale I' :

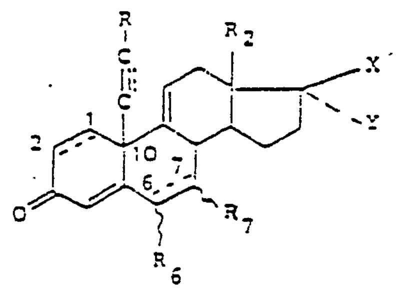

(I')

dans laquelle R représente :

— soit un atome d'hydrogène,

— soit un radical alkyle, alkényle ou alkynyle ayant au plus 8 atomes de carbone, éventuellement substitué par les radicaux hydroxyle, carboxy éventuellement estérifié, amino, tritylamino, chloroacétylamino, trifluoroacétylamino ; trichloroéthoxycarbonylamino, monoalkyl ou dialkylamino ou halogènes,

— soit un radical aryle ou aralkyle éventuellement substitué par les radicaux hydroxyle, carboxy éventuellement estérifié, amino, mono ou dialkylamino, alkyle, alkoxy ou alkylthio,

— soit un radical hydroxyle, tétrahydropyrannyloxy, tert-butyloxy, carboxy éventuellement estérifié, amino, mono ou dialkylamino, tritylamino, chloroacétylamino, trifluoroacétylamino ou trichloroéthoxycarbonylamino,

— soit un atome d'halogène,

— soit un radical trialkylsilyle,

$R_6$ et $R_7$ sont tels que soit $R_6$ et $R_7$ forment avec les carbones qui les portent un radical cyclopropyle, soit $R_6$ représente un atome d'hydrogène et $R_7$ représente le radical $R_1$, $R_1$ représentant :

— soit un atome d'hydrogène,

— soit un radical alkyle, alkényle ou alkynyle ayant au plus 6 atomes de carbone éventuellement substitué par les radicaux hydroxyle, carboxy éventuellement estérifié, amino, tritylamino, chloroacétylamino, trifluoroacétylamino, trichloroéthoxycarbonylamino, monoalkyl ou dialkylamino ou halogènes.

$R_2$ représente un radical méthyle ou éthyle,

X et Y sont tels que :

— soit X représente un radical hydroxyle, acétyloxy, propionyloxy, méthoxy ou éthoxy et Y représente ou bien un radical $—CH_2CH_2CO_2M$ dans lequel

M représente un atome d'hydrogène un atome de métal alcalin ou un radical ammonium,

ou bien un radical $—CH_2CH_2—CH_2OH$,

— soit X et Y représentent ensemble un radical :

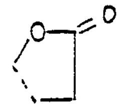 ou 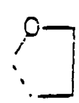

— soit X et Y représentent ensemble un radical

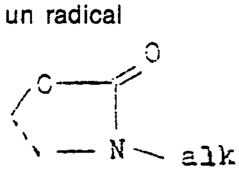

dans lequel alk représente un radical alkyle renfermant au plus 8 atomes de carbone,

— soit X et Y représentent ensemble un radical

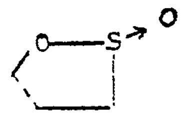

ou X représente un radical hydroxyle et Y un radical

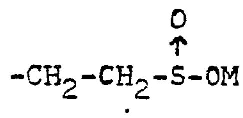

M étant défini comme ci-dessus,

— soit X représente un radical hydroxyle, acétyloxy, propionyloxy, méthoxy ou éthoxy et Y représente, ou bien un atome d'hydrogène ou bien Y représente $R_4$, $R_4$ représente un radical alkyle, alkényle ou alkynyle ayant au plus 6 atomes de carbone,

les traits pointillés en position 1 (2) et 6 (7) indiquent la présence éventuelle d'une seconde liaison entre les carbones qui les portent, étant entendu qu'il ne peut pas y avoir de seconde liaison en position 6 (7) lorsque $R_6$ et $R_7$ forment avec les carbones qui les portent un radical cyclopropyle, le trait pointillé sur le substituant R—C≡C— en position 10 indique la présence éventuelle d'une troisième liaison entre les carbones qui les portent,

les traits ondulés en position 6 et 7 indiquent que les substituants $R_6$ et $R_7$ peuvent se trouver dans l'une des positions possibles α ou β.

9. A titre de médicaments, les produits de formule générale I' dans laquelle R est choisi dans le groupe formé par un atome d'hydrogène, un radical alkyle ayant de 1 à 3 atomes de carbone, un radical hydroxyméthyle et un radical phényle, $R_1$ est choisi dans le groupe formé par un atome d'hydrogène, un radical alkyle ayant de 1 à 4 atomes de carbone et un radical acétylthio et X et Y sont tels que soit X représente un radical hydroxy et Y représente ou bien un radical $CH_2CH_2CO_2M'$ dans lequel M' représente un atome d'hydrogène ou un atome de métal alcalin ou bien Y représente un atome d'hydrogène, soit X et Y représentent un radical

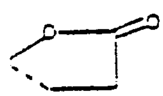

10. A titre de médicaments, les produits de formule générale I' dont les noms suivent :

— la γ-lactone de l'acide 10β-éthynyl 17β-hydroxy 3-oxo 19-nor 17α-pregna 4,9 (11)-diène 21-carboxylique,

— la γ-lactone de l'acide 10β-(1-propynyl) 17β-hydroxy 3-oxo 19-nor 17α-pregna 4,9 (11)-diène 21-carboxylique,

— et la 10β-éthynyl 17β-hydroxy estra 4,9 (11)-diène-3-one.

11. Les compositions pharmaceutiques renfermant, comme principe actif, au moins un médicament défini à l'une quelconque des revendications 8 à 10.

## Claims

1. Products of the general formula I :

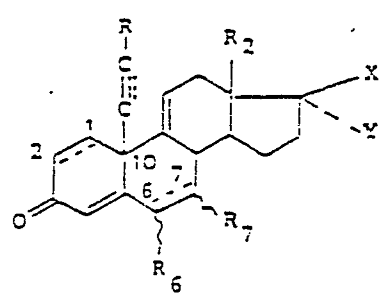

(I)

in which R represents :

— either a hydrogen atom,

— or an alkyl, alkenyl or alkynyl radical having at the most 8 carbon atoms, possibly substituted by the following radicals : hydroxy and carboxy possibly esterified, amino, tritylamino, chloroacetylamino, trifluoroacetyl amino ; trichloroethoxycarbonylamino, monoalkylamino or dialkylamino or by halogens,

— or an aryl or aralkyl radical possibly substituted by the following radicals hydroxyl, carboxy possibly esterified, amino, monoalkylamino or dialkylamino, tritylamino, chloroacetylamino, trifluoroacetylamino or trichloroethoxycarbonylamino,

— or a halogen atom,

— or a trialkylsilyl radical,

$R_6$ and $R_7$ are such that either $R_6$ and $R_7$ form a cyclopropyl radical with the carbons carrying them, or $R_6$ represents a hydrogen atom and $R_7$ represents the radical $R_1$, $R_1$ representing :

— either a hydrogen atom,

— or an alkyl, alkenyl or alkynyl radical having at the most 6 carbon atoms possibly substituted by the following radicals hydroxyl, carboxy possibly esterified, amino, tritylamino, chloroacetylamino, trifluoroacetylamino, trichloroethoxycarbonylamino, monoalkylamino or dialkylamino or by halogens.

$R_2$ represents a methyl or ethyl radical,

X and Y are such that :

— either X represents a hydroxyl, acetyloxy, propionyloxy, methoxy or ethoxy radical and Y represents either a $—CH_2CH_2CO_2M$ radical in which

M represents a hydrogen atom an alkali metal atom or an ammonium atom,

or a $CH_2CH_2CH_2OH$ radical

or X and Y together represent a :

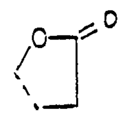

or

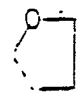

radical,

— or X and Y together represent a

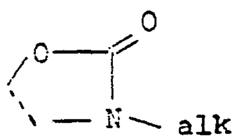

radical in which alk represents an alkyl radical containing at the most 8 carbon atoms,

— or X and Y together represent a

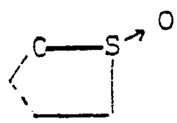

radical, or X represents a hydroxyl radical and Y represents a

$$-CH_2-CH_2-\overset{\overset{O}{\uparrow}}{S}-OM$$

radical, M being defined as above,

— or X represents a hydroxyl, acetyloxy, propionyloxy, methoxy or ethoxy radical and Y represents either a hydrogen atom or Y represents $R_4$, $R_4$ represents an alkyl, alkenyl, or alkynyl radical having at the most 6 carbon atoms,

the dotted lines in position 1 (2) and 6 (7) indicate the possible presence of a second bond between the carbons carrying them, it being understood that there cannot be a second bond in position 6 (7) when $R_6$ and $R_7$ form a cyclopropyl radical with the carbons carrying them, the dotted line on the substituent R—C≡C— in position 10 indicates the possible presence of a third bond between the carbons carrying it,

the wavy lines in position 6 and 7 indicate that the substituents $R_6$ and $R_7$ can be found in one of the possible positions alpha or beta, it being understood that R cannot represent a hydrogen atom when $R_6$ and $R_7$ each represent a hydrogen atom,

$R_2$ represents a methyl radical, X represents a hydroxy or acetoxy radical and Y represents a

hydrogen atom, the dotted lines in position 1 (2) and 6 (7) do not represent a second bond between the carbons carrying them and the dotted line on the substituent in position 10 indicates the presence of a third bond between the carbons carrying it.

2. Products of the general formula (I), as defined in claim 1, corresponding to the formula ($I_1$) :

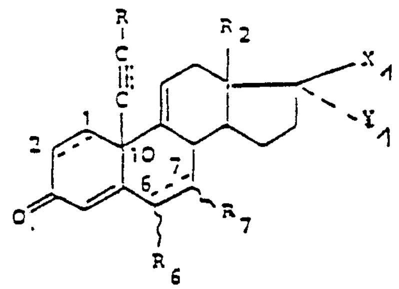

in which R, $R_2$, $R_6$ and $R_7$ have the meanings already given in claim 1,

$X_1$ and $Y_1$ are such that :

— either $X_1$ represents a hydroxyl radical and $Y_1$ represents either a $-CH_2-CH_2-CO_2M$ radical in which M represents a hydrogen atom, an alkali metal atom or an ammonium radical, or a $-CH_2-CH_2-CH_2-OH$ radical,

— or $X_1$ and $Y_1$ together represent a

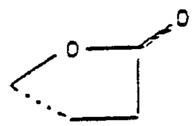

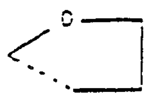

radical,

— or $X_1$ represents a hydroxyl radical possibly acylated or etherified and $Y_1$ represents either a hydrogen atom, or Y represents $R_4$, $R_4$ representing an alkyl, alkenyl or alkynyl radical having at the most 6 carbon atoms,

the dotted lines in position 1 (2) and 6 (7) indicate the possible presence of a second bond between the carbons carrying them, it being understood that there can be no second bond in position 6 (7) when $R_6$ and $R_7$ form a cyclopropyl radical with the carbons carrying them, the dotted line on the substituent R—C=C— in position 10 indicates the possible presence of a third bond between the carbons carrying them,

the wavy lines in position 6 and 7 indicate that the substituents $R_6$ and $R_7$ can be found in one of the possible positions, alpha or beta, it being understood that R cannot represent a hydrogen atom when $R_6$ and $R_7$ each represent a hydrogen atom, $R_2$ represents a methyl radical, $X_1$ represents a hydroxy or acetoxy radical and $Y_1$ a hydrogen atom, the dotted lines in position 1 (2) and 6 (7) do not represent a second bond between the carbons carrying them and the dotted line on the substituent in position 10 indicates the presence of a third bond between the carbons carrying it.

3. Products of the general formula $I_1$, as defined in claim 2, corresponding to the formula

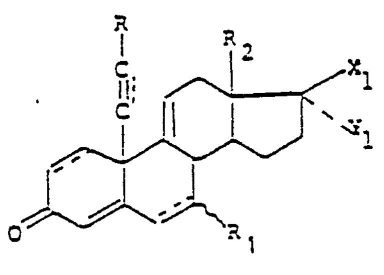

in which R, $R_1$, $R_2$, $X_1$, $Y_1$, the dotted lines and the wavy lines have the meanings already given in claim 2.

4. Products of the formula I as defined in any one of the claims 1 to 3, in which the dotted line on the substituent in position 10 represents a third bond and $R_2$ is chosen from the group formed by a hydrogen atom, an alkyl radical having 1 to 3 carbon atoms, a hydroxymethyl radical and a phenyl radical.

5. Products of the formula I as defined in any one of the claims 1 to 4, in which the substituent $R_1$ is chosen from the group formed by a hydrogen atom, an alkyl radical having from 1 to 4 carbon atoms and an acetylthio radical and X and Y are such that they either X represents a hydroxy radical and Y represents either a $CH_2CH_2CO_2M'$ in which M' represents a hydrogen atom or an alkali metal atom, or Y represents a hydrogen atom,

—or X and Y represent a

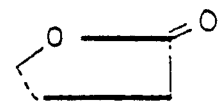

radical

6. Products of the general formula I which have the following names :

— 10beta-ethynyl 17beta-hydroxy 3-oxo 19-nor 17alpha-pregna-4,9 (11)-dien 21-carboxylic acid gammalactone,

— and 10beta-(1-propynyl) 17beta-hydroxy 3-oxo 19-nor 17alpha-pregna-4,9 (11)-dien 21 carboxylic acid gamma-lactone.

7. Preparation process of products of formula I as defined in claim 1, characterised in that a product of formula II :

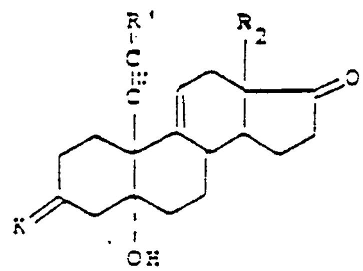

(II)

in which R' represents either R, R having the meaning given in claim 1 or the substituent R in which the reactive functions are protected, $R_2$ has the meaning given in claim 1 and K represents a protector group of the ketone function, is treated

I) either first by a trimethylsulphonium halogenide in the presence of a strong base then by a metallic derivative of acetonitrile and finally by a base then by an acid,

or first by a reagent of the formula :

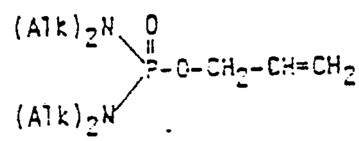

in which Alk represents an alkyl radical having from 1 to 4 carbon atoms in the presence of a strong base then by an acid to obtain a product of the formula $I_A$ :

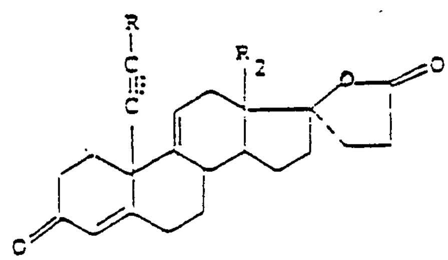

($I_A$)

II) or by a reagent of the formula :

$$XMg—CH_2—CH_2—CH_2—OB$$

in which X represents a halogen atom and B represents a protector group of the hydroxyl radical or magnesium alcoholate to obtain, after treatment with an acid, a product of formula $I'_A$ :

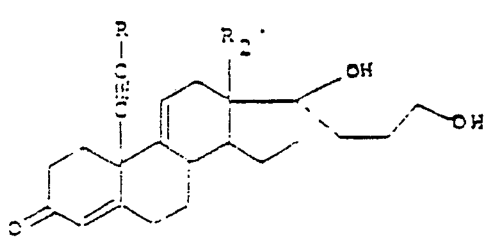

($I'_A$)

which is possibly treated with sulphonic acid halogenide in the presence of a base to obtain a product of formula $I''_A$ :

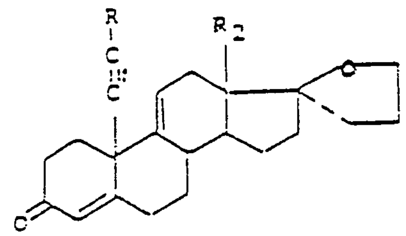

($I''_A$)

III) or by a reduction reagent then by an acid to obtain a product of formula $I_{3A}$ :

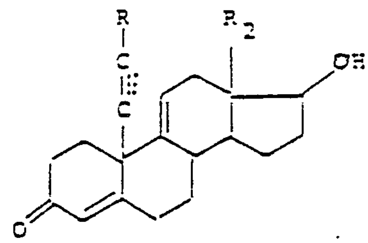

($I_{3A}$)

IV) or by an organometallic compound $R_4MgX$ or $(R_4)_2CuLi$, X representing a halogen atom, then by an acid to obtain a product of formula $I_{4A}$ :

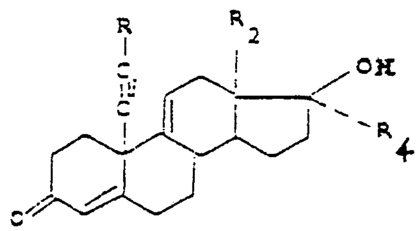

($I_{4A}$)

V) or first by a trimethylsulfonium halogenide in the presence of a strong base, then by a primary amine of formula $H_2N$—alk, alk being defined as previously, in the presence of an acid, by an alkyl chloroformate, by a strong base and finally by an acid to obtain a product of formula $I_{5A}$ :

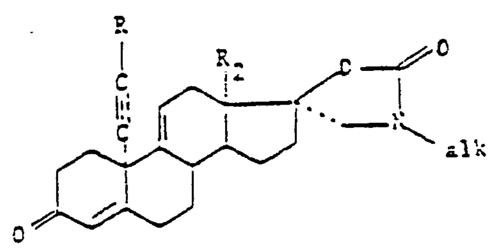

VI) or first by a trimethylsulfonium halogenide in the presence of a strong base, then by methyltertbutylsulfoxide in the presence of n-butyl-lithium to obtain a product of formula

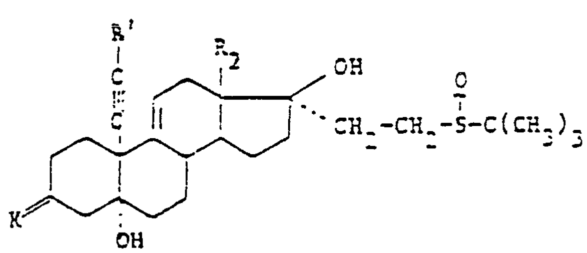

in the form of a mixture of two diastereo-isomers at the sulphur atom, if desired the two diastereo-isomers are separated then either their mixture, or each of them separately is submitted to the action of an acid, to obtain a product of formula

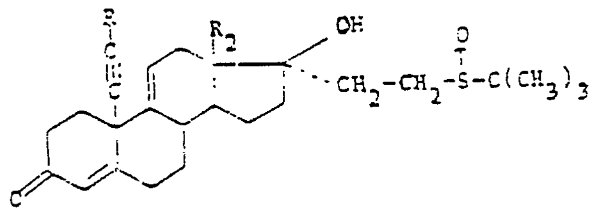

in the form of a mixture of two diastereo-isomers or one diastereo-isomer, the diastereo-isomers are separated if necessary, then either their mixture or each of them is separately is submitted to the action of N-chloro or N-bromo succinimide, to obtain a product of formula $I_{6A}$ :

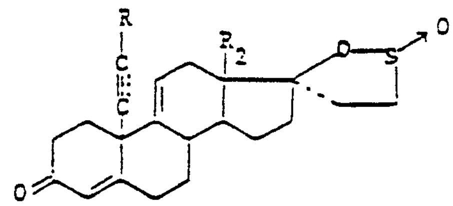

($I_{6A}$)

and that, if desired, the products of formulae $I_A$, $I'_A$, $I''_A$, $I_{3A}$, $I_{4A}$, $I_{5A}$ and $I_{6A}$ are treated by an alkyl-orthoformate in the presence of an acid then by a dehydrogenation agent to obtain products of formulae $I_B$, $I'_B$, $I''_B$, $I_{3B}$, $I_{4B}$, $I_{5B}$ and $I_{6B}$ of the following formulae :

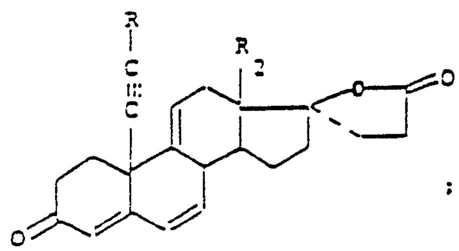

$I_B$

;

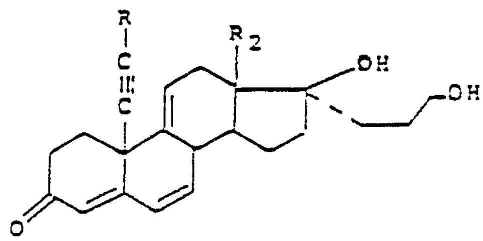

$I'_B$

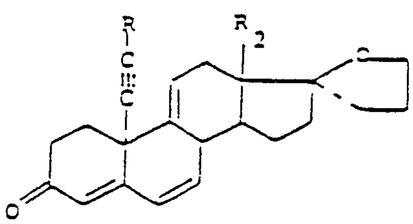

$I''_B$

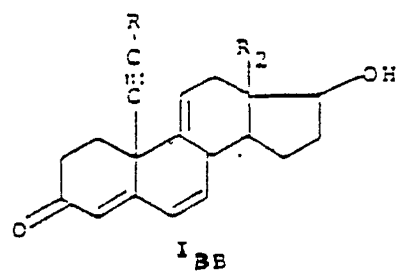

$I_{3B}$

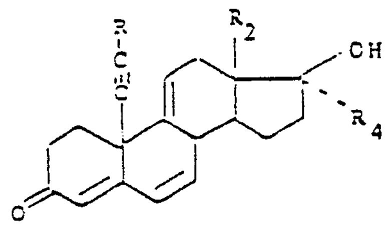

$I_{4B}$

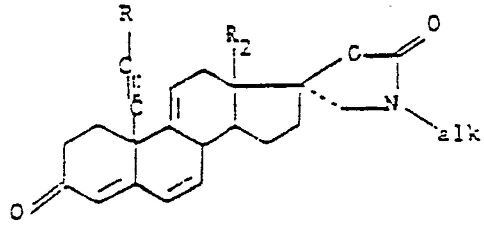

$I_{5B}$

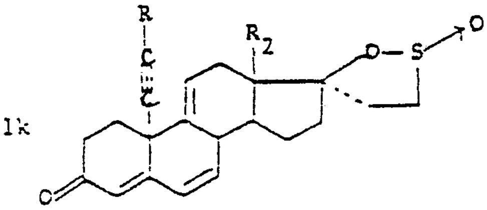

$I_{6B}$

which products of formulae $I_B$, $I'_B$, $I''_B$, $I_{3B}$, $I_{4B}$, $I_{5B}$ and $I_{6B}$, if desired, are treated :

— either by an organomagnesian of the formula $R_1MgX'$ in which $R_1$ has the meaning already given and X' represents a halogen atom, possibly in the presence of a copper salt

— or by an organometallic compound of the formula $(R_1)_2CuLi$ then by an acid, to obtain products of the formulae $I_C$, $I'_C$, $I''_C$, $I_{3C}$, $I_{4C}$, $I_{5C}$ and $I_{6C}$ :

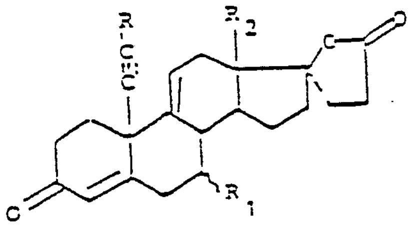 ;

$I_C$

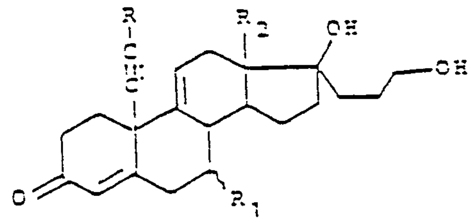

$I'_C$

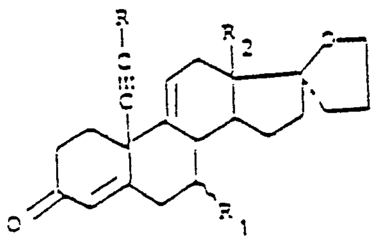 ;

$I''_C$

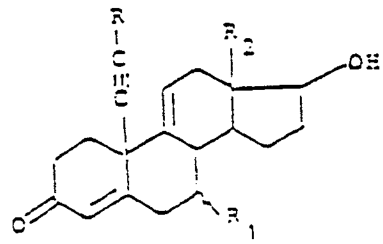

$I_{3C}$

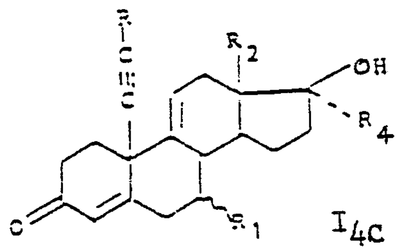

$I_{4C}$

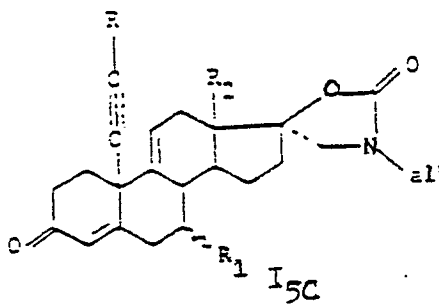

$I_{5C}$

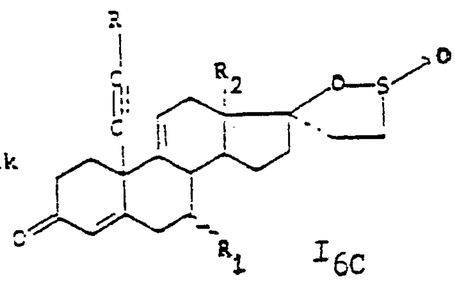

$I_{6C}$

in the form of a mixture of isomers 7alpha and 7beta, which mixture, if desired, is separated and that, if desired the 7beta products are submitted to a dehydrogenation reagent to obtain the corresponding products delta6 (7) ;

— or the products of formulae $I_B$, $I'_B$, $I''_B$, $I_{3B}$, $I_{4B}$, $I_{5B}$ and $I_{6B}$ are submitted to the action of a reagent selected from the group constituted by trimethylsulfonium iodide and trimethylsulfoxonium iodide in the presence of a strong base to obtain the products of formulae $I_D$, $I'_D$, $I''_D$, $I_{3D}$, $I_{4D}$, $I_{5D}$ and $I_{6D}$ :

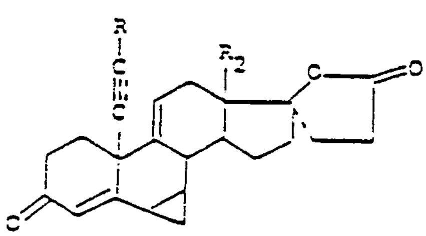

$I_D$

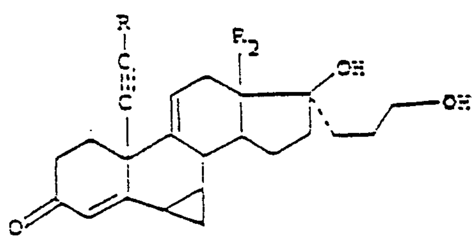

$I'_D$

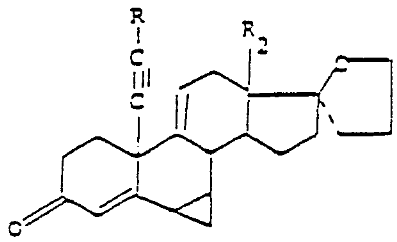

I"D

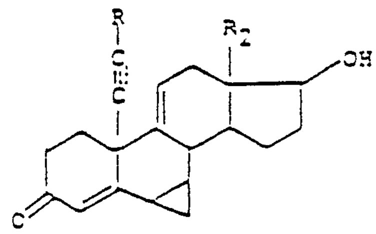

$I_{3D}$

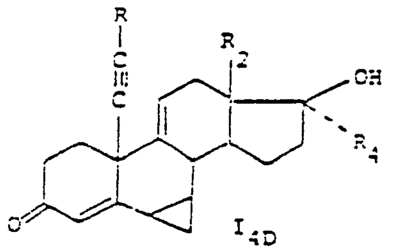

$I_{4D}$

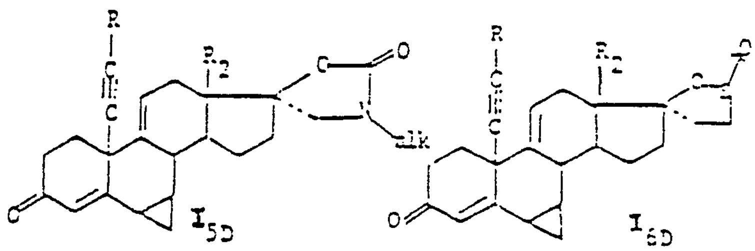

$I_{5D}$ $I_{6D}$

in the form of mixtures 6alpha, 7alpha and 6beta, 7beta and the isomers thus obtained are separated, if desired, and, if desired, the products $I_A$, $I'_A$, $I''_A$, $I_{3A}$, $I_{4A}$, $I_{5A}$, $I_{6A}$, $I_B$, $I'_B$, $I''_B$, $I_{3B}$, $I_{4B}$, $I_{5B}$, $I_{6B}$, $I_C$, $I'_C$, $I''_C$, $I_{3C}$, $I_{4C}$, $I_{5C}$, $I_{6C}$, $I_D$, $I'_D$, $I''_D$, $I_{3D}$, $I_{4D}$, $I_{5D}$, $I_{6D}$, are treated by a dehydrogenation reagent or by a microorganism capable of dehydrogenating the molecule in position 1 (2) to obtain the corresponding products containing an unsaturation at position 1 (2) and that, if desired, the products of formulae $I_A$, $I_B$, $I_C$ and $I_D$ and the corresponding products containing an unsaturation in position 1 (2) are treated by an alkaline hydroxide or ammoniac to obtain the corresponding products in which X represents a hydroxyl radical and Y represents $-CH_2CH_2CO_2M'$ radical in which M' represents a hydroxyl radical and Y represents an alkali metal or an ammonium radical and, if desired the products thus obtained are submitted to the action of an acid agent to obtain the products in which X represents a hydroxyl radical and Y represents a $-CH_2CH_2CO_2H$ radical, and that if desired the products in which the substituent in position 10 contains a substituent R of the hydroxyalkyl type is treated by carbontetrabromide or carbon tetrachloride in the presence of triphenylphosphine, to obtain the corresponding bromated and chlorated derivatives, and that, if desired, the products in which the substituent in position 10 contains a triple bond are treated by a selective hydrogenation agent to obtain the corresponding products in which the substituent in position 10 contains a double bond and that, if desired the corresponding products containing an ethynyl substituent in position 10 are treated by a halo-succinimide to obtain the corresponding products containing a $-C{\equiv}C-Hal$ substituent in position 10, and that if desired the hydroxyl radical in position 17beta of the products having a hydrogen atom, a $R_4$ radical or a $-CH_2-CH_2-CH_2OH$ radical in position 17alpha is acylated or etherified.

8. As medicaments products of the general formula I' :

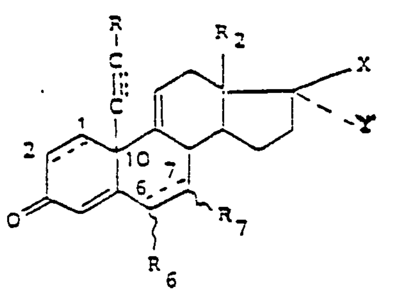

(I')

in which R represents :

— either a hydrogen atom,

— or an alkyl, alkenyl or alkynyl radical having at the most 8 carbon atoms, possibly substituted by the following radicals, hydroxyl, carboxy possibly esterified, amino, tritylamino, chloroacetylamino, trifluoroacetylamino ; trichloroethoxycarbonylamino, monoalkylamino or dialkylamino or by halogens,

— or an aryl or aralkyl radical possibly substituted by the radicals hydroxyl, carboxy possibly esterified, amino, monoalkylamino or dialkylamino, alkyl alkoxy or alkylthio,

— or a hydroxyl, tetrahydropyrannyloxy, tertbutyloxy, carboxy possibly esterified, amino, monoalkylamino or dialkylamino tritylamino, chloroacetylamino, trifluoroacetylamino or trichloroethoxycarbonylamino,

— or a halogen atom,

— or a trialkylsilyl radical,

$R_6$ and $R_7$ are such that either $R_6$ and $R_7$ form a cyclopropyl radical with the carbons carrying them, or $R_6$ represents a hydrogen atom and $R_7$ represents the radical $R_1$, $R_1$ representing :

— either a hydrogen atom,

— or an alkyl, alkenyl or alkynyl radical having at the most 6 carbon atoms possibly substituted by the following radicals hydroxyl, carboxy possibly esterified, amino, tritylamino, chloroacetylamino, trifluoroacetyl amino, trichloroethoxy-carbonyl-amino, monoalkylamino or dialkylamino or by halogens.

$R_2$ represents a methyl or ethyl radical,

X and Y are such that :

— either X represents a hydroxyl, acetyloxy, propionyloxy, methoxy or ethoxy radical and Y represents either a $—CH_2CH_2CO_2M$ radical in which

M represents a hydrogen atom an alkali metal radical or an ammonium radical,

— or a $—CH_2CH_2—CH_2OH$ radical

— or X and Y together represent a :

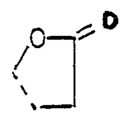

or

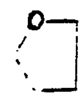

radical,

— or X and Y together represent a

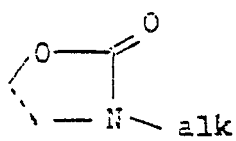

radical in which alk represents an alkyl radical containing at the most 8 carbon atoms,

— or X and Y together represent a

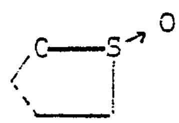

radical, or X represents a hydroxyl radical and Y represents a

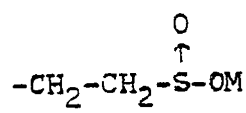

M being defined as above,

— or X represents a hydroxyl, acetyloxy, propionyloxy, methoxy or ethoxy radical and Y represents either a hydrogen atom or Y represents $R_4$, $R_4$ represents an alkyl, alkenyl, or alkynyl radical having at the most 6 carbon atoms,

the dotted lines in position 1 (2) and 6 (7) indicate the possible presence of a second bond between the carbons carrying them, it being understood that there cannot be a second bond in position 6 (7) when $R_6$ and $R_7$ form a cyclopropyl radical with the carbons carrying them, the dotted line on the substituent R—C=C— in position 10 indicates the possible presence of a third bond between the carbons carrying them,

the wavy lines in position 6 and 7 indicate that the substituents $R_6$ and $R_7$ can be in one of the possible positions, alpha or beta.

9. As medicaments, products of the general formula I' in which R is selected from the group formed by a hydrogen radical, an alkyl radical having from 1 to 3 carbon atoms, a hydroxymethyl radical and a phenyl radical, $R_1$ is selected from the group formed by a hydrogen atom, an alkyl radical having from 1 to 4 carbon atoms and an acetylthio radical and X and Y are such that either X represents a hydroxy radical and Y represents either a $CH_2CH_2CO_2M'$ radical in which M' represents a hydrogen atom or an alkali metal atom or Y represents a hydrogen atom, or X and Y together represent a

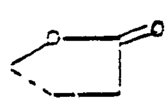

radical.

10. As medicaments, products of the general formula I', which have the following names :
—10beta-ethynyl 17beta-hydroxy 3-oxo 19-nor 17alpha-pregna-4,9(11)-dien 21-carboxylic acid gammalactone,
—10beta-(1-propynyl) 17beta-hydroxy 3-oxo 19-nor 17alpha-pregna-4,9(11)-dien 21-carboxylic acid gammalactone,
— and 10beta-ethynyl 17beta-hydroxy estra-4,9(11)-dien-3-one.

11. Pharmaceutical compositions containing at least one of the medicaments defined in any one of the claims 8 to 10 as active principle.

**Patentansprüche**

1. Produkte der allgemeinen Formel I

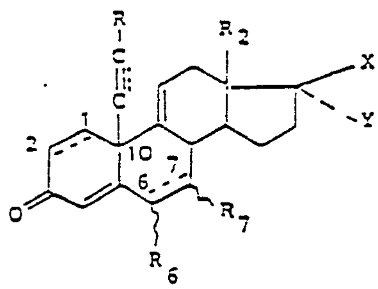

(I)

worin R bedeutet :
— entweder ein Wasserstoffatom
— oder einen Alkyl-, Alkenyl- oder Alkinylrest mit höchstens 8 Kohlenstoffatomen, gegebenenfalls substituiert durch Hydroxyl-, gegebenenfalls veresterte Carboxy-, Amino-, Tritylamino-, Chloracetylamino-, Trifluoracetylaminoreste ; Trichlorethoxycarbonylamino, Monoalkyl- oder Dialkylamino oder Halogene,
— oder einen Aryl- oder Aralkylrest, gegebenenfalls substituiert durch Hydroxyl-, gegebenenfalls veresterte Carboxy-, Amino-, Mono- oder Dialkylamino-, Alkyl-, Alkoxy- oder Alkylthioreste,
— oder einen Hydroxyl-, Tetrahydropyranyloxy-, tert.-Butyloxy-, gegebenenfalls veresterten Carboxy-, Amino-, Mono- oder Dialkylamino-, Tritylamino-, Chloracetylamino-, Trifluoracetylamino- oder Trichlorethoxycarbonylaminorest,
— oder ein Halogenatom,
— oder einen Trialkylsilylrest,
$R_6$ und $R_7$ derart sind, daß entweder $R_6$ und $R_7$ mit den Kohlenstoffatomen, die sie tragen, eine Cyclopropylgruppe bilden, oder $R_6$ ein Wasserstoffatom bedeutet und $R_7$ den Rest $R_1$ wiedergibt, wobei $R_1$ bedeutet :
— entweder ein Wasserstoffatom,
— oder einen Alkyl-, Alkenyl- oder Alkinylrest mit höchstens 6 Kohlenstoffatomen, gegebenenfalls substituiert durch Hydroxyl-, gegebenenfalls veresterte Carboxy-, Amino-, Tritylamino-, Chloracetylamino-, Trifluoracetylamino-, Trichlorethoxycarbonylamino-, Monoalkyl- oder Dialkylaminoreste oder Halogene,
$R_2$ einen Methyl- oder Ethylrest bedeutet,
X und Y derart sind, daß
— entweder X einen Hydroxyl-, Acetyloxy-, Propionyloxy-, Methoxy- oder Ethoxyrest bedeutet und Y entweder einen Rest $-CH_2CH_2CO_2M$, worin M für ein Wasserstoffatom, ein Alkalimetallatom oder eine Ammoniumgruppe steht,
oder einen Rest $-CH_2CH_2-CH_2OH$ wiedergibt,
— oder X und Y gemeinsam einen Rest

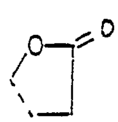 oder 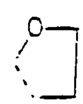

bedeuten,
— oder X und Y gemeinsam einen Rest

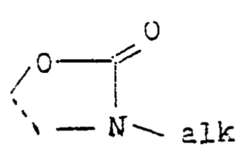

bedeuten, in dem alk eine Alkylgruppe mit höchstens 8 Kohlenstoffatomen darstellt,

— oder X und Y gemeinsam einen Rest

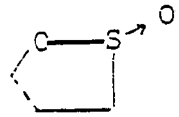

wiedergeben oder X eine Hydroxylgruppe und Y einen Rest

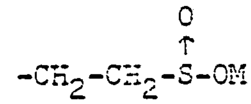

darstellt, worin M wie vorstehend definiert ist,

— oder X einen Hydroxyl-, Acetyloxy-, Propionyloxy-, Methoxy- oder Ethoxyrest darstellt und Y entweder für ein Wasserstoffatom steht oder Y $R_4$ bedeutet, wobei $R_4$ einen Alkyl-, Alkenyl- oder Alkinylrest mit höchstens 6 Kohlenstoffatomen darstellt,

die gestrichelten Linien in 1(2)- und 6(7)-Stellung die etwaige Anwesenheit einer zweiten Bindung zwischen den Kohlenstoffatomen, die sie tragen, wobei keine zweite Bindung in 6(7)-Stellung vorliegen kann, wenn $R_6$ und $R_7$ mit den Kohlenstoffatomen, die sie tragen, einen Cyclopropylrest bilden, anzeigen, die gestrichelte Linie an dem Substituenten R—C≡C— in 10-Stellung die etwaige Anwesenheit einer dritten Bindung zwischen den Kohlenstoffatomen, die sie tragen, anzeigt,

die gewellten Linien in 6- und 7-Stellung anzeigen, daß sich die Substituenten $R_6$ und $R_7$ in einer der möglichen α- oder β-Stellungen befinden können, wobei R kein Wasserstoffatom bedeuten kann, wenn $R_6$ und $R_7$ jeweils ein Wasserstoffatom wiedergeben,

$R_2$ für einen Methylrest steht, X eine Hydroxy- oder Acetoxygruppe wiedergibt und Y für ein Wasserstoffatom steht, die gestrichelten Linien in 1(2)- und 6(7)-Stellung keine zweite Bindung zwischen den Kohlenstoffatomen, die sie tragen, darstellen und die gestrichelte Linie an dem Substituenten in 10-Stellung die Anwesenheit einer dritten Bindung zwischen den Kohlenstoffatomen, die sie tragen, wiedergibt.

2. Produkte der allgemeinen Formel (I) gemäß Anspruch 1 der Formel ($I_1$)

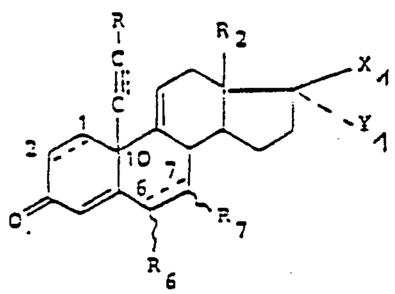

($I_1$)

worin R, $R_2$, $R_6$ und $R_7$ die in Anspruch 1 angegebene Bedeutung besitzen,

$X_1$ und $Y_1$ derart sind, daß

— entweder $X_1$ für eine Hydroxylgruppe steht und $Y_1$ entweder einen Rest $—CH_2CH_2CO_2M$ wiedergibt, worin M ein Wasserstoffatom, ein Alkalimetallatom oder eine Ammoniumgruppe bedeutet, oder einen Rest $—CH_2—CH_2—CH_2—OH$ wiedergibt,

— oder $X_1$ und $Y_1$ gemeinsam einen Rest

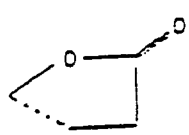

oder

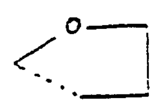

darstellen,

— oder $X_1$ für eine gegebenenfalls acylierte oder veretherte Hydroxylgruppe steht und $Y_1$ entweder ein Wasserstoffatom bedeutet oder $Y_1$ für $R_4$ steht, wobei $R_4$ einen Alkyl-, Alkenyl- oder Alkinylrest mit höchstens 6 Kohlenstoffatomen darstellt,

die gestrichelten Linien in 1(2)- und 6(7)-Stellung die etwaige Anwesenheit einer zweiten Bindung zwischen den Kohlenstoffatomen, die sie tragen, anzeigen, wobei keine zweite Bindung in 6(7)-Stellung vorliegt, wenn $R_6$ und $R_7$ mit den Kohlenstoffatomen, die sie tragen, einen Cyclopropylrest bilden, die gestrichelte Linie an dem Substituenten R—C≡C— in 10-Stellung die etwaige Anwesenheit einer dritten Bindung zwischen den Kohlenstoffatomen, die sie tragen, anzeigt,

die gewellten Linien in 6- und 7-Stellung anzeigen, daß sich die Substituenten $R_6$ und $R_7$ in einer der möglichen α- oder β-Stellungen befinden können, wobei R kein Wasserstoffatom wiedergeben kann, wenn $R_6$ und $R_7$ jeweils ein Wasserstoffatom bedeuten, $R_2$ einen Methylrest bedeutet, $X_1$ einen Hydroxy- oder Acetoxyrest darstellt und $Y_1$ für ein Wasserstoffatom steht, die gestrichelten Linien in 1(2)- und 6(7)-Stellung keine zweite Bindung zwischen den Kohlenstoffatomen, die sie tragen, wiedergeben und die gestrichelte Linie an dem Substituenten in 10-Stellung die Anwesenheit einer dritten Bindung zwischen den Kohlenstoffatomen, die sie tragen, anzeigt.

3. Produkte der allgemeinen Formel $I_1$ gemäß Anspruch 2 der Formel

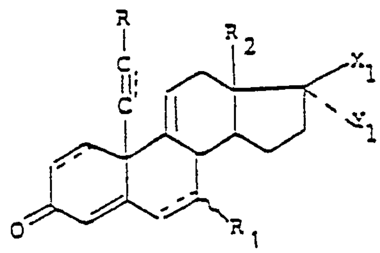

worin R, $R_1$, $R_2$, $X_1$, $Y_1$, die gestrichelten Linien und die gewellten Linien die in Anspruch 2 gegebene Bedeutung besitzen.

4. Produkte der Formel I gemäß einem der Ansprüche 1 bis 3, worin die gestrichelte Linie an dem Substituenten in 10-Stellung eine dritte Bindung wiedergibt und $R_2$ unter einem Wasserstoffatom, einem Alkylrest mit 1 bis 3 Kohlenstoffatomen, einer Hydroxymethylgruppe und einem Phenylrest ausgewählt ist.

5. Produkte der Formel I gemäß einem der Ansprüche 1 bis 4, worin der Substituent $R_1$ unter einem Wasserstoffatom, einem Alkylrest mit 1 bis 4 Kohlenstoffatomen und einem Acetylthiorest ausgewählt ist und X und Y derart sind, daß entweder X eine Hydroxygruppe wiedergibt und Y entweder für einen Rest $CH_2CH_2CO_2M'$ steht, worin M' ein Wasserstoffatom oder ein Alkalimetallatom bedeutet, oder Y ein Wasserstoffatom bedeutet,

— oder X und Y einen Rest

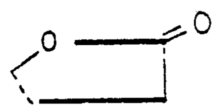

wiedergeben.

6. Produkte der allgemeinen Formel I mit den folgenden Bezeichnungen :

γ-Lacton der 10β-Ethinyl-17β-hydroxy-3-oxo-19-nor-17α-pregna-4,9(11)-dien-21-carbonsäure,

γ-Lacton der 10β-(1-Propinyl)-17β-hydroxy-3-oxo-19-nor-17α-pregna-4,9(11)-dien-21-carbonsäure.

7. Verfahren zur Herstellung der Produkte der Formel I gemäß Anspruch 1, dadurch gekennzeichnet, daß man ein Produkt der Formel II

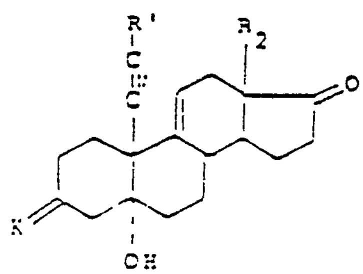

(II)

worin R' entweder für R steht, wobei R die in Anspruch 1 angegebene Bedeutung besitzt, oder für den Substituenten R steht, in dem die reaktiven Funktionen geschützt sind, $R_2$ wie in Anspruch 1 definiert ist und K eine Schutzgruppe für die Ketonfunktion wiedergibt,

I) entweder zunächst mit einem Trimethylsulfoniumhalogenid in Anwesenheit einer starken Base, dann mit einem Metallderivat des Acetonitrils und schließlich mit einer Base und danach mit einer Säure behandelt,

oder zunächst mit einem Reagens der Formel

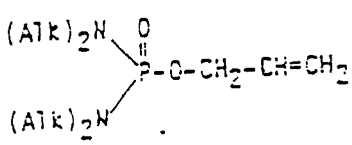

worin Alk für einen Alkylrest mit 1 bis 4 Kohlenstoffatomen steht, in Anwesenheit einer starken Base, dann mit einer Säure behandelt, um zu einem Produkt der Formel $I_A$

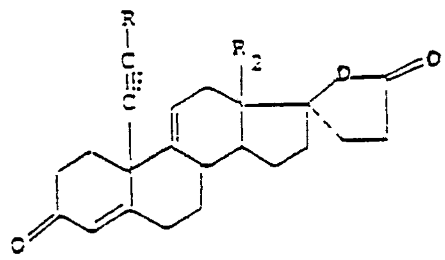

($I_A$)

zu gelangen ;

II) oder mit einem Reagens der Formel

$$XMg—CH_2CH_2CH_2OB$$

worin X für ein Halogenatom steht und B eine Schutzgruppe des Hydroxylrestes bedeutet oder mit einem Magnesiumalkoholat behandelt, um nach Behandlung mit einer Säure zu einem Produkt der Formel $I'_A$

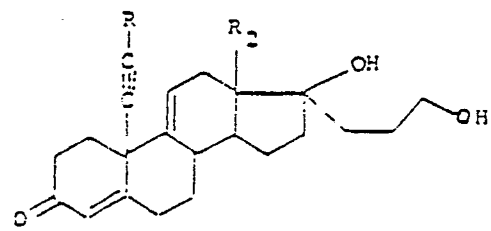

($I'_A$)

zu gelangen, das man gegebenenfalls mit einem Sulfonsäurehalogenid in Anwesenheit einer Base behandelt, um zu einem Produkt der Formel $I''_A$

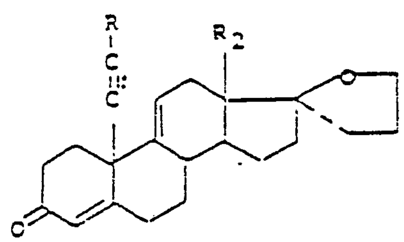

($I''_A$)

zu gelangen ;

III) oder mit einem Reduktionsreagens und danach mit einer Säure behandelt, um zu einem Produkt der Formel $I_{3A}$

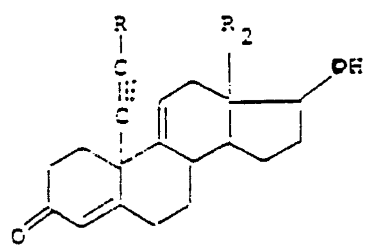

($I_{3A}$)

zu gelangen ;

IV) oder mit einer organometallischen Verbindung $R_4MgX$ oder $(R_4)_2$-CuLi, worin X für ein Halogenatom steht, und danach mit einer Säure behandelt, um zu einem Produkt der Formel $I_{4A}$

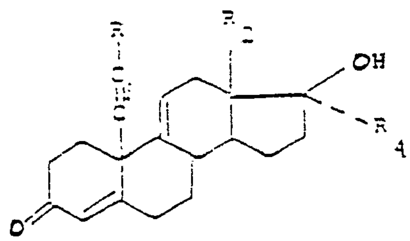

($I_{4A}$)

zu gelangen ;

V) oder zunächst mit einem Trimethylsulfoniumhalogenid in Anwesenheit einer starken Base, dann mit einem primären Amin der Formel $H_2N$—alk, worin alk wie vorstehend definiert ist, in Anwesenheit einer Säure, mit einem Alkylchlorformiat, mit einer starken Base und schließlich mit einer Säure behandelt, um zu einem Produkt der Formel $I_{5A}$

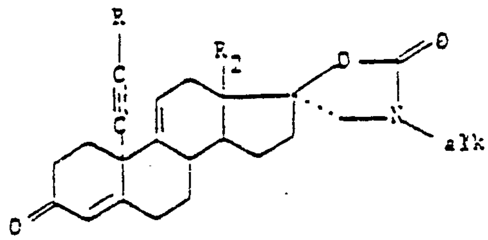

($I_{5A}$)

zu gelangen ;

VI) oder zunächst mit einem Trimethylsulfoniumhalogenid in Gegenwart einer starken Base, dann mit Methyl-tert.-butylsulfoxid in Anwesenheit von n-Butyllithium behandelt, um zu einem Produkt der Formel

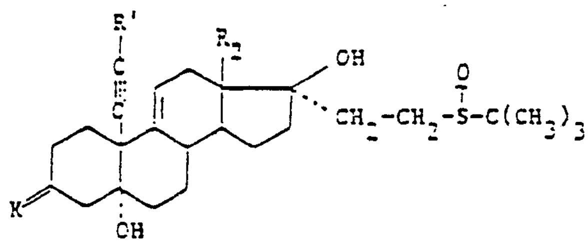

in Form eines Gemisches der beiden Diastereomeren hinsichtlich des Schwefelatoms zu gelangen, die beiden Diastereomeren gewünschtenfalls trennt, hiernach entweder deren Gemisch oder ein jedes von ihnen getrennt der Einwirkung einer Säure unterzieht, um zu einem Produkt der Formel

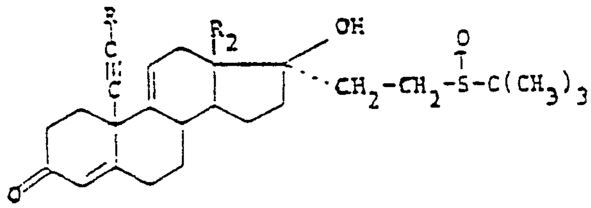

in Form eines Gemisches der beiden Diastereomeren oder einem Diastereomeren zu gelangen, gegebenenfalls die Diastereomeren trennt, hiernach entweder deren Gemisch oder ein jedes derselben getrennt der Einwirkung von N-Chlor- oder N-Bromsuccinimid unterzieht, um zu einem Produkt der Formel $I_{6A}$

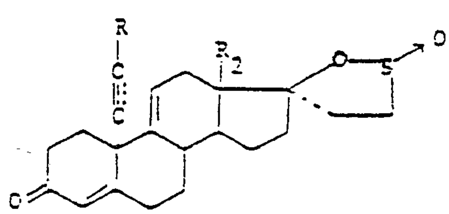

($I_{6A}$)

zu gelangen, und gewünschtenfalls die Produkte der Formeln $I_A$, $I'_A$, $I''_A$, $I_{3A}$, $I_{4A}$, $I_{5A}$ und $I_{6A}$ mit einem Alkylorthoformiat in Gegenwart einer Säure, dann mit einem Dehydrierungsmittel behandelt, um die Produkte der Formel $I_B$, $I'_B$, $I''_B$, $I_{3B}$, $I_{4B}$, $I_{5B}$ und $I_{6B}$ der folgenden Formeln

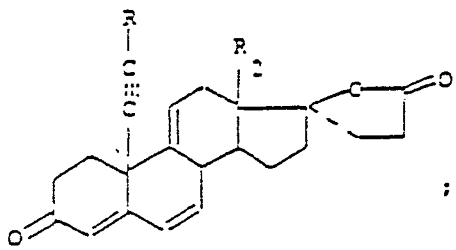

$I_B$

;

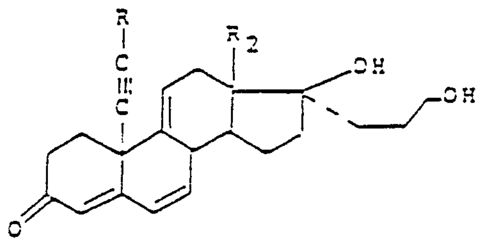

$I'_B$

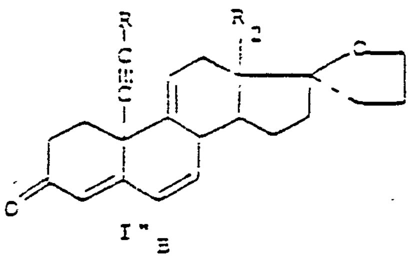

$I''_B$

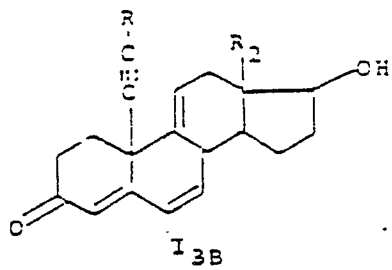

$I_{3B}$

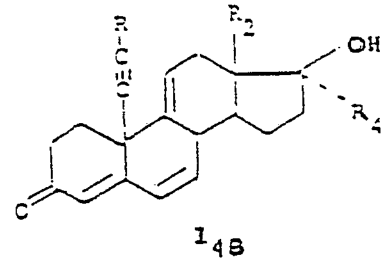

$I_{4B}$

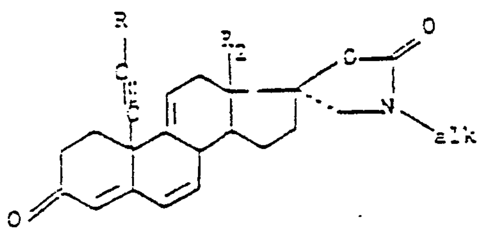

$I_{5B}$

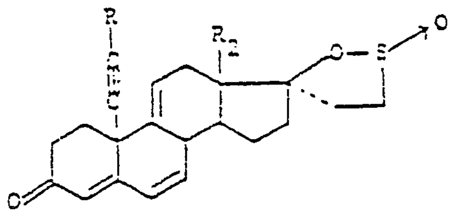

$I_{6B}$

zu erhalten, die Produkte der Formeln $I_B$, $I'_B$, $I''_B$, $I_{3B}$, $I_{4B}$, $I_{5B}$ und $I_{6B}$ man gewünschtenfalls behandelt :

— entweder mit einer Organomagnesiumverbindung der Formel $R_1MgX'$ gegebenenfalls in Gegenwart eines Kupfersalzes, wobei $R_1$ die vorstehend angegebene Bedeutung besitzt und X' für ein Halogenatom steht,

— oder mit einer organometallischen Verbindung der Formel $(R_1)_2CuLi$, dann mit einer Säure, um zu den Produkten der Formeln $I_C$, $I'_C$, $I''_C$, $I_{3C}$, $I_{4C}$, $I_{5C}$ und $I_{6C}$

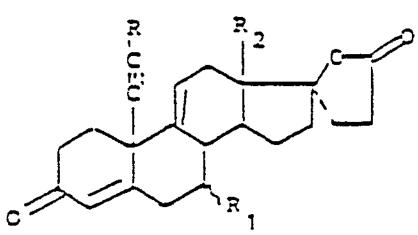

$I_C$

;

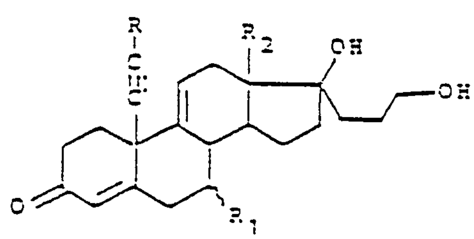

$I'_C$

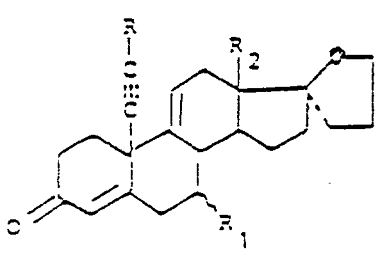

$I''_C$

;

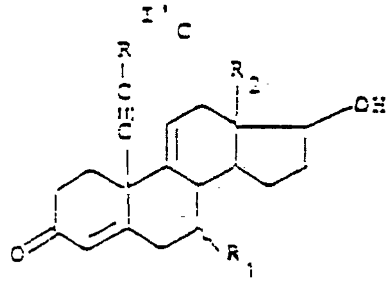

$I_{3C}$

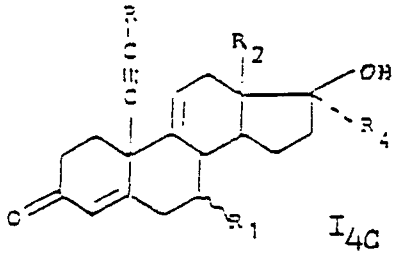

$I_{4C}$

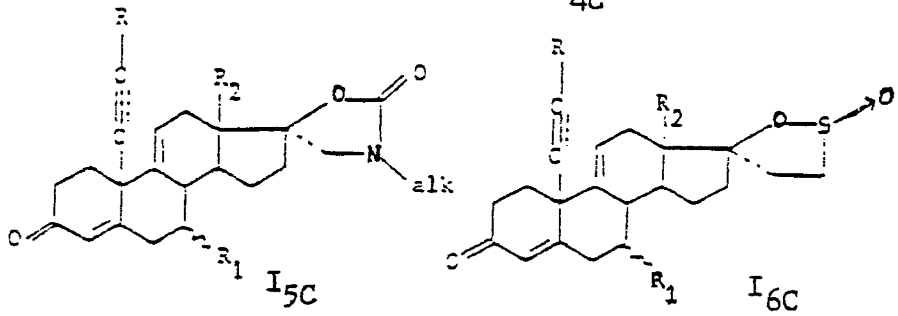

$I_{5C}$ $I_{6C}$

in Form eines Gemisches der 7α- und 7β-Isomeren zu gelangen, gewünschtenfalls das Gemisch trennt und gewünschtenfalls die 7β-Produkte einem Dehydrierungsreagens unterzieht, um die entsprechenden Δ6(7)-Produkte zu erhalten,

— oder die Produkte der Formel $I_B$, $I'_B$, $I''_B$, $I_{3B}$, $I_{4B}$, $I_{5B}$ und $I_{6B}$ der Einwirkung eines Reagens, ausgewählt unter Trimethylsulfoniumjodid und Trimethylsulfoxoniumjodid, in Anwesenheit einer starken Base unterzieht, um die Produkte der Formeln $I_D$, $I'_D$, $I''_D$, $I_{3D}$, $I_{4D}$, $I_{5D}$ und $I_{6D}$

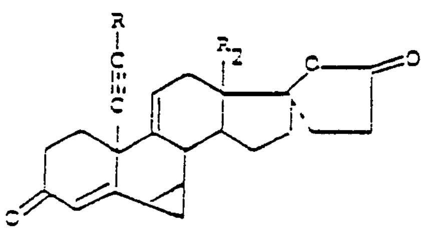

$I_D$

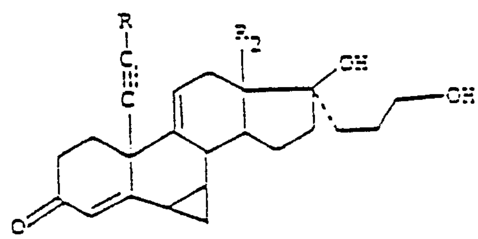

$I'_D$

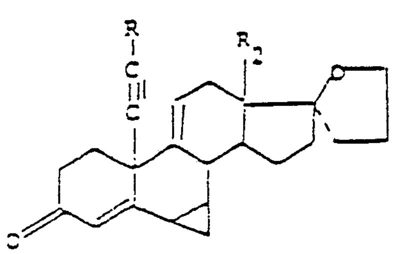

$I''_D$

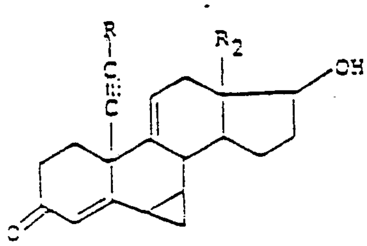

$I_{3D}$

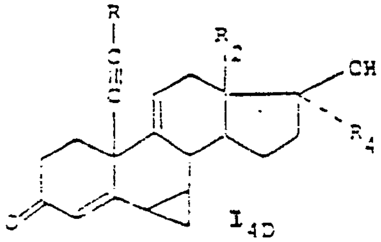

$I_{4D}$

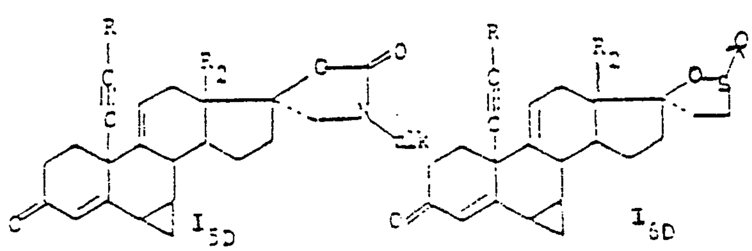

$I_{5D}$ $I_{6D}$

in Form eines 6α, 7α- und 6β, 7β-Gemisches zu erhalten, und gewünschtenfalls die so erhaltenen Isomeren trennt und gewünschtenfalls die Produkte $I_A$, $I'_A$, $I''_A$, $I_{3A}$, $I_{4A}$, $I_{5A}$, $I_{6A}$, $I_B$, $I'_B$, $I''_B$, $I_{3B}$, $I_{4B}$, $I_{5B}$, $I_{6B}$, $I_C$, $I'_C$, $I''_C$, $I_{3C}$, $I_{4C}$, $I_{5C}$, $I_{6C}$, $I_D$, $I'_D$, $I''_D$, $I_{3D}$, $I_{4D}$, $I_{5D}$, $I_{6D}$ mit einem Dehydrierungsreagens oder mit einem Mokroorganismus behandelt, der befähigt ist, das Molekül in 1(2)-Stellung zu dehydrieren, um die entsprechenden Produkte mit einer Unsättigung in 1(2)-Stellung zu erhalten, und gewünschtenfalls die Produkte der Formel $I_A$, $I_B$, $I_C$, und $I_D$ und die entsprechenden Produkte mit einer Unsättigung in 1(2)-Stellung mit Hilfe eines Alkalihydroxids oder von Ammoniak behandelt, um zu den entsprechenden Produkten zu gelangen, worin X für eine Hydroxylgruppe steht und Y einen Rest $—CH_2CH_2CO_2M'$ wiedergibt, worin M' ein Alkalimetallatom oder eine Ammoniumgruppe bedeutet, und gewünschtenfalls die so erhaltenen Produkte der Einwirkung eines sauren Mittels unterzieht, um die Produkte zu erhalten, worin X für eine Hydroxylgruppe steht und Y einen Rest $—CH_2CH_2CO_2H$ wiedergibt, und gewünschtenfalls die Produkte, worin der Substituent in 10-Stellung einen Substituenten R vom Hydroxyalkyl-Typ aufweist, mit Tetrabromkohlenstoff oder Tetrachlorkohlenstoff in Gegenwart von Triphenylphosphin behandelt, um zu den entsprechenden bromierten und chlorierten Derivaten zu gelangen, und gewünschtenfalls die Produkte, bei denen der Substituent in 10-Stellung eine Dreifachbindung besitzt, mit einem selektiven Hydrierungsreagens behandelt, um die entsprechenden Produkte zu erhalten, in denen der Substituent in 10-Stellung eine Doppelbindung aufweist, und gewünschtenfalls die Produkte mit einem Ethinyl-Substituenten in 10-Stellung mit einem Halosuccinimid behandelt, um zu den entsprechenden Produkten zu gelangen, die in 10-Stellung einen Substituenten —C≡C—Hal aufweisen, und gewünschtenfalls die Hydroxylgruppe in 17β-Stellung der Produkte, die in 17α-Stellung ein Wasserstoffatom, einen Rest $R_4$ oder einen Rest $—CH_2—CH_2—CH_2OH$ besitzen, acyliert oder verethert.

8. Als Arzneimittel die Produkte der allgemeinen Formel I'

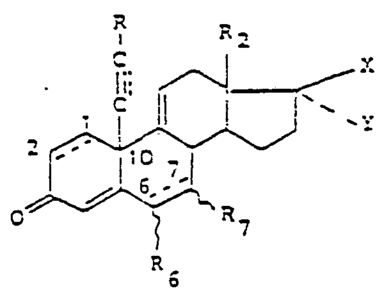

(I')

worin R bedeutet :

— entweder ein Wasserstoffatom,

— oder einen Alkyl-, Alkenyl- oder Alkinylrest mit höchstens 8 Kohlenstoffatomen, gegebenenfalls substituiert durch Hydroxyl-, gegebenenfalls veresterte Carboxy-, Amino-, Tritylamino-, Chloracetylamino-, Trifluoracetylamino-Reste ; Trichlorethoxycarbonylamino, Monoalkyl- oder Diakylamino oder Halogene,

— oder einen Aryl- oder Aralkylrest, der gegebenenfalls substituiert ist durch Hydroxyl-, gegebenenfalls veresterte Carboxy-, Amino-, Mono- oder Dialkylamino-, Alkyl-, Alkoxy- oder Alkylthiogruppen,

— oder einen Hydroxyl-, Tetrahydropyranyloxy-, tert.-Butyloxy-, gegebenenfalls veresterten Carboxy-, Amino-, Mono- oder Dialkylamino-, Tritylamino-, Chloracetylamino-, Trifluoracetylamino- oder Trichlorethoxycarbonylaminorest,

— oder ein Halogenatom,

— oder einen Trialkylsilylrest,

$R_6$ und $R_7$ derart sind, daß entweder $R_6$ und $R_7$ mit den Kohlenstoffatomen, die sie tragen, einen Cyclopropylrest bilden, oder $R_6$ ein Wasserstoffatom bedeutet und $R_7$ den rest $R_1$ wiedergibt, wobei $R_1$ bedeutet :

— entweder ein Wasserstoffatom,

— oder einen Alkyl-, Alkenyl- oder Alkinylrest mit höchstens 6 Kohlenstoffatomen, der gegebenenfalls durch Hydroxyl-, gegebenenfalls veresterte Carboxy-, Amino-, Tritylamino-, Chloracetylamino-, Trifluoracetylamino-, Trichlorethoxycarbonylamino-, Monoalkyl- oder Dialkylamino- Reste oder Halogene substituiert ist,

$R_2$ für einen Methyl- oder Ethylrest steht,

X und Y derart sind, daß

— entweder X für einen Hydroxyl-, Acetyloxy-, Propionyloxy-, Methoxy- oder Ethoxyrest steht und Y entweder einen Rest $—CH_2CH_2CO_2M$ wiedergibt, worin

M für ein Wasserstoffatom, ein Alkalimetallatom oder einen Ammoniumrest steht,

oder einen Rest $—CH_2CH_2—CH_2OH$ bedeutet,

— oder X und Y gemeinsam einen Rest

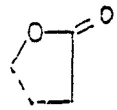

oder

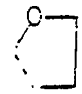

darstellen,

— oder X und Y gemeinsam einen Rest

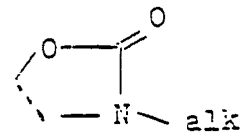

darstellen, worin alk für einen Alkylrest mit höchstens 8 Kohlenstoffatomen steht,

— oder X und Y gemeinsam einen Rest

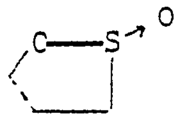

wiedergeben oder X eine Hydroxylgruppe darstellt und Y für einen Rest

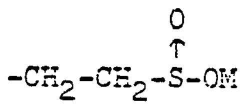

steht, worin M wie vorstehend definiert ist,

— oder X für ein Hydroxyl-, Acetyloxy-, Propionyloxy-, Methoxy- oder Ethoxyrest steht und Y entweder ein Wasserstoffatom bedeutet oder Y für $R_4$ steht, wobei $R_4$ einen Alkyl-, Alkenyl- oder Alkinylrest mit höchstens 6 Kohlenstoffatomen darstellt,

die gestrichelten Linien in 1(2)- und 6(7)-Stellung die etwaige Anwesenheit einer zweiten Bindung zwischen den Kohlenstoffatomen, die sie tragen, anzeigen, wobei keine zweite Bindung in 6(7)-Stellung vorliegen kann, wenn $R_6$ und $R_7$ mit den Kohlenstoffatomen, die sie tragen, einen Cyclopropylrest bilden, die gestrichelte Linie an dem Susbtituenten R—C=C— in 10-Stellung die etwaige Anwesenheit einer dritten Bindung zwischen den Kohlenstoffatomen, die sie tragen, anzeigt,

die gewellten Linien in 6- und 7-Stellung anzeigen, daß sich die Substituenten $R_6$ und $R_7$ in einer der möglichen α- oder β-Positionen befinden können.

9. Als Arzneimittel die Produkte der allgemeinen Formel I', worin R unter einem Wasserstoffatom, einem Alkylrest mit 1 bis 3 Kohlenstoffatomen, einem Hydroxymethylrest und einem Phenylrest ausgewählt ist, $R_1$ unter einem Wasserstoffatom, einem Alkylrest mit 1 bis 4 Kohlenstoffatomen und einem Acetylthiorest ausgewählt ist und X und Y derart sind, daß entweder X für einen Hydroxyrest steht und Y entweder eine Gruppe $CH_2CH_2CO_2M'$ darstellt, worin M' ein Wasserstoffatom oder ein Alkalimetallatom wiedergibt, oder Y für ein Wasserstoffatom steht, oder X und Y einen Rest

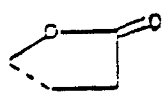

wiedergeben.

10. Als Arzneimittel die Produkte der allgemeinen Formel I' mit den folgenden Bezeichnungen :

γ-Lacton der 10β-Ethinyl-17β-hydroxy-3-oxo-19-nor-17α-pregna-4,9(11)-dien-21-carbonsäure,

γ-Lacton der 10β-(1-Propinyl)-17β-hydroxy-3-oxo-19-nor-17α-pregna-4,9(11)-dien-21-carbonsäure, und

10β-Ethinyl-17β-hydroxy-östra-4,9(11)-dien-3-on.

11. Pharmazeutische Zusammensetzungen, enthaltend als Wirkstoff zumindest ein Arzneimittel gemäß einem der Ansprüche 8 bis 10.